# EUROPEAN PATENT SPECIFICATION

(11) **EP 4 326 717 B1**
(45) Date of publication and mention of the grant of the patent: **16.04.2025**
(21) Application number: 22724448.0
(22) Date of filing: 21.04.2022
(51) Int. Cl.: C07D 413/14, C07D 471/04, C07D 491/056, A61P 7/00, A61K 31/437

(54) **MODIFIED FERROPORTIN INHIBITORS**
MODIFIZIERTE FERROPORTIN-INHIBITOREN
INHIBITEURS DE LA FERROPORTINE MODIFIÉS

(30) Priority: 22.04.2021 EP 21169916
(43) Date of publication of application: 28.02.2024
(73) Proprietor: VIFOR (INTERNATIONAL) AG, 9001 St. Gallen (CH)
(72) Inventor: BUHR, Wilm, 78465 Konstanz (DE); KALOGERAKIS, Aris, 4657 Dulliken (CH); UMLAND, Klaus-Daniel, 8400 Winterthur (CH); REIM, Stefan, 9487 Gamprin (LI); MANOLOVA, Vania, 8702 Zollikon (CH); ALTERMATT, Patrick, 8103 Unterengstringen (CH); FLACE, Anna, 8052 Zürich (CH)
(74) Representative: Gille Hrabal Partnerschaftsgesellschaft mbB Patentanwälte
(86) International application number: PCT/EP2022/060546
(87) International publication number: WO 2022/223689

(56) References cited:
- WO-A1-2017/068090

## Description

### INTRODUCTION

The invention relates to novel compounds of the general formula (I) 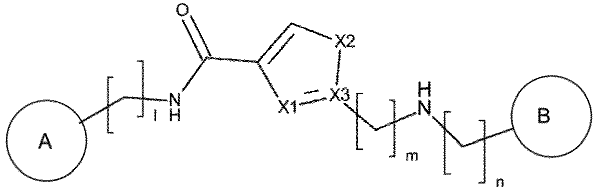 and pharmaceutically acceptable salts thereof. The compounds of the general formula (I) of the present invention act as ferroportin inhibitors and are thus particularly suitable for the use as medicaments in the prophylaxis and/or treatment of diseases caused by a lack of hepcidin or of iron metabolism disorders leading to increased iron levels or increased iron absorption. The compounds of the general formula (I) of the present invention are further particularly suitable for the use in the prophylaxis and/or treatment of iron overload, including thalassemia, sickle cell disease and hemochromatosis, as well as for the use in the prophylaxis and/or treatment of diseases related to or caused by increased iron levels, increased iron absorption or iron overload.

### BACKGROUND AND PRIOR ART

Iron is an essential trace element for almost all organisms and is relevant in particular with respect to growth and the formation of blood. The balance of the iron metabolism is in this case primarily regulated on the level of iron recovery from haemoglobin of ageing erythrocytes and the duodenal absorption of dietary iron. The released iron is taken up via the intestine, in particular via specific transport systems (DMT-1, ferroportin), transferred into the blood circulation and thereby conveyed to the appropriate tissues and organs (transferrin, transferrin receptors).

Mammalian organisms are unable to actively discharge iron. The iron metabolism is substantially controlled by hepcidin, a peptide hormone produced in the liver, via the cellular release of iron from macrophages, hepatocytes and enterocytes. Hepcidin acts on the absorption of iron via the intestine and via the placenta and on the release of iron from the reticuloendothelial system. In the body, hepcidin is synthesized in the liver from what is known as pro-hepcidin, pro-hepcidin being coded by the gene known as the HAMP gene. The formation of hepcidin is regulated in direct correlation to the organisms iron level, i.e. if the organism is supplied with sufficient iron and oxygen, more hepcidin is formed, if iron and oxygen levels are low, or in case of increased erythropoiesis less hepcidin is formed. In the small intestinal mucosal cells and in the macrophages hepcidin binds with the transport protein ferroportin, which conventionally transports the phagocytotically recycled iron from the interior of the cell into the blood.

The transport protein ferroportin is a transmembrane protein consisting of 571 amino acids which is formed in the liver, spleen, kidneys, heart, intestine and placenta. In particular, ferroportin is localized in the basolateral membrane of intestinal epithelial cells. Ferroportin bound in this way thus acts to export the iron into the blood. In this case, it is most probable that ferroportin transports iron as Fe²⁺. If hepcidin binds to ferroportin, ferroportin is transported into the interior of the cell, where its breakdown takes place so that the release of the phagocytotically recycled iron from the cells is then almost completely blocked. If the ferroportin is inactivated, for example by hepcidin, so that it is unable to export the iron which is stored in the mucosal cells, the stored iron is lost with the natural shedding of cells via the stools. The absorption of iron in the intestine is therefore reduced, when ferroportin is inactivated or inhibited, for example by hepcidin. In addition, ferroportin is markedly localized in the reticuloendothelial system (RES), to which the macrophages also belong. On the other hand, if the serum iron level decreases, hepcidin production in the hepatocytes of the liver is reduced so that less hepcidin is released and accordingly less ferroportin is inactivated, allowing a larger amount of stored iron to be transported into the serum.

Therefrom it becomes apparent that the hepcidin-ferroportin system directly regulates the iron metabolism and that a disorder of the hepcidin regulation mechanism therefore has a direct effect on iron metabolism in the organism. In principle the hepcidin-ferroportin regulation mechanism acts via the two following opposite principles:
On the one hand, an increase of hepcidin leads to inactivation of ferroportin, thus blocking the release of stored iron from the cells into the serum, thus decreasing the serum iron level. In pathological cases a decreased serum iron level leads to a reduced hemoglobin level, reduced erythrocyte production and thus to iron deficiency anemia.

On the other hand, a decrease of hepcidin results in an increase of active ferroportin, thus allowing an enhanced release of stored iron and an enhanced iron uptake e.g. from the food, thus increasing the serum iron level. In pathological cases an increased iron level leads to iron overload.

Iron overload states and diseases are characterized by excess iron levels. Therein, the problems arise from excess serum iron level which lead to non-transferrin bound iron (NTBI). The NTBI is rapidly taken up unspecifically by the organs, leading to an accumulation of iron in tissue and organs. Iron overload causes many diseases and undesired medical conditions, including cardiac, liver and endocrine damage. Further, iron accumulation in brain has been observed in patients suffering from neurodegenerative diseases such as for example Alzheimer's disease and Parkinson's disease. As a particular detrimental aspect of excess free iron the undesired formation of radicals must be mentioned. In particular iron(II) ions catalyze the formation (inter alia via Fenton reaction) of reactive oxygen species (ROS). These ROS cause damage to DNA, lipids, proteins and carbohydrates which has far-reaching effects in cells, tissue and organs and is well known and described in the literature to cause the so-called oxidative stress.

Besides the conventional methods for treating iron overload by removing iron from the body e.g. with chelating agents such as deferoxamine (also known as desferrioxamine B, N'-{5-[acetyl(hydroxy)amino]pentyl}-N-[5-({4-[(5-aminopentyl)(hydroxy)amino]-4-oxobutanoyl} amino)pentyl]-N-hydroxysuccinamide or Desferal^{®}), deferasirox (Exjade^{®}, 4-(3,5-bis(2-hydroxyphenyl)-1H-1,2,4-triazol-1-yl)benzoic acid) and deferiprone (Ferriprox^{®}, 3-hydroxy-1 ,2-dimethylpyridin-4(1H)-one), compounds acting as hepcidin agonists or having an inhibiting or supporting effect on the biochemical regulatory pathways in the iron metabolism, such as hepcidin mimetic peptides have been described. Said therapeutic approaches are based on a direct involvement into the disturbed iron metabolism pathway by directly acting via the primary regulator hepcidin by providing a hepcidin mimetic or a hepcidin agonist, i.e. acting in the sense of a kind of hepcidin substitute or supply. The approach is based on the therapeutic rationale to treat iron overload, i.e. excess serum iron level, by inhibiting ferroportin, via the hepcidin-inactivation mechanism, thus blocking excessive iron absorption.

Ferroportin inhibitors and methods for preparing the same have been described in WO2017/068089, in WO2017/068090 and in the international application WO2021/191202. Further, the international application WO2018/192973 describes the preparation and crystallization of various specific salts of selected ferroportin inhibitors described therein and as described in WO2017/068089 and in WO2017/068090.

WO2011/029832 relates to thiazol and oxazol compounds which act as hepcidin antagonists being described as suitable in the use for the treatment of iron deficiency diseases.

### OBJECT OF THE INVENTION

The object of the present invention was to provide new therapeutically effective compounds that can be used for an effective therapy for the prophylaxis and treatment of iron metabolism disorders which are associated with increased iron levels, such as in particular iron overload. In a further object, the new compounds should exhibit few side effects and have a very low toxicity and good bioavailability and compatibility. Moreover, these new compounds, in contrast to the known iron chelating compounds, should be suitable to prevent the occurrence of increased iron levels and thus the related disorders, instead of removing excess iron from the body when the iron overload has already occurred. In a further object the new compounds should have a defined structure (stoichiometry) and should be preparable by simple synthesis processes, exhibit less sensitivity and improved long-lasting efficiency as compared to the known biomolecular compounds, such as antibodies.

This goal was achieved by the development of the novel compounds according to the formulae as defined herein, such as in particular formula (I), which have been found to act as ferroportin inhibitors, thus being suitable for the use in the inhibition of iron transport, and thus being effective in the prophylaxis and treatment of iron metabolism disorders which are associated with increased iron levels, such as in particular iron overload, as well as in in the prophylaxis and treatment of diseases caused by a lack of hepcidin, diseases related to or caused by increased iron levels or iron overload and diseases associated with ineffective erythropoiesis.

### DETAILED DESCRIPTION OF THE INVENTION

The inventors have surprisingly found that specific compounds having the general structural formula (I) as defined herein, act as ferroportin inhibitors, thus effectively inhibiting iron transport and accordingly being particularly suitable for the use as medicaments, in particular for the use in the treatment and/or prophylaxis of diseases caused by a lack of hepcidin, diseases associated with ineffective erythropoiesis or iron metabolism disorders leading to increased iron levels, such as particularly iron overload states such as in particular thalassemia and hemochromatosis. Very particularly the new compounds turned out to be suitable for treating thalassemia and hemochromatosis. The new compounds are also suitable for the treatment of diseases caused by pathologically low hepcidin-levels and for the use in the inhibition of iron transport. In particular, the new compounds described herein show good metabolic stability and good bioavailability, which makes them particularly suitable as drug compounds.

Any references in the description to methods of treatment refer to the compounds, pharmaceutical compositions and medicaments of the present invention for use in a method for treatment of the human (or animal) body by therapy (or for diagnosis).

Accordingly, the invention relates to novel compounds of general formula (I) 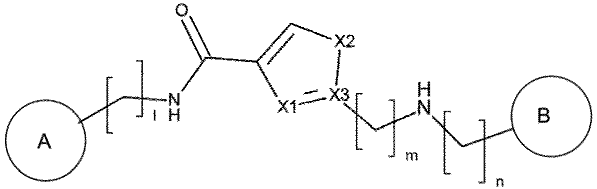 wherein
l is an integer of 1 or 2;
m and n are independently an integer of 1, 2 or 3;
X¹ is N, S or O;
X² is N, S, O or CR⁴;
X³ is C or N;
with the proviso that one of X¹ and X² is N
and if X³ is N, then X² is CR⁴; and wherein
R⁴ represents
   - H,
   - halogen,
   - linear or branched C₁-C₃-alkyl, or
   - linear or branched C₁-C₃-haloalkyl,
A represents one of the following groups (a-1), (a-2), (a-3), (a-4) and (a-5) 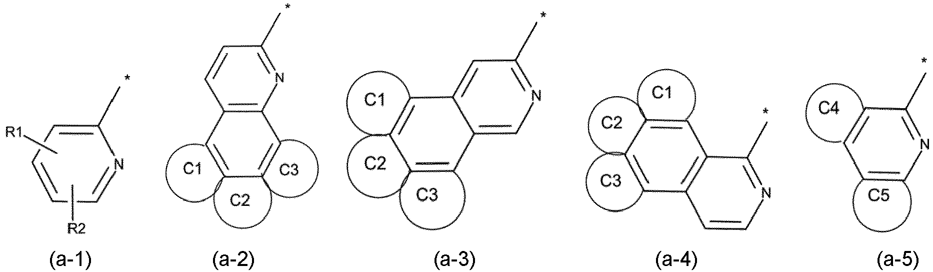 wherein * indicates the binding position;
R¹ and R² independently represent
   - H,
   - halogen,
   - linear or branched C₁-C₃-alkyl,
   - linear or branched C₁-C₃-haloalkyl, or
   - linear or branched C₁-C₃-alkoxy;
in formulae (a-2), (a-3) and (a-4) one of C1, C2 and C3 is present and in formula (a-5) both C4 and C5 are present, and C1, C2, C3, C4 and C5 independently respresent
   - a fused 6-membered aryl ring,
   - a fused 5- or 6-membered heteroaryl ring,
   - a fused 5- or 6-membered cycloalkyl ring, or
   - a fused 5- or 6-membered heterocyclyl ring; and
wherein the groups (a-2), (a-3), (a-4) and (a-5) carry 0, 1, 2 or 3 substituents, which are independently selected from
   - halogen,
   - linear or branched C₁-C₃-alkyl,
   - linear or branched C₁-C₃-haloalkyl, or
   - linear or branched C₁-C₃-alkoxy;
B represents one of the following groups (b-1), (b-2) and (b-3) 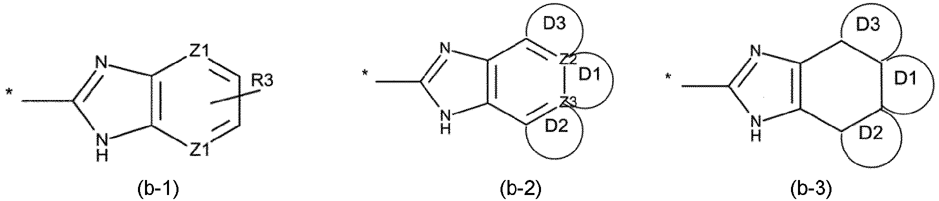
wherein * indicates the binding position;
R³ represents
   - H,
   - unsubstituted or substituted 6-membered aryl,
   - unsubstituted or substituted 5- or 6-membered heteroaryl,
   - unsubstituted or substituted bicyclic heteroaryl,
   - 5- or 6-membered cycloalkyl,
   - 5- or 6-membered heterocyclyl,
   - 5- or 6-membered heterocyclylalkyl,
   - 6-membered arylalkyl,
   - unsubstituted or substituted 6-membered arylalkinyl,
   - unsubstituted or substituted 5- or 6-membered heteroarylalkinyl, or
   - a phenyl group, which forms a fused bicyclic ring with a 5- or 6-membered cycloalkyl- or heterocyclyl group;
Z¹ represents N or C,
with the proviso that not more than one Z¹ represents N;
in formulae (b-2) and (b-3) one of D1, D2 and D3 is present and respresents
   - a fused 6-membered aryl ring,
   - a fused 5- or 6-membered heteroaryl ring,
   - a fused 5- or 6-membered cycloalkyl ring,
   - a fused 5- or 6-membered heterocyclyl ring; and
wherein the groups (b-2) and (b-3) carry 0, 1, 2 or 3 substituents, which are independently selected from
   - halogen,
   - linear or branched C₁-C₃-alkyl,
   - linear or branched C₁-C₃-haloalkyl,
   - linear or branched C₁-C₃-alkoxy;
Z² and Z³ represent N or C,
with the proviso that Z² may represent N when D2 is present and Z³ may represent N when D3 is present;
and wherein the compounds (I) are characterized in that at least one of the groups A and B contains at least 3 rings;
and pharmaceutically acceptable salts thereof.

### DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" or "optional substituent(s)" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3, 4 or 5, in particular 1, 2 or 3.

If used herein, the term "one or more", e.g. in the definition of the substituents of the compounds of general formula (I) of the present invention, means "1, 2, 3, 4 or 5, particularly 1, 2, 3 or 4, more particularly 1, 2 or 3, even more particularly 1 or 2".

The term "comprising" or "containing" when used in the claims or specification includes "consisting of'.

If within the present specification any item is referred to as "as mentioned herein" or "as defined (anywhere) herein", it means that it may be mentioned anywhere in the present specification or may have the meaning as defined anywhere in the present specification.

The terms used in the claims and specification have the following meanings:
"Halogen" or "halogen atom" means a fluorine, chlorine, bromine or iodine atom, particularly a fluorine, chlorine or bromine atom, a preferred selection relates to chlorine or fluorine, a further preferred selection relates to bromine or fluorine, most preferred is fluorine.

The term "C₁-C₃-alkyl" means a linear or branched, saturated, monovalent hydrocarbon group having 1, 2 or 3 carbon atoms, e.g. a methyl, ethyl, n-propyl or isopropyl group.

The C₁-C₃-alkyl group may optionally be substituted with 1 or 2 substituents, preferably with 1 substituent. Such optional substituents are preferably selected from the group consisting of: halogen (forming a halogen-substituted C₁-C₃-alkyl group as defined below and herein also indicated as "C₁-C₃-haloalkyl), an aryl, heteroaryl, cycloalkyl or heterocyclyl ring. Examples include C₃-C₆-cycloalkyl containing preferably 3, 4, 5 or 6 carbon atoms, such as preferably cyclopropyl and cyclohexyl (herein also indicated as "C₁-C₃-cyclylalkyl"), a C₅-C₆-membered heterocyclyl ring such as preferably morpholinyl and piperazinyl (herein also indicated as "heterocyclylalkyl"), an aryl ring such as preferably phenyl (herein also indicated as "arylalkyl"), a C₅-C₆-membered heteroaryl ring (herein also indicated as "heteroarylalkyl").

The term "C₁-C₃-haloalkyl" means a linear or branched, saturated, monovalent C₁-C₃-alkyl group, having the meaning as defined above, in which one or more of the hydrogen atoms are replaced, identically or differently, with a halogen atom. Particularly, said halogen atom is a chlorine or fluorine atom. More particularly, said halogen atom is a fluorine atom and even more particularly, all said halogen atoms are fluorine atoms ("C₁-C₃-fluoroalkyl"). Said C₁-C₃-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl, wherein a trifluoromethyl-group is particularly preferred.

The term "C₁-C₃-alkoxy" means a linear or branched, saturated, monovalent group of formula (C₁-C₃-alkyl)-O-, in which the term "C₁-C₃-alkyl" is as defined *supra, e.g.* a methoxy, ethoxy, *n*-propoxy or isopropoxy group, with a methoxy-group being particularly preferred.

The term "aminocarbonyl group" indicates a group -[NR^{y}-(C=O)-] or in the case of a terminal aminocarbonyl group as a substituent may be represented by a group NR^{y}R^{z}(C=O)-, wherein R^{y} and R^{z} preferably and independently represent H or a C₁-C₃-alkyl group, such as preferably a methyl group, including an aminocarbonyl group, a monoalkyl-aminocarbonyl group and a dialkyl-aminocarbonyl group.

Generally, the term "aryl" includes aromatic hydrocarbon residues containing 6 to 14 carbon atoms (excluding the carbon atoms of the possible substituents), which may be monocyclic or bicyclic, including, for example: phenyl, naphthyl, phenanthrenyl and anthracenyl. Preferred is 6-membered aryl, such as phenyl.

Generally, the term "heteroaryl" includes heteroaromatic hydrocarbon residues containing 4 to 9 ring carbon atoms, which additionally contain 1 to 3 of the same or different heteroatoms selected from S, O and N in the ring, and therefore form 5- to 12-membered heteroaromatic residues which may be monocyclic or bicyclic.

Monocyclic heteroaryl groups preferably include 5- and 6-membered monocyclic heteroaryl groups, such as pyridyl (pyridinyl), pyridyl-N-oxide, pyridazinyl, pyrimidyl, pyrazinyl, thienyl (thiophenyl), furyl, pyrrolyl, pyrazolyl, imidazolyl, triazolyl, tetrazolyl, thiazolyl, isothiazolyl, oxazolyl or isoxazolyl, including from the group of 5-membered heteroaryl, for example thiazolyl such as thiazol-2-yl, 2-thiazol-2-yl, 2-thiazol-4-yl, thienyl (thiophenyl), such as thien-3-yl, pyrazolyl such as 1-pyrazol-4-yl, 3-pyrazol-5-yl, imidazolyl such as imidazole-2-yl, 2-imidazol-4-yl, 1-imidazol-4-yl, triazolyl such as 1-triazol-3-yl, 1-triazol-4-yl, such as 1,2,4-triazol-3-yl or 1,2,3-triazol-4-yl, oxazolyl such as 2-oxazol-4-yl, 2-oxazol-5-yl, oxadiazolyl such as 1,2,4-oxadiazol-3-yl and from the group of 6-membered heteroaryl, for example, pyridyl (pyridinyl) such as pyrid-1-yl, pyrid-2-yl, pyrid-3-yl, pyrid-4-yl, 2-pyrid-4-yl, 2-pyrid-6-yl, 3-pyrid-5-yl (pyridin-1-yl, pyridin-2-yl, pyridin-3-yl, pyridin-4-yl, 2-pyridin-4-yl, 2-pyridin-6-yl, 3-pyridin-5-yl, pyrimidin-2-yl, pyrimidin-4-yl, pyrimidin-5-yl. Preferred heteroaryl groups are pyridinyl, pyrrolyl, oxazolyl and tetrazolyl.

Bicyclic heteroaryl groups preferably include indolizinyl, indolyl, benzo[b]thienyl, benzo[b]furyl, indazolyl, quinolyl, isoquinolyl, naphthyridinyl, quinazolinyl, quinoxalinyl, and benzimidazolyl such as benzimidazol-2-yl, benzimidazol-4-yl, benzimidazol-5-yl. A benzimidazolyl group is particularly preferred.

Generally, the term "cycloalkyl" includes aliphatic rings containing 3 to 8, more preferably 5 or 6 ring carbon atoms. Cycloalkyl includes a cyclopropyl group, a cyclobutyl group, a cyclopentyl group, a cyclohexyl group, a cycloheptyl group and a cyclooctyl group, with a cyclopentyl group and a cyclohexyl group being preferred.

Generally the term "heterocyclyl" includes saturated or unsaturated mono- or bicyclic 4- to 8-membered heterocyclic residues containing 1 to 3, preferably 1 to 2 same or different hetero atoms selected from N, O and S., including azetidinyl, oxetanyl, pyrrolidinyl, pyrazolidinyl, imidazolidinyl, tetrahydrofuranyl, dioxolanyl, tetrahydrothiophenyl, oxathiolanyl, piperidinyl, piperazinyl, tetrahydropyranyl, thianyl, dithianyl, trithianyl, tetrahydrothiopyranyl, morpholinyl, thiomorpholynyl, dioxanyl, etc., such as azetidin-1-yl, azetidin-2-yl, azetidin-3-yl, tetrahydrofuran-2-yl, tetrahydrofuran-3-yl, tetrahydro-thiophen-2-yl, tetrahydro-thiophen-3-yl, pyrrolidin-1-yl, pyrrolidin-2-yl, pyrrolidin-3-yl, morpholin-1-yl, morpholin-2-yl, morpholin-3-yl, piperidin-1-yl, piperidin-2-yl, piperidin-3-yl, piperidin-4-yl, piperazin-1-yl, piperazin-2-yl, tetrahydropyran-2-yl, tetrahydropyran-3-yl, tetrahydropyran-4-yl, etc.. Particularly preferred are dioxolanyl, dioxanyl, piperazinyl and morpholinyl residues.

The aryl, heteroaryl, cycloalkyl or heterocyclyl groups can be bound via a direct bond, a C₁-C₃-alkyl-chain, preferably a C₁-alkyl-chain or an alkinyl-chain, such as preferably an ethinyl-chain (-C≡C-), or the aryl, heteroaryl, cycloalkyl or heterocyclyl groups can be condensed with aromatic rings (preferably with a phenyl ring) forming fused ring systems as defined herein.

The aryl, heteroaryl, cycloalkyl or heterocyclyl group, including fused aryl, heteroaryl, cycloalkyl and heterocyclyl groups, may carry 1, 2 or 3 of the same or different substituents selected from halogen as defined above such as preferably F, Br and Cl, C₁-C₃-alkyl such as preferably methyl, C₁-C₃-haloalkyl as defined above such as preferably trifluoromethyl, and C₁-C₃-alkoxy as defined above such as preferably methoxy.

The aryl, heteroaryl, cycloalkyl or heterocyclyl groups as defined herein in particular form one of the groups A and/or B as defined herein.

Therein, the group A represents one of the following groups (a-1), (a-2), (a-3), (a-4) and (a-5)

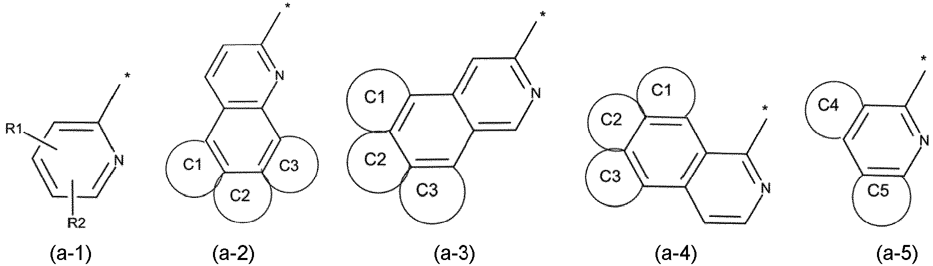

Therein, a group (a-1) is a pyridinyl-group, which carries 0 substituents (R¹/R² represent hydrogen) or 1 or 2 same or different substituents R¹/R², independently selected from halogen as defined above such as preferably F, Br and Cl, C₁-C₃-alkyl such as preferably methyl, C₁-C₃-haloalkyl as defined above such as preferably trifluoromethyl, and C₁-C₃-alkoxy as defined above such as preferably methoxy.

The groups (a-2), (a-3) and (a-4) represent fused (condensed) ring systems, wherein in formulae (a-2), (a-3) and (a-4) a fused aryl, heteroaryl, cycloalkyl or heterocyclyl ring as defined above is present in one of the positions indicated by C1, C2 and C3, preferably forming a fused tricyclic ring system.

The group (a-5) represents a fused (condensed) ring systems, wherein two same or different fused rings selected from aryl, heteroaryl, cycloalkyl and heterocyclyl rings as defined above are present in the positions indicated by C4 and C5.

The group B represents one of the following groups (b-1), (b-2) and (b-3)

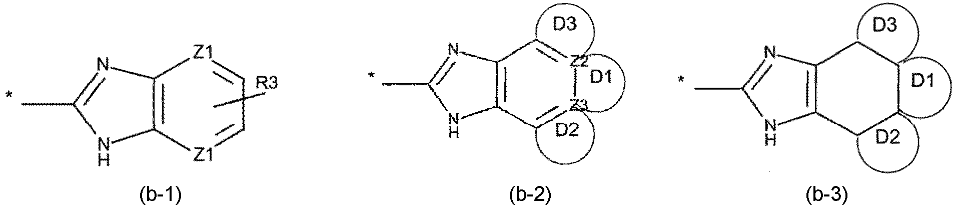

Therein, a group (b-1) may represent a benzimidazolyl group (Z¹ = C) or an azabenzimidazole group (one Z¹ = N), which carries 0 substituents (R³ represents hydrogen) or 1 or 2 same or different substituents, preferably 1 substituent, R³ selected from an aryl, heteroaryl, cycloalkyl, heterocyclyl group and a fused bicyclic ring system, including a phenyl ring carrying a fused cycloalkyl or heterocyclyl ring, each as defined above, which is bound via a direct bond, corresponding to R³ representing an aryl group, a (mono- or bicyclic) heteroaryl group, a cycloalkyl group, a heterocyclyl group or a fused bicyclic ring system, including a phenyl ring carrying a fused cycloalkyl or heterocyclyl ring, or which is bound via a C₁-C₃-alkyl-chain, preferably a C₁-alkyl-chain or an alkinyl-chain, such as preferably an ethinyl-chain. If Z¹ represents N, then under the proviso that not more than one Z¹ represents N.

An aryl, heteroaryl, cycloalkyl or heterocyclyl ring bound via a C₁-C₃-alkyl-chain, preferably a C₁-alkyl-chain, corresponds to R³ representing an arylalkyl, a heteroarylalkyl, a cycloalkylalkyl or a heterocyclylalkyl group, wherein "alkyl" preferably represents C₁-C₃-alkyl. An aryl group, such as a phenyl group, and a heterocyclylalkyl group, such as a piperazinylmethyl group or a morpholinylmethyl group, are preferred.

An aryl, heteroaryl, cycloalkyl or heterocyclyl ring bound via an alkinyl-chain, such as preferably an ethinyl-chain, corresponds to R³ representing an arylalkinyl, a heteroarylalkinyl, a cycloalkylalkinyl or a heterocyclylalkinyl group, wherein "alkinyl" preferably represents ethinyl. An arylalkinyl group, such as a phenylethinyl group, and a heteroarylalkinyl group, such as a pyridinylethinyl group, are preferred. Therein the aryl, heteroaryl, cycloalkyl or heterocyclyl ring may carry 1, 2 or 3 of the same or different substituents selected from halogen as defined above such as preferably F, Br and Cl, C₁-C₃-alkyl such as preferably methyl, C₁-C₃-haloalkyl as defined above such as preferably trifluoromethyl, C₁-C₃-alkoxy as defined above such as preferably methoxy, an aminocarbonyl group as defined above such as preferably a dimethylaminocarbonyl group, and a 5- or 6-membered heteroaryl group as defined above such as preferably an oxazolyl group.

Such groups are also designated herein as "unsubstituted or substituted arylalkinyl", "unsubstituted or substituted heteroarylalkinyl", "unsubstituted or substituted cycloalkylalkinyl" and "unsubstituted or substituted heterocyclylalkinyl".

The groups (b-2) and (b-3) represent fused (condensed) ring systems, wherein in formulae (b-2) and (b-3) a fused aryl, heteroaryl, cycloalkyl or heterocyclyl ring as defined above is present in one of the positions indicated by D1, D2 and D3, preferably forming a fused tricyclic ring system. In formula (b-2) preferably both of Z²/Z³ represent C. If one of Z² or Z³ is N, then under the proviso that Z² may represent N when D2 is present and Z³ may represent N when D3 is present. It is also possible that D1, D2 or D3 is present and both Z²/Z³ represent C.

The groups (a-2), (a-3), (a-4) and (a-5), (b-2) and (b-3) may optionally carry 1, 2 or 3, preferably 1 or 2, same or different substituents, which are independently selected from halogen such as preferably Cl or F, linear or branched C₁-C₃-alkyl such as preferably methyl or ethyl, linear or branched C₁-C₃-haloalkyl such as preferably trifluoromethyl, linear or branched C₁-C₃-alkoxy such as preferably methoxy, an alkoxyalkylether group such as preferably a methoxyethylether group [-O-CH₂-CH₂-O-CH₃], an aminocarbonyl group such as preferably a dimethylaminocarbonyl group, and a 5- or 6-membered heteroaryl group such as preferably an oxazolyl group, each as defined above. Such optional substituent is hereinafter also represented by R^{x}. The formulae below include possible unsubstituted groups, wherein R^{x} is absent or corresponds to hydrogen.

From the group (a-1) an unsubstituted pyridinyl group (R¹ and R² represent hydrogen) and the following substituted pyridinyl groups are particularly preferred:

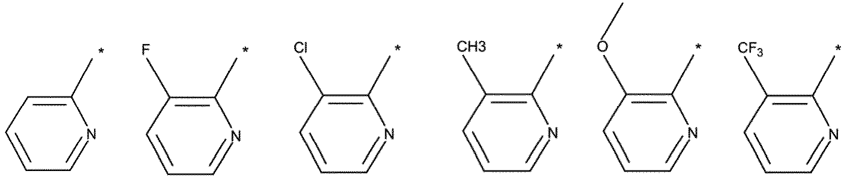

From the group (a-2) the following group is particularly preferred:

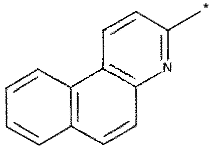

From the group (a-3) the following group is particularly preferred:

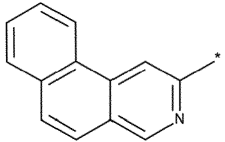

From the group (a-5) the following group is particularly preferred:

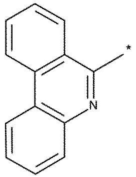

From the group (b-1) the following groups are particularly preferred, wherein R^{x} may be absent (i.e. represent hydrogen) or may have the meaning as defined above:

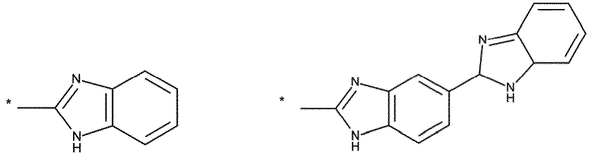

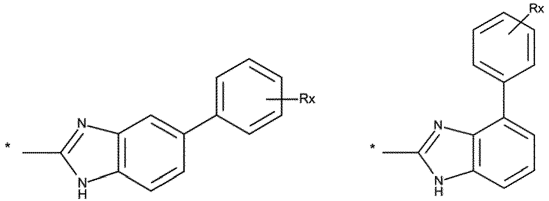

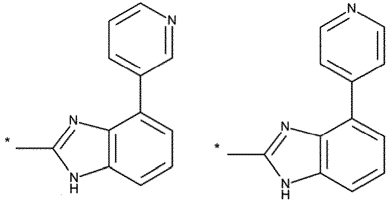

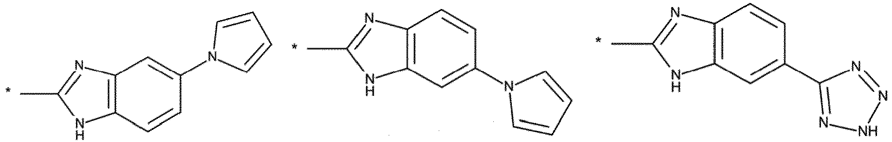

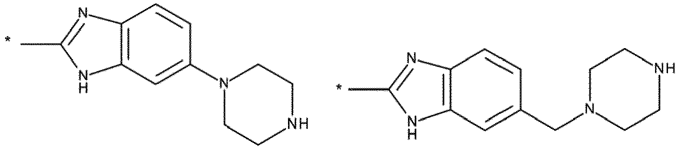

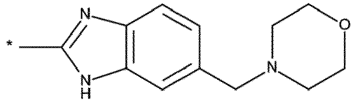

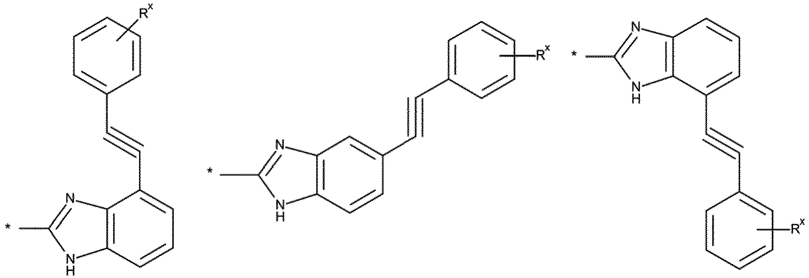

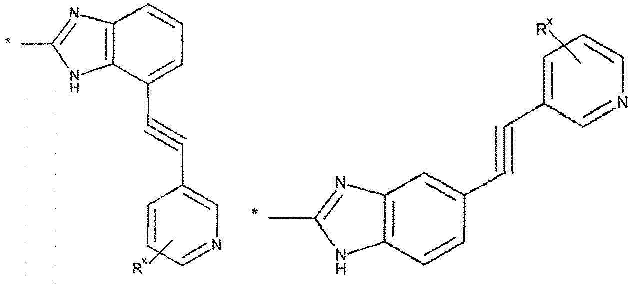

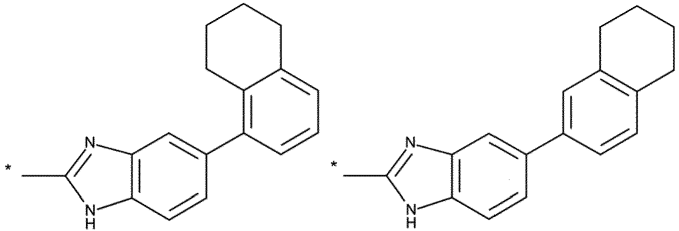

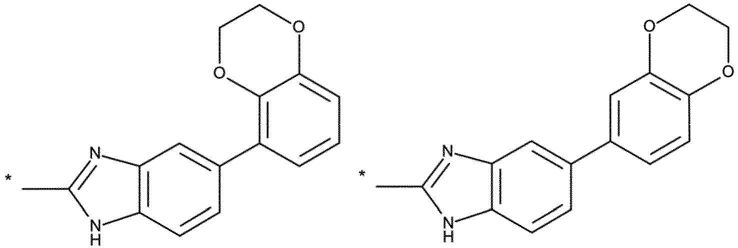

From the group (b-2) the following groups are particularly preferred:

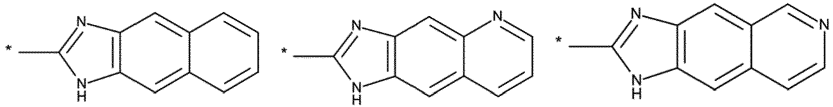

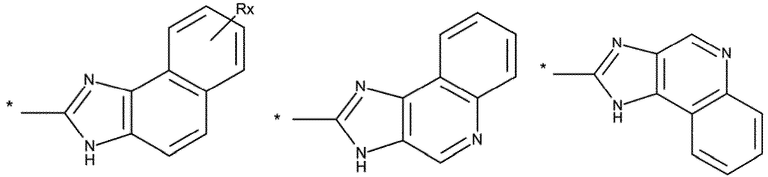

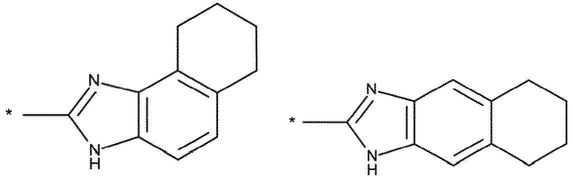

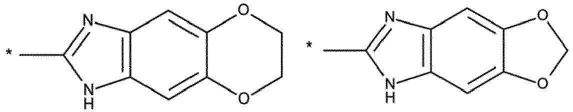

From the group (b-3) the following groups are particularly preferred:

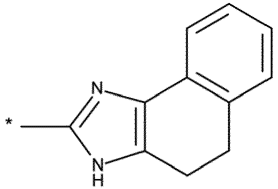

The compounds of the formula (I) of the present invention are characterized in that at least one of the groups A and B contains at least 3 (three) rings. This means, that one or both of the groups A and B represent a ring system of at least 3 fused rings, wherein the individual fused rings may independently be selected from aryl, heteroaryl, cycloalkyl and heterocyclyl as defined above. The expression "at least one of the groups A and B contains at least 3 rings" also includes compounds wherein A is represented by a group (a-1) and B is represented by a group (b-1), provided that in such cases R³ does not represent hydrogen but an (substituted or unsubstituted) aryl, heteroaryl, cycloalkyl or heterocyclyl ring, or a fused bicyclic ring system, including a phenyl ring carrying a fused cycloalkyl or heterocyclyl ring as defined above, which is bound via a direct bond or a C₁-C₃-alkyl-chain or an alkinyl-chain as defined above. This means, that the expression "at least one of the groups A and B contains at least 3 rings" is not limited to fused tricyclic ring systems.

In the formulae (a-1), (a-2), (a-3), (a-4), (a-5), (b-1), (b-2) and (b-3) "*" indicates the binding position.

In the formula (I) "I" represents an integer of 1 or 2, preferably I = 1.

In the formula (I) "m" and "n" independently represents an integer of 1, 2 or 3, preferably m = 2 and preferably n = 1 or 2.

In the formula (I) X¹, X² and X³ are selected from:
X¹ = N, S or O;
X² = N, S, O or CR⁴;
X³ = C or N;
with the proviso that one of X¹ and X² is N and if X³ is N, then X² is CR⁴, forming one of the following groups: 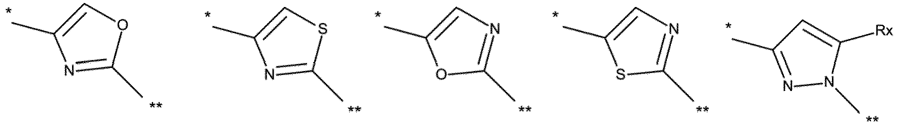 wherein * indicates the binding site to the aminocarbonyl-group and ** indicates the binding site to the - [(CH₂)]ₘ-amino-[(CH₂)]ₙ- group in the formula (I).

R⁴ represents an optional substituent selected from halogen, linear or branched C₁-C₃-alkyl and linear or branched C₁-C₃-haloalkyl, each as defined above. If no substituent is present, then R⁴ represents hydrogen.

Preferred are an oxazole-, an isooxazole-, a thiazole- and an isothiazole-group: 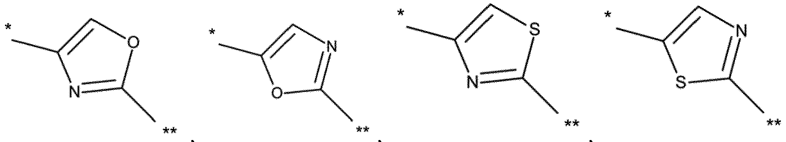 with an oxazole- and isooxazole-group being more preferred.

A further aspect relates to compounds of the formula (I) as defined above, wherein
I is an integer of 1 or 2;
m and n are independently an integer of 1, 2 or 3;
X¹ is N, S or O;
X² is N, S, O or CR⁴;
X³ is C or N;
with the proviso that one of X¹ and X² is N
and if X² is N, then X² is CR⁴; and wherein
R⁴ represents H;
A represents one of the groups (a-1), (a-2), (a-3), (a-4) and (a-5) as defined above;
R¹ and R² independently represent
   - hydrogen
   - halogen,
   - linear or branched C₁-C₃-alkyl,
   - linear or branched C₁-C₃-haloalkyl,
   - linear or branched C₁-C₃-alkoxy; each as defined above
in formulae (a-2), (a-3) and (a-4) one of C1, C2 and C3 is present and in formula (a-5) both C4 and C5 are present, and C1, C2, C3, C4 and C5 respresent a fused phenyl ring; and
the groups (a-2), (a-3), (a-4) and (a-5) carry 0 or 1 substituent, which is selected from
   - halogen,
   - linear or branched C₁-C₃-alkyl,
   - linear or branched C₁-C₃-haloalkyl,
   - linear or branched C₁-C₃-alkoxy; each as defined above
B represents one of the following groups (b-1), (b-2) and (b-3) as defined above;
R³ represents
   - H,
   - unsubstituted or substituted phenyl,
   - unsubstituted or substituted 5- or 6-membered heteroaryl,
   - unsubstituted or substituted bicyclic heteroaryl,
   - 6-membered heterocyclyl,
   - 6-membered heterocyclylalkyl,
   - phenylalkyl,
   - unsubstituted or substituted phenylethinyl or unsubstituted or substituted 6-membered heteroarylethinyl, or
   - a phenyl group, which forms a fused bicyclic ring with a 5- or 6-membered cycloalkyl- or heterocyclyl group;
   each as defined above and wherein with respect to the possible substituents reference is made to possible substituents of aryl, heteroaryl, cycloalkyl and heterocyclyl and the respective specific groups as defined above;
Z¹ represents N or C,
with the proviso that not more than one Z¹ represents N;
in formulae (b-2) and (b-3) one of D1, D2 and D3 is present and respresents
   - a fused phenyl ring,
   - a fused 6-membered heteroaryl ring,
   - a fused 6-membered cycloalkyl ring,
   - a fused 5- or 6-membered heterocyclyl ring; and
wherein the groups (b-2) and (b-3) carry 0 or 1 substituent, which is selected from
   - halogen,
   - linear or branched C₁-C₃-alkyl,
   - linear or branched C₁-C₃-haloalkyl,
   - linear or branched C₁-C₃-alkoxy; each as defined above
Z² and Z³ represent N or C,
with the proviso that Z² may represent N when D2 is present and Z³ may represent N when D3 is present; and
wherein the compounds (I) are characterized in that at least one of the groups A and B contains at least 3 rings;
and pharmaceutically acceptable salts thereof.

In a further aspect of the invention it is preferred that the compounds of the formula (I) as defined anywhere herein are characterized in that one of the groups A and B contains 3 rings.

In a further aspect of the invention it is preferred that the group A of the compounds of the formula (I) as defined anywhere herein is a group (a-1).

In a further aspect of the invention it is preferred that the group A of the compounds of the formula (I) as defined anywhere herein is a group (a-2), (a-3) or (a-5).

In a further aspect of the invention it is preferred that the group A of the compounds of the formula (I) as defined anywhere herein is a group (a-5).

In a further aspect of the invention it is preferred that the group B of the compounds of the formula (I) as defined anywhere herein is a group (b-1) or (b-2).

In a further aspect of the invention it is preferred that possible substituents of groups of the compounds of the formula (I) as defined anywhere herein are selected from the following groups
- halogen substituents represent F, Cl and Br, preferably F or Br, alternatively F or Cl, most preferred F;
- linear or branched C₁-C₃-alkyl substituents represent methyl or ethyl, most preferred is methyl,
- linear or branched C₁-C₃-haloalkyl substituents represent trifluoromethyl (CF₃),
- linear or branched C₁-C₃-alkoxy substituents represent methoxy.

In a further aspect of the invention it is preferred that for the group A of the compounds of the formula (I) as defined anywhere herein the following among groups (a-1), (a-2), (a-3) and (a-5) are selected:

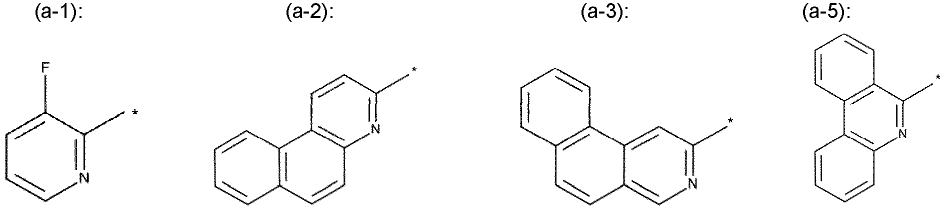

In a further aspect of the invention it is preferred that for the group A of the compounds of the formula (I) as defined anywhere herein the following among groups (a-1), (a-2), (a-3) and (a-5) are selected:
In a particularly preferred aspect the compounds according to formula (I) as defined anywhere herein are selected from the following compounds:

| **No.** | Chemical Structure |
|---|---|
| **1** | 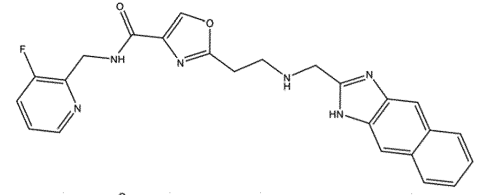 |
| **2** | 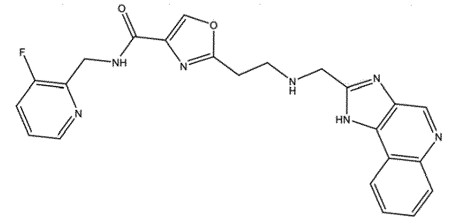 |
| **3** | 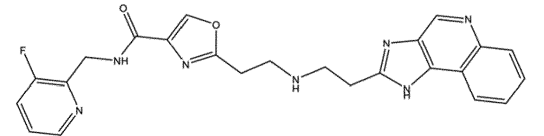 |
| **4** | 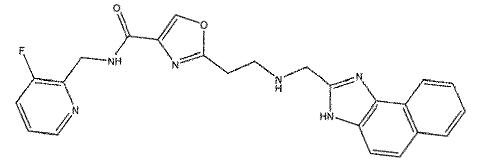 |
| **5** | 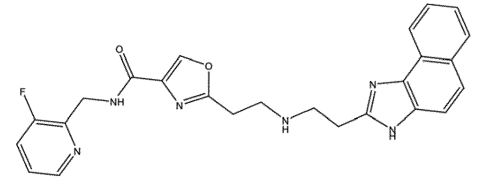 |
| **6** | 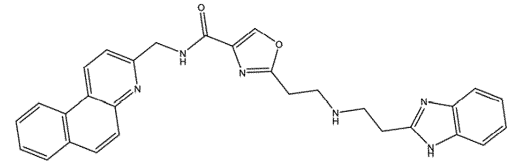 |
| **7** | 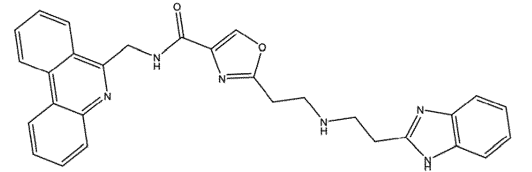 |
| **8** | 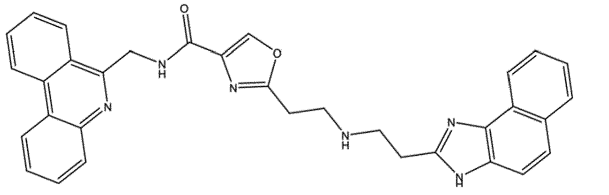 |
| **9** | 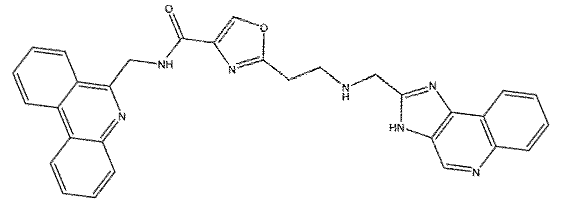 |
| **10** | 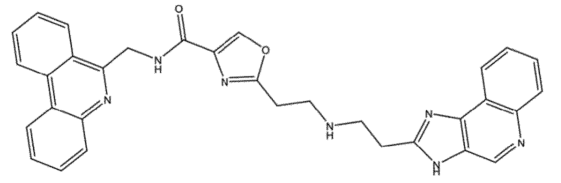 |
| **11** | 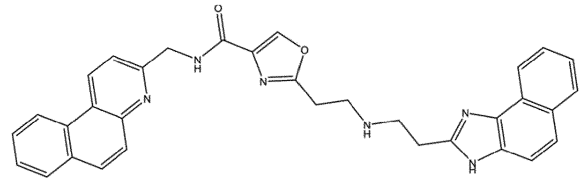 |
| **12** | 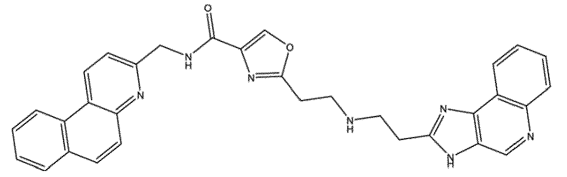 |
| **13** | 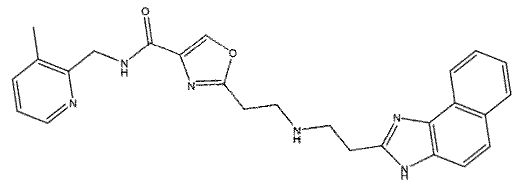 |
| **14** | 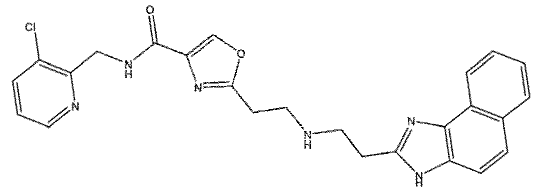 |
| **15** | 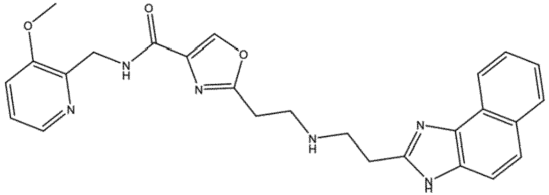 |
| **16** | 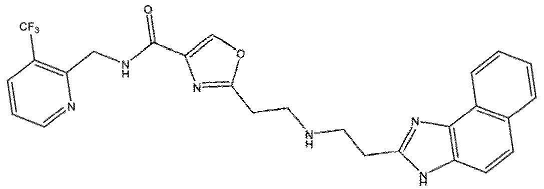 |
| **17** | 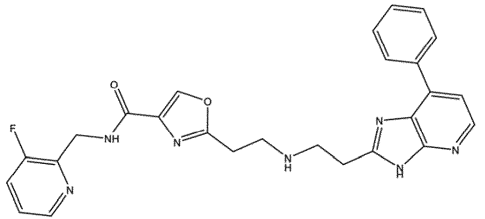 |
| **18** | 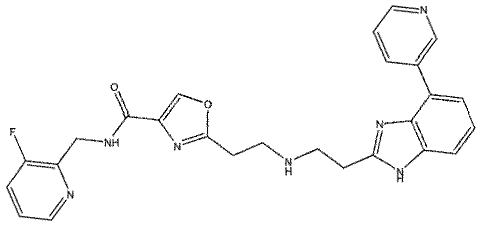 |
| **19** | 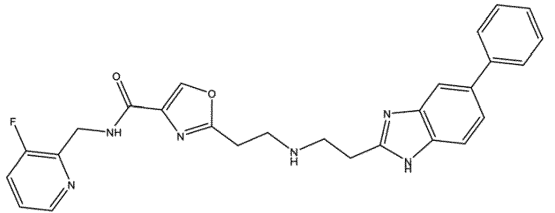 |
| **20** | 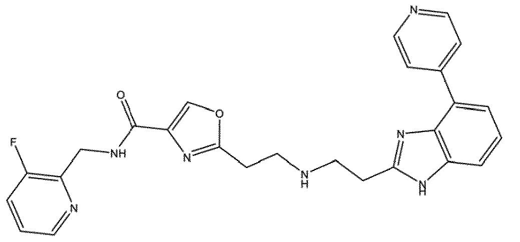 |
| **21** | 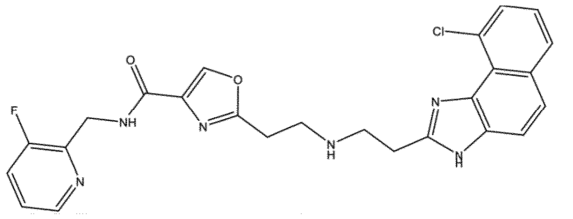 |
| **22** | 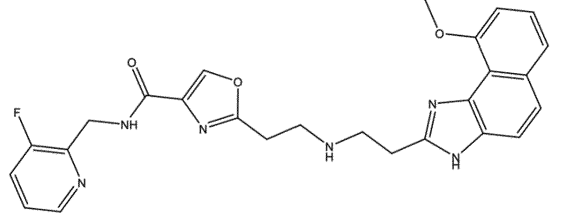 |
| **23** | 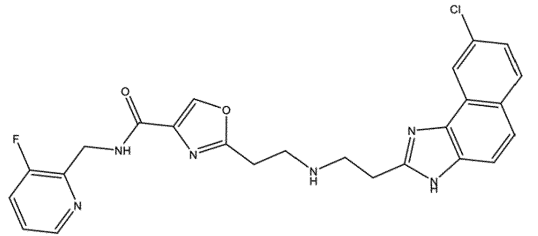 |
| **24** | 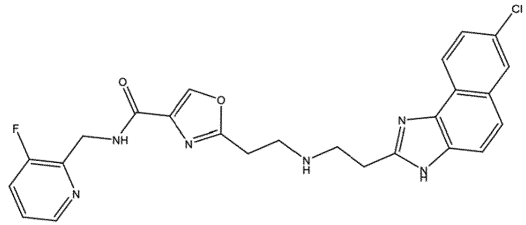 |
| **25** | 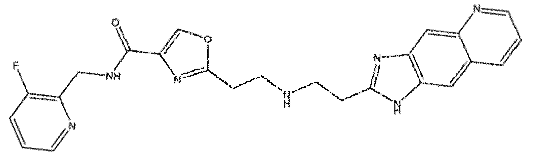 |
| **26** | 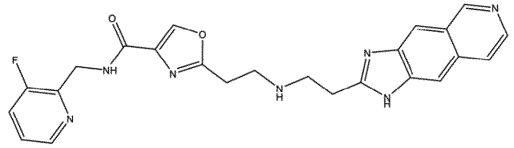 |
| **27** | 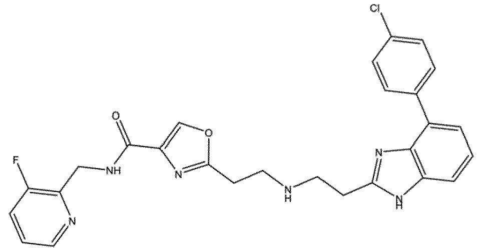 |
| **28** | 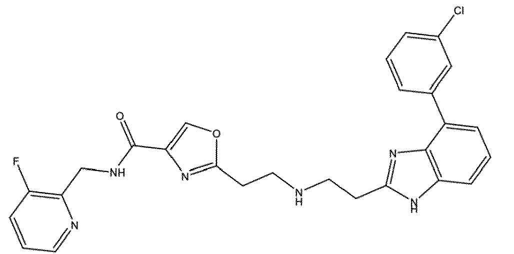 |
| **29** | 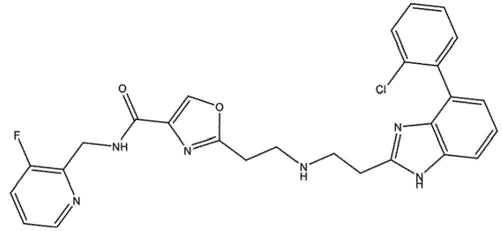 |
| **30** | 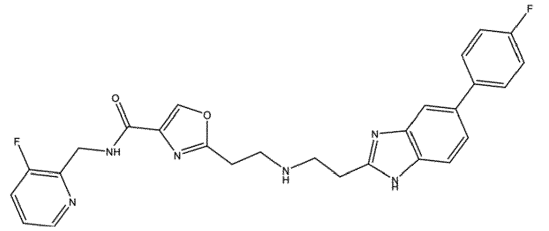 |
| **31** | 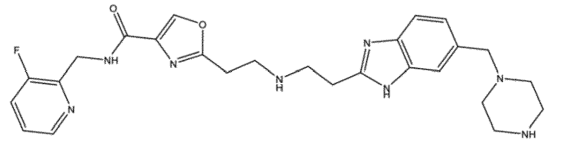 |
| **32** | 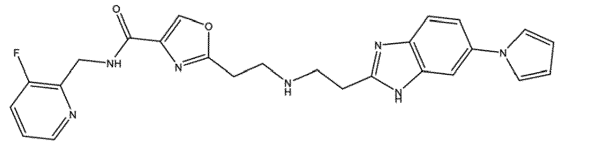 |
| **33** | 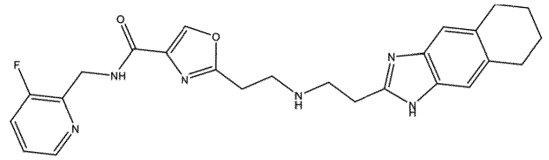 |
| **34** | 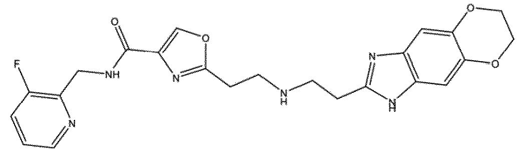 |
| **35** | 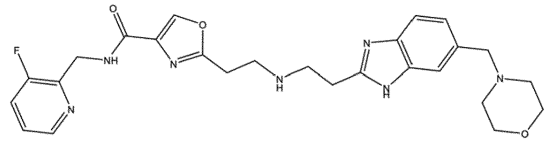 |
| **36** | 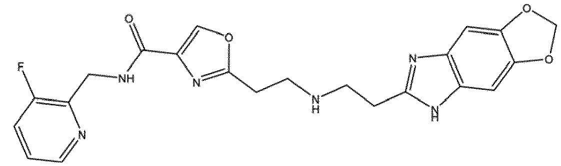 |
| **37** | 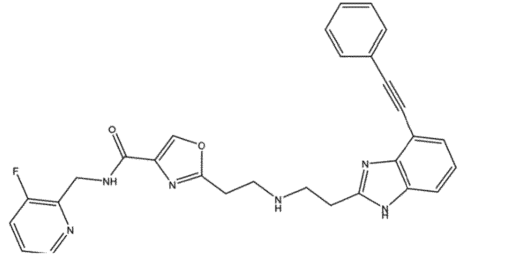 |
| **38** | 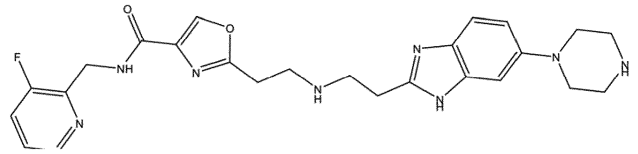 |
| **39** | 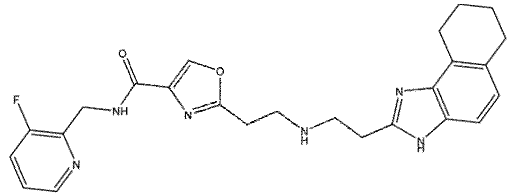 |
| **40** | 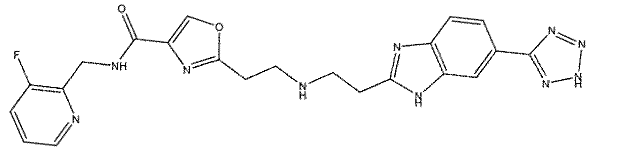 |
| **41** | 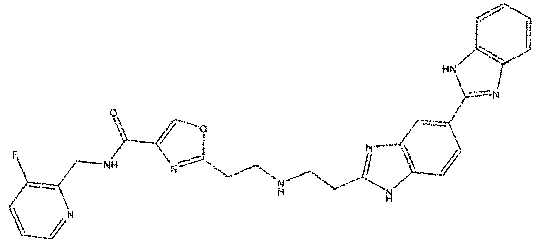 |
| **42** | 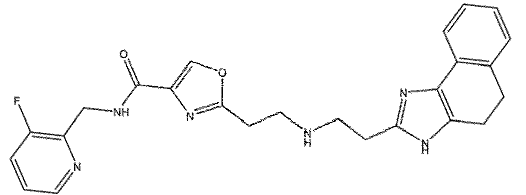 |
| **43** | 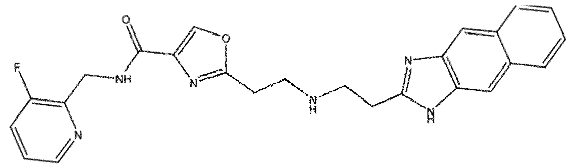 |
| **44** | 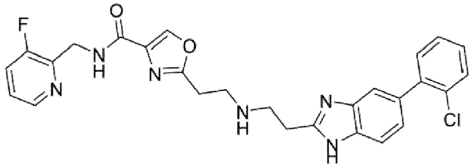 |
| **45** | 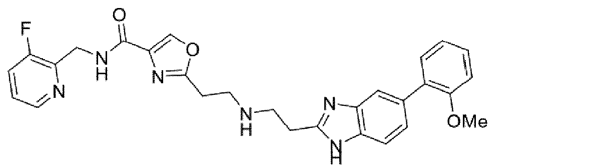 |
| **46** | 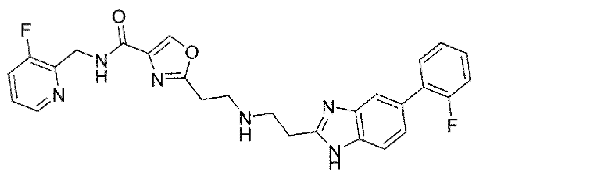 |
| **47** | 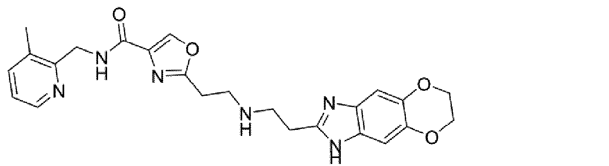 |
| **48** | 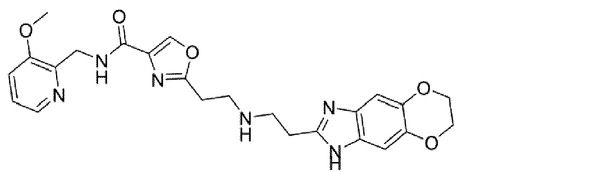 |
| **49** | 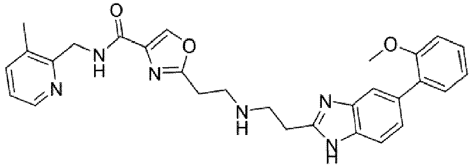 |
| **50** | 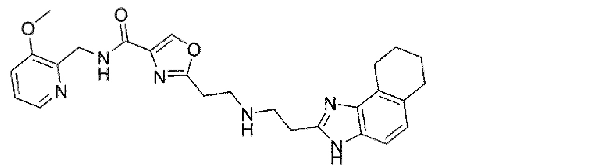 |
| **51** | 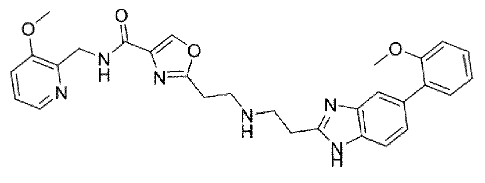 |
| **52** | 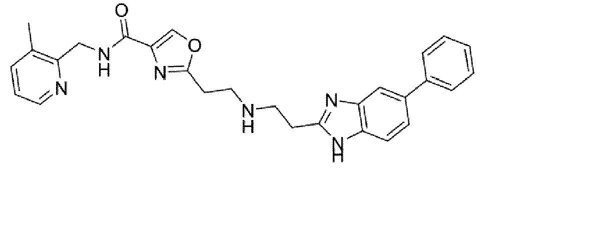 |
| **53** | 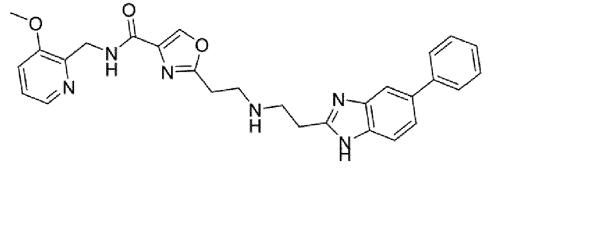 |
| **54** | 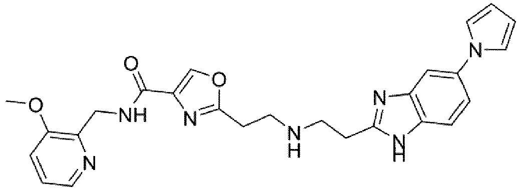 |
| **55** | 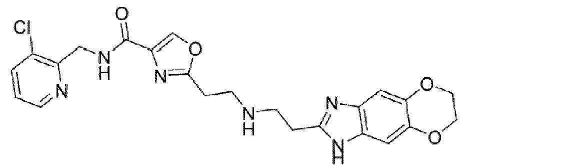 |
| **56** | 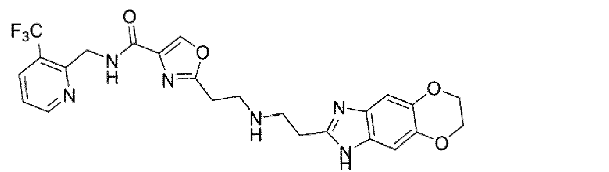 |
| **57** | 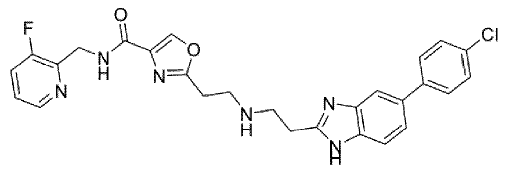 |
| **58** | 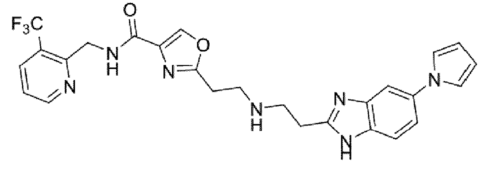 |
| **59** | 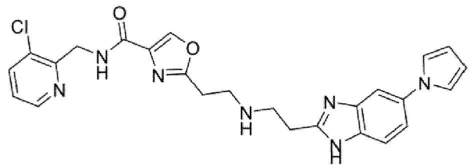 |
| **60** | 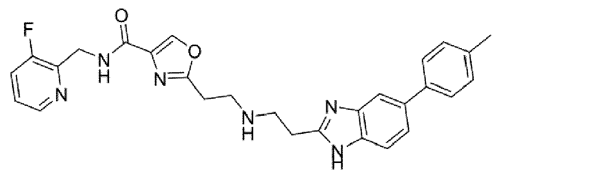 |
| **61** | 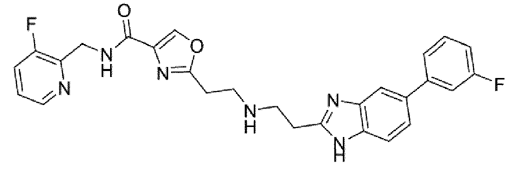 |
| **62** | 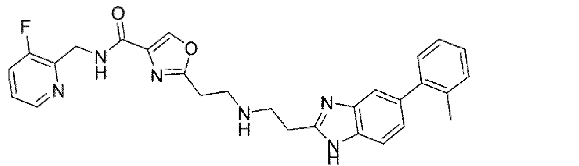 |
| **63** | 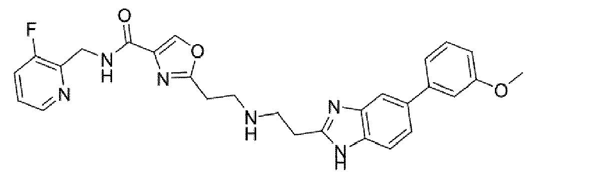 |
| **64** | 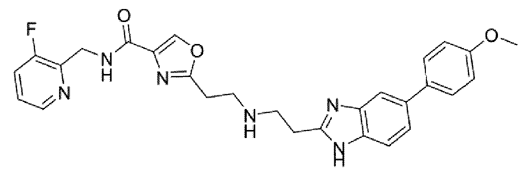 |
| **65** | 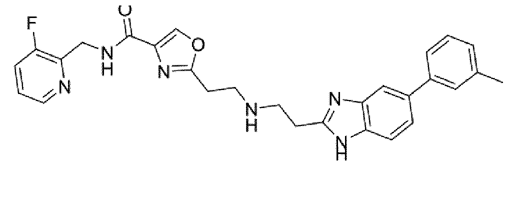 |
| **66** | 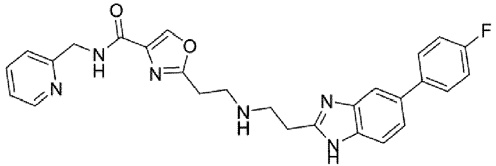 |
| **68** | 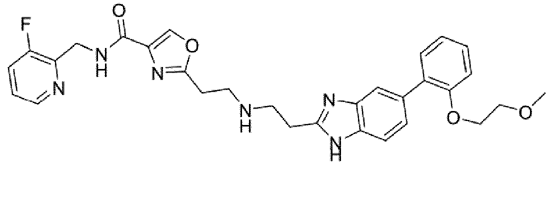 |
| **69** | 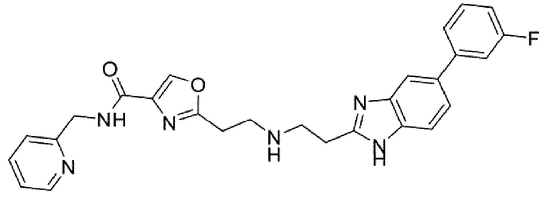 |
| **70** | 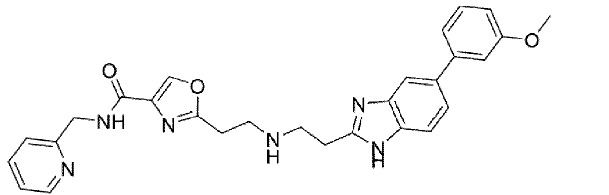 |
| **71** | 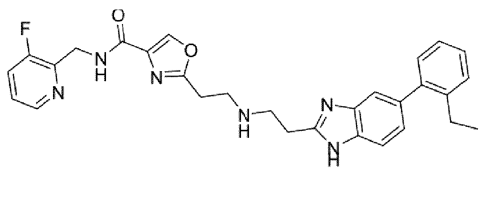 |
| **72** | 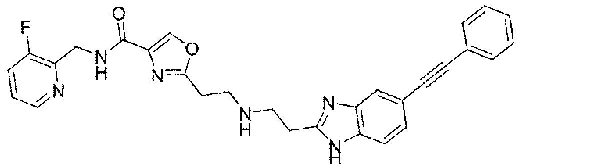 |
| **73** | 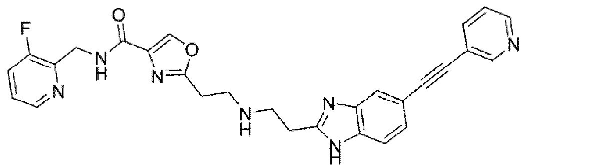 |
| **74** | 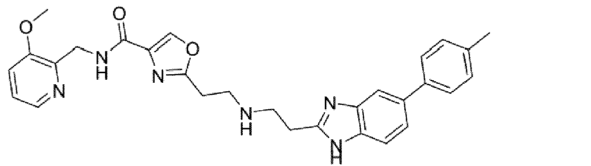 |
| **75** | 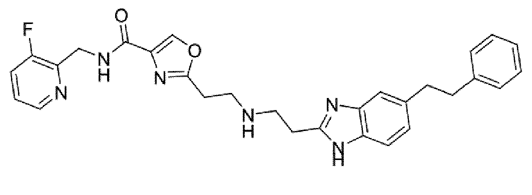 |
| **76** | 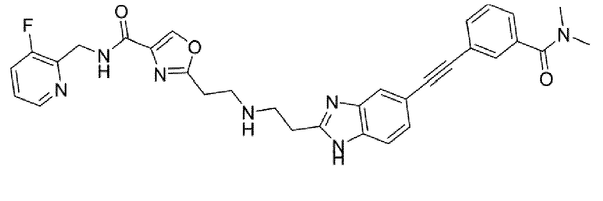 |
| **78** | 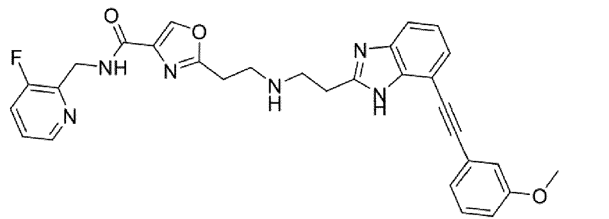 |
| **79** | 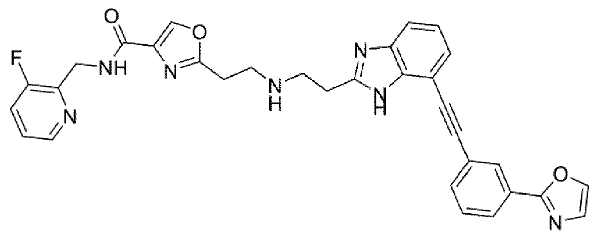 |
| **80** | 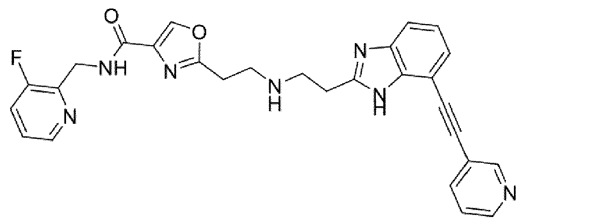 |
| **81** | 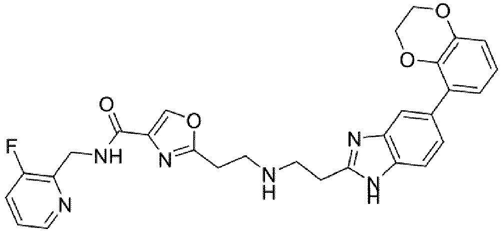 |
| **82** | 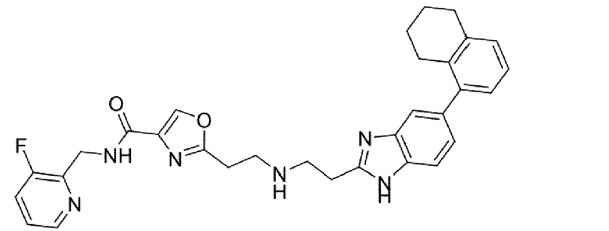 |
| **83** | 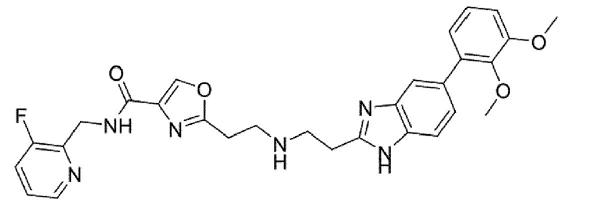 |
| **84** | 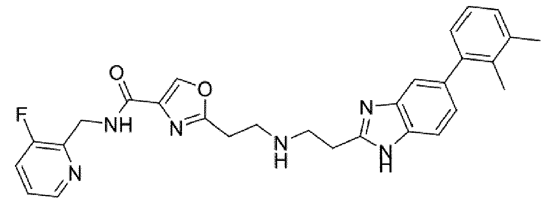 |
| **85** | 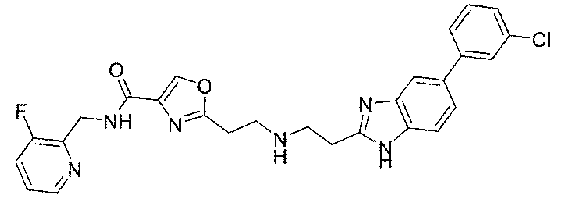 |
| **86** | 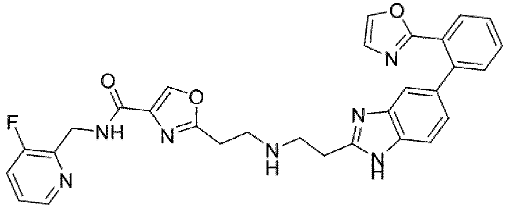 |
| **87** | 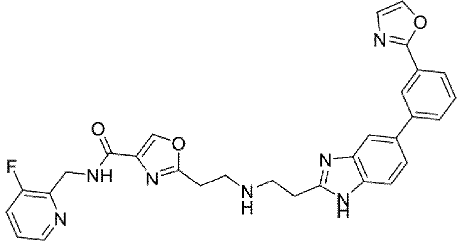 |
| **88** | 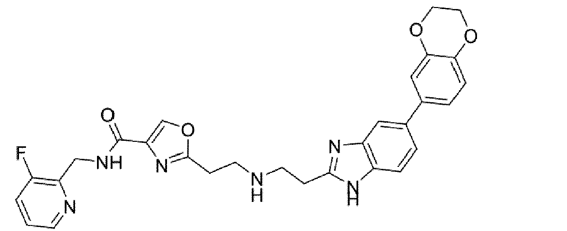 |
| **89** | 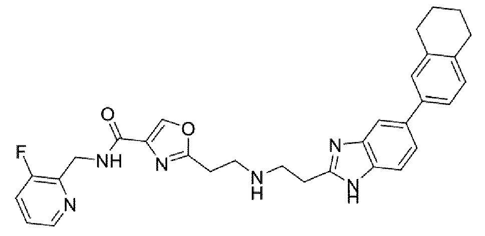 |
| **90** | 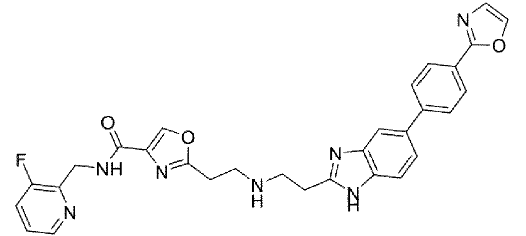 |

Pharmaceutically acceptable salts of the compounds according to the invention include, for example, salts with suitable anions, such as carboxylates, sulfonates, sulfates, chlorides, bromides, iodides, phosphates, tartrates, methane sulfonates, hydroxyethane sulfonates, glycinates, maleates, propionates, fumarates, toluene sulfonates, benzene sulfonates, trifluoroacetates, naphthalenedisulfonates-1,5, salicylates, benzoates, lactates, salts of malic acid, salts of 3-hydroxy-2-naphthoic acid-2, citrates and acetates.

Pharmaceutically acceptable salts of the compounds according to the invention further include, for example, salts with suitable pharmaceutically acceptable bases, such as, for example, salts with alkaline or alkaline-earth hydroxides, such as NaOH, KOH, Ca(OH)₂, Mg(OH)₂ etc., amine compounds such as ethylamine, diethylamine, triethylamine, ethyldiisopropylamine, ethanolamine, diethanolamine, triethanolamine, methylglucamine, dicyclohexylamine, dimethylaminoethanol, procaine, dibenzylamine, N-methylmorpholine, arginine, lysine, ethylenediamine, N-methylpiperidin, 2-amino-2-methyl-propanol-(1), 2-amino-2-methyl-propandiol-(1,3), 2-amino-2-hydroxyl-methyl-propandiol-(1,3) (TRIS) etc..

The novel compounds of the present invention can be present in an amorphous, crystalline or partially crystalline form or they may also be present exist as hydrates.

The novel compounds according to formula (I) as defined anywhere herein, have surprisingly been found to act as ferroportin inhibitors and are thus suitable for the use as a medicament, such as in particular for the use as ferroportin inhibitors.

As already explained above, ferroportin is the iron transport protein, which is responsible for the uptake of the released iron via the intestine and its transfer into the blood circulation, thereby conveying the iron to the appropriate tissues and organs. Inactivation or inhibition of the ferroportin disables the export of the iron, thereby reducing the absorption of iron in the intestine. Ferroportin inhibition in the sense of the present invention therefore includes the inhibition of iron transport from the cells into the blood circulation and the inhibition of iron absorption in the intestine. Therein, the inhibition of iron transport and/or iron reflux may be effected by different ways of mechanism, comprising for example inhibition of iron transport activity of ferroportin and thus inhibition of iron reflux, triggering internalization, degradation and/or reduction of ferroportin, administering hepcidin agonists, i.e. compounds which compete with hepcidin or by compounds, which inhibit the binding of hepcidin to ferroportin.

Ferroportin inhibition may be determined by measuring the inhibition of ferroportin mediated iron transport activity in an iron response assay (BLAzer-Assay), as described in more detail in the Examples below. Further, ferroportin inhibition may be determined by measuring ferroportin internalization and/or degradation in the Ferroportin Internalization and Degradation Assay (FACS) or by examining the Ferroportin Ubiquitination and Degradation, each as described in more detail in the Examples below. Further, ferroportin inhibition may be determined by measuring the activity as an hepcidin agonist, for example by determining the Hepcidin binding capacity to ferroportin in the Hepcidin Internalization Assay (J774), as described in more detail in the Examples below. Further, ferroportin inhibition may be determined by confirming the inhibition of hepcidin binding to ferroportin, for example in the Biophysical Ferroportin-Hepcidin Binding Assay (Hep Bind FP), as described in more detail in the Examples below. Further, ferroportin inhibition may be determined by determining the activity of a compound regarding its ability to block iron export via ferroportin, for example with a test for measuring inhibition of iron efflux, as described in more detail in the Examples below.

Ferroportin inhibition in the sense of the present invention can thus in particular be defined by exhibiting a ferroportin inhibiting activity in at least one of the aforementioned test methods, shown in particular by:
Inhibition of ferroportin mediated iron transport activity in an iron response assay (Blazer Assay): IC₅₀ value [µM] of not more than 100 (≤ 100), preferably not more than 50 (≤ 50), more preferably below 50 (< 50).

Ferroportin Internalization and Degradation Assay (FACS): EC₅₀ value [µM] of not more than 100 (≤ 100), preferably not more than 50 (≤ 50), more preferably below 50 (< 50).

Ferroportin Ubiquitination and Degradation: visually inspected effect in Western blots of "+ comparable to hepcidin", "+/- intermediate effect" and "+ / +/- stronger intermediate effect", preferred is an effect "+" or "+ / + / -", most preferred is an effect "+" .

Hepcidin Internalization Assay (J774): IC₅₀ value [µM] of not more than 100 (≤ 100), preferably not more than 50 (≤ 50), more preferably below 50 (< 50).

Biophysical Ferroportin-Hepcidin Binding Assay: IC₅₀ value [µM] of not more than 100 (≤ 100), preferably not more than 50 (≤ 50), more preferably below 50 (< 50).

Inhibition of Iron Efflux: IC₅₀ value of not more than 100 (≤ 100), preferably not more than 50 (≤ 50), more preferably below 50 (< 50).

Ferroportin inhibition may further be determined in *in vivo* models, as described in more detail in the Examples below. Suitable *in vivo* models may comprise, for example, examination of hypoferremia in naïve mice via measurement of serum iron reduction; examination of prevention of iron absorption in anemic rats via measurement of serum iron inhibition; examination of correction of hyperferremia in beta2-microglobulin deficient mice via measurement of serum iron reduction; examination of prevention of iron overload in beta2-microglobulin deficient mice via measurement of total iron in spleen or liver; examination of improvement of anemia, ineffective erythropoiesis and iron overload in a mouse model of β-thalassemia intermedia.

The activity of the compounds of the present invention as ferroportin inhibitors can in particular be determined by the methods as described in the Examples below.

As further already explained above, ferroportin inhibition may for example be effected by hepcidin, which is thus an essential regulating factor of iron absorption, inhibiting ferroportin and thus blocking iron transport from the cells into the blood circulation and iron absorption. It has further surprisingly been found that several of the compounds as defined herein act as hepcidin mimetics or hepcidin agonists, which is also included by ferroportin inhibition in the sense of the present invention.

Accordingly, the compounds as defined in the present invention are also suitable for use in the inhibition of iron transport from the cells into the blood circulation and the inhibition of iron absorption in the intestine, as well as for the use as hepcidin mimetics or hepcidin agonists.

Due to the activity of the compounds as defined herein as ferroportin inhibitors, the compounds of the present invention are further particularly suitable for the use in the inhibition of iron transport mediated by ferroportin and thereby for the use in the prophylaxis and/or treatment of iron metabolism disorders leading to increased iron levels, of diseases related to or caused by increased iron levels, increased iron absorption or iron overload, such as in particular of tissue iron overload, of diseases associated with ineffective erythropoiesis, or of diseases caused by reduced levels of hepcidin. Further, the compounds of the present invention are suitable for the use in an adjunctive therapy by limiting the amount of iron available to pathogenic microorganisms, such as the bacterium Vibrio vulnificus, thereby preventing or treating infections caused by said pathogenic microorganisms.

Therein, diseases being associated with, being related to, being caused by or leading to increased iron levels, increased iron absorption, iron overload (e.g. tissue iron overload) or ineffective erythropoiesis comprise thalassemia, hemoglobinopathy, such as hemoglobin E disease (HbE), hemoglobin H disease (HbH), haemochromatosis, hemolytic anemia, such as sickle cell anemia (sickle cell disease) and congenital dyserythropoietic anemia.

Diseases being associated with, being related to, being caused by or leading to increased iron levels, increased iron absorption, iron overload (e.g. tissue iron overload) further comprise neurodegenerative diseases, such as for example Alzheimer's disease and Parkinson's disease, wherein the compounds are considered to be effective by limiting the deposition or increase of iron in tissue or cells.

The compounds of the present invention are further suitable for the use in the prophylaxis and/or treatment of formation of radicals, reactive oxygen species (ROS) and oxidative stress caused by excess iron or iron overload as well as in the prophylaxis and/or treatment of cardiac, liver and endocrine damage caused by excess iron or iron overload, and further in the prophylaxis and/or treatment of inflammation triggered by excess iron or iron overload.

Diseases associated with ineffective erythropoiesis comprise in particular myelodysplastic syndromes (MDS, myelodysplasia) and congenital dyserythropoietic anemia, as well as myeloproliferative neoplasms, such as polycythemia vera.

Further diseases, disorders and/or diseased conditions comprise iron overload caused by mutations in genes involved in sensing the systemic iron stores, such as hepcidin (Hamp1), hemochromatosis protein (HFE), hemojuvelin (HJV) and transferrin receptor 2 (TFR2), such as in particular diseases related to HFE and HJV gene mutations, chronic hemolysis associated diseases, sickle cell diseases, red cell membrane disorders, Glucose-6-phosphate dehydrogenase deficiency (G6PD deficiency), erythrpoietic porphyria, Friedrich's Ataxia, as well as subgroups of iron overload such as transfusional iron overload, iron intoxication, pulmonary hemosiderosis, osteopenia, insulin resistense, African iron overload, Hallervordan Spatz disease, hyperferritinemia, ceruloplasmin deficiency, neonatal hemochromatosis and red blood cell disorders comprising thalassemia, including alpha thalassemia, beta thalassemia and delta thalassemia, thalassemia intermedia, sickle cell disease and myelodyplastic syndrome.

Further diseases and/or disorders and/or diseased conditions associated with elevated iron levels include, but are not limited to, diseases with elevated iron level, comprising ataxia, Friedrich's ataxia, age-related macular degeneration, age-related cataract, age-related retinal diseases and neurodegenrative disease, such as pantothenate kinase-associated neurodegeneration, restless leg syndrom and Huntington's disease,

The compounds of the present invention my further be suitable for the use in the prophylaxis and treatment of diseases caused by a lack of hepcidin.

In view thereof a further object of the present invention relates to a medicament containing one or more of the compounds as defined above, such as in particular a medicament for the prophylaxis and treatment in any of the indications, states, disorders or diseases as defined above.

A further object of the present invention relates to pharmaceutical compositions and medicaments comprising one or more of the compounds according to the invention as defined above as well as optionally one or more pharmacologically acceptable carriers and/or auxiliary substances and/or solvents. A further object of the present invention relates to pharmaceutical compositions and medicaments comprising one or more of the compounds according to the invention as defined above as well as optionally one or more further pharmaceutically effective compounds. The said pharmaceutical compositions contain, for example up to 99 weight-% or up to 90 weight-% or up to 80 weight-% or or up to 70 weight-% of the compounds of the invention, the remainder being each formed by pharmacologically acceptable carriers and/or auxiliaries and/or solvents and/or optionally further pharmaceutically active compounds.

Therein, the pharmaceutically acceptable carriers, auxiliary substances or solvents are common pharmaceutical carriers, auxiliary substances or solvents, including various organic or inorganic carrier and/or auxiliary materials as they are customarily used for pharmaceutical purposes, in particular for solid medicament formulations. Examples include excipients, such as saccharose, starch, mannitol, sorbitol, lactose, glucose, cellulose, talcum, calcium phosphate, calcium carbonate; binding agents, such as cellulose, methylcellulose, hydroxypropylcellulose, polypropyl pyrrolidone, gelatine, gum arabic, polyethylene glycol, saccharose, starch; disintegrating agents, such as starch, hydrolyzed starch, carboxymethylcellulose, calcium salt of carboxymethylcellulose, hydroxypropyl starch, sodium glycol starch, sodium bicarbonate, calcium phosphate, calcium citrate; lubricants, such as magnesium stearate, talcum, sodium laurylsulfate; flavorants, such as citric acid, menthol, glycin, orange powder; preserving agents, such as sodium benzoate, sodium bisulfite, paraben (for example methylparaben, ethylparaben, propylparaben, butylparaben); stabilizers, such as citric acid, sodium citrate, acetic acid and multicarboxylic acids from the titriplex series, such as, for example, diethylenetriaminepentaacetic acid (DTPA); suspending agents, such as methycellulose, polyvinyl pyrrolidone, aluminum stearate; dispersing agents; diluting agents, such as water, organic solvents; waxes, fats and oils, such as beeswax, cocoa butter; polyethylene glycol; white petrolatum; etc..

Liquid medicament formulations, such as solutions, suspensions and gels usually contain liquid carrier, such as water and/or pharmaceutically acceptable organic solvents. Furthermore, such liquid formulations can also contain pH-adjusting agents, emulsifiers or dispersing agents, buffering agents, preserving agents, wetting agents, gelatinizing agents (for example methylcellulose), dyes and/or flavouring agents, for example as defined above. The compositions may be isotonic, that is, they can have the same osmotic pressure as blood. The isotonicity of the composition can be adjusted by using sodium chloride and other pharmaceutically acceptable agents, such as, for example, dextrose, maltose, boric acid, sodium tartrate, propylene glycol and other inorganic or organic soluble substances. The viscosity of the liquid compositions can be adjusted by means of a pharmaceutically acceptable thickening agent, such as methylcellulose. Other suitable thickening agents include, for example, xanthan gum, carboxymethylcellulose, hydroxypropylcellulose, carbomer and the like. The preferred concentration of the thickening agent will depend on the agent selected.

Pharmaceutically acceptable preserving agents can be used in order to increase the storage life of the liquid composition. Benzyl alcohol can be suitable, even though a plurality of preserving agents including, for example, paraben, thimerosal, chlorobutanol and benzalkonium chloride can also be used.

The above-mentioned pharmaceutical compositions are suitable, for example, for intravenous, intraperitoneal, intramuscular, intravaginal, intrabuccal, percutaneous, subcutaneous, mucocutaneous, oral, rectal, transdermal, topical, intradermal, intragasteral or intracutaneous application and are provided, for example, in the form of pills, tablets, enteric-coated tablets, film tablets, layer tablets, sustained release formulations for oral, subcutaneous or cutaneous administration (in particular as a plaster), depot formulations, dragees, suppositories, gels, salves, syrup, granulates, suppositories, emulsions, dispersions, microcapsules, microformulations, nanoformulations, liposomal formulations, capsules, enteric-coated capsules, powders, inhalation powders, microcrystalline formulations, inhalation sprays, epipastics, drops, nose drops, nose sprays, aerosols, ampoules, solutions, juices, suspensions, infusion solutions or injection solutions etc..

A further object of the present invention relates to medicaments or combined preparations containing one or more of the compounds as defined above and at least one further pharmaceutically active compound, such as in particular a compound for the prophylaxis and treatment of iron overload and the associated symptoms, preferably an iron-chelating compound, or a compound for the prophylaxis and treatment of any of the states, disorders or diseases as defined above, such as in particular a pharmaceutically active compound for the prophylaxis and treatment of thalassemia, haemochromatosis, neurodegenerative diseases (such as Alzheimer's disease or Parkinson's disease) and the associated symptoms.

A further object of the present invention relates to the use of the compounds as defined above per se, in a combination therapy (fixed dose or free dose combinations for sequential use) with one or two other active ingredients (drugs). Such combination therapy comprises co-administration of the compounds of the present invention with the at least one additional pharmaceutically active compound (drug). Combination therapy in a fixed dose combination therapy comprises co-administration of the compounds of the present invention with the at least one additional pharmaceutically active compound in a fixed-dose formulation. Combination therapy in a free dose combination therapy comprises co-administration of the compounds of the present invention and the at least one additional pharmaceutically active compound in free doses of the respective compounds, either by simultaneous administration of the individual compounds or by sequential use of the individual compounds distributed over a time period. The at least one additional pharmaceutically active compound (drug) comprises in particular drugs for reducing iron overload (e.g. Tmprss6-ASO) or iron chelators, in particular curcumin, SSP-004184, Deferitrin, deferasirox, deferoxamine and/or deferiprone, or antioxidants such as n-acetyl cysteine, anti-diabetics such as GLP-1 receptor agonists, antibiotics such as vancomycin (Van) or tobramycin, drugs for the treatment of malaria, anticancer agents, antifungal drugs, drugs for the treatment of neurodegenerative diseases such as Alzheimer's disease and Parkinson's disease (e.g. dopamine agonists such as Levodopa), anti-viral drugs such as interferon-a or ribavirin, or immunosuppressents (cyclosporine A or cyclosporine A derivatives), iron supplements, vitamin supplements, red cell production stimulators, anti-inflammatory biologies, anti-thrombolytics, statins, vasopressors and inotropic compounds .

A further object of the present invention relates to the use of the above combinations for the prophylaxis and/or treatment of diseases caused by a lack of hepcidin or iron metabolism disorders, such as particularly iron overload states such as in particular thalassemia and hemochromatosis and other disorders as described in the present application.
A further object of the present invention relates to the use of the compounds as defined herein per se or the hereinabove described combination therapies, in combination with Blood transfusion.

The compounds, medicaments and or combined preparations according to the present invention may be administered orally, parentally, as well as intravenously.

For this purpose, the compounds according to the invention are preferably provided in medicaments or pharmaceutical compositions in the form of pills, tablets, such as enteric-coated tablets, film tablets and layer tablets, sustained release formulations for oral administration, depot formulations, dragees, granulates, emulsions, dispersions, microcapsules, microformulations, nanoformulations, liposomal formulations, capsules, such as enteric-coated capsules, powders, microcrystalline formulations, epipastics, drops, ampoules, solutions, suspensions, infusion solutions or injection solutions or in the form of a preparation suitable for inhalation.

In a preferred embodiment of the invention the compounds are administered in the form of a tablet or capsule, as defined above. These may be present, for example, as acid resistant forms or with pH dependent coatings.

The compounds of the present invention as the active substance can be administered, for example, with a unit dose of 0.001 mg/kg to 500 mg/kg body weight, for example 1 to 4 times a day. However, the dose can be increased or reduced depending on the age, weight, condition of the patient, severity of the disease or type of administration.

Accordingly, a further object of the present invention relates to compounds, medicaments, compositions and combined preparations as defined above for the preparation of a medicament, particularly for the prophylaxis and treatment of any indication, state, disorder or disease as defined above, in particular for oral or parenteral administration.

A further object of the present invention relates to a method for the prophylaxis and treatment as defined above, such as in particular for the prophylaxis and/or treatment of iron metabolism disorders being associated with or leading to increased iron levels and in particular iron overload, diseases related to or caused by increased iron levels or iron overload, iron storage diseases being associated with or leading to increased iron levels, and diseases being associated with ineffective erythropoiesis, the method comprising administering, to a patient (human or animal) in need thereof, a compound, a medicament, a composition or a combined preparation as defined above.

Therein, diseases being associated with, being related to, being caused by or leading to increased iron levels or iron overload are as defined above.

A further object of the present invention relates to the use of the compounds as defined above for the preparation of a medicament, particularly for the prophylaxis and treatment and of any indication, state, disorder or disease as defined above.

The compounds according to the invention of general structural formula (I) can basically be prepared by the processes described in the unpublished international application PCT/EP2021/057424, herein incorporated by reference.

In particular, the following general procedure describes a suitable preparation process:

### Step 1:

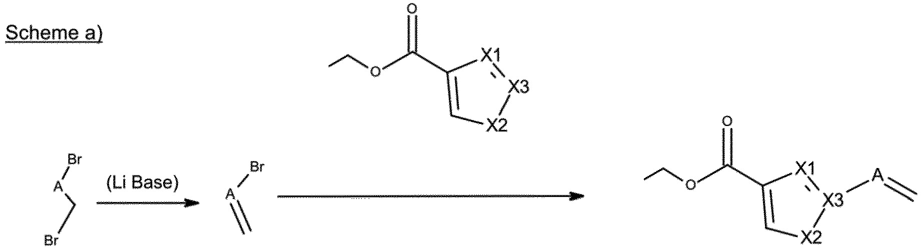

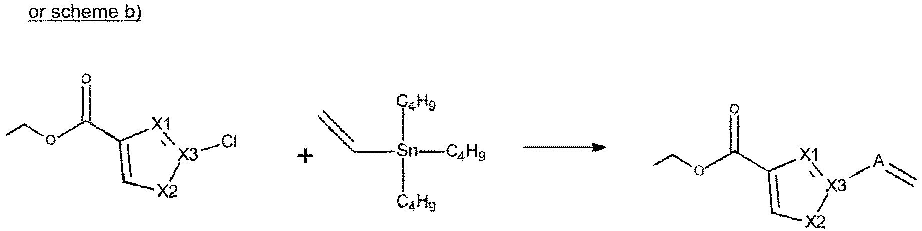

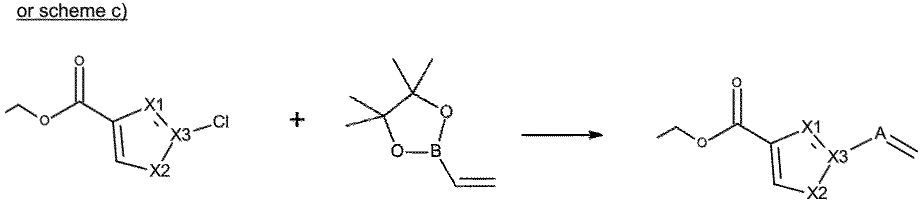

followed by Ester Cleavage:

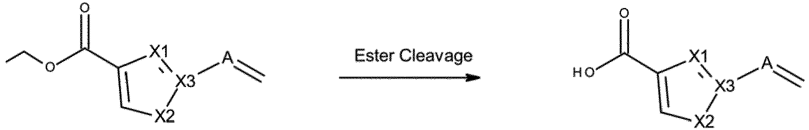

### Step 2:

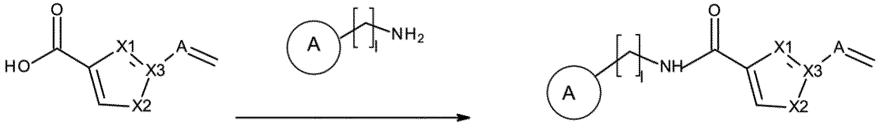

### Step 3:

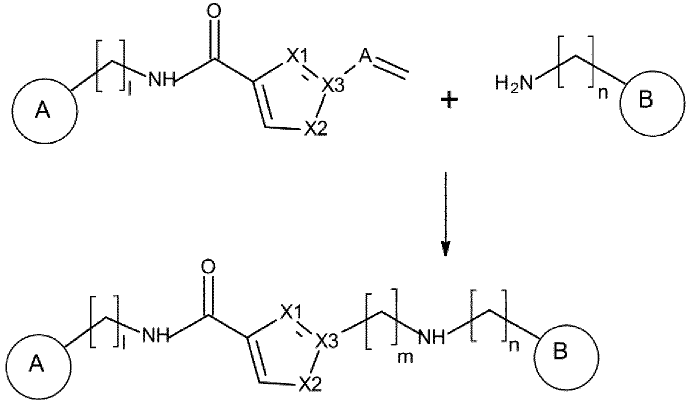

wherein X¹, X², X³ and the groups A and B, as well as I, m and n have the meaning as defined anywhere herein.

In a further aspect the invention covers the intermediate compounds obtainable in the preparation methods described herein, such as in particular the intermediate compounds resulting from the individual steps of the general reaction scheme above and as described further in detail herein. Details of the preparation conditions provide the Examles below.

### EXAMPLES

The invention is illustrated in more detail by the following examples. The examples are merely explanatory, and the person skilled in the art can extend the specific examples to further claimed compounds.

### Pharmacological Assays

### 1. Hepcidin Internalization Assay (J774)

This cellular assay allows quantification of the binding of hepcidin to ferroportin (Fpn) through microscopic detection of internalization of a fluorescently labeled hepcidin into J774 cells. J774 is a mouse macrophage cell line which was shown to express Fpn endogenously upon incubation with iron (Knutson et al, 2005). Binding of hepcidin to Fpn triggers internalization and degradation of both hepcidin and Fpn. However, the TMR (6-carboxytetramethylrhodamine) fluorophore attached to hepcidin remains associated with the cell after degradation of the hepcidin peptide backbone. Therefore, microscopic detection of cell-associated TMR fluorescence is a measure of hepcidin binding to Fpn and internalization of hepcidin and Fpn. If TMR-hepcidin is prevented from binding to Fpn, cellular TMR fluorescence remains low (Dürrenberger et al, 2013). The effect of small molecular weight Fpn inhibitor compounds in this assay was evaluated in vitro as described below.

J774 cells, harvested from ca. 80% confluent cultures, were plated at 8×10⁵ cells/ml in complete medium (DMEM, 10% FBS, 1% Penicillin-Streptomycin) containing 200 µM Fe(III)NTA (nitrilotriacetic acid), 100 µl per well of 96 well MicroClear plates (Greiner; Cat. 655090) and grown at 37°C with 5% CO₂. After overnight incubation, cells were washed 3 times with pre-warmed DMEM w/o phenol red, 30 µl/well of DMEM w/o phenol red was added after the final wash and 10 µl/well of dilution series of test compounds were added in triplicates. J774 cells were pre-incubated with test compounds at 37°C with 5% CO₂ for 15 min. before TMR-hepcidin was added at 25 nM final concentration. Cells were incubated in a total volume of 50 µl at 37°C with 5% CO₂ for 2 hours, then Hoechst 33342 dye was added to a final concentration of 0.5 µg/ml to stain nuclei and further incubated for 10 min. at 37°C with 5% CO₂. Cells were washed 3 times with PBS and fixed in 100 µl of 4% paraformaldehyde in PBS for 15 min. at room temperature. After removal of the paraformaldehyde solution, cells were washed 3 times with PBS leaving 100 µl per well and the plates were sealed with foil plate seal. TMR (530-550 nm excitation / 575-625 nm emission / 400 ms exposure time) and Hoechst 33342 (360-370 nm excitation / 420-460 nm emission / 10 ms exposure time) fluorescence images were acquired using a ScanR plate imager (Olympus) with a 20x high NA objective. Four pictures were acquired per well and fluorescence channel covering ca. 1500 cells per well. The acquired image data was analysed with the ScanR image analysis software. Image analysis included detection of nuclei (Hoechst 33342 fluorescence), identification of cell-associated regions, application of a virtual channel and thresholding for rolling-ball-type background reduction, followed by application of the Sum(Mean) algorithm to measure the TMR fluorescence associated with cells as a quantitative measure for internalized TMR-hepcidin. IC₅₀ values were calculated with the Sum(Mean) raw data using "log(inhibitor) vs. response" curve fitting of Prism 5 software (GraphPad Software Inc., version 5.02). For each data set the fit of the "log(inhibitor) vs. response (three parameters)" model was compared to the fit of the "log(inhibitor) vs. response - Variable slope (four parameters)" model and the IC₅₀ data of the preferred model was used. IC₅₀ data of the Fpn inhibitors that were tested in the hepcidin internalization assay are listed in Table1. The IC₅₀ of unlabeled hepcidin in this assay is 0.015 ± 0.011 µM.

**Table 1 Average (AVE) IC₅₀ data of Fpn inhibitors tested in the hepcidin internalization assay is shown for multiple measurements**

| **Table 1** | | | | | | | |
|---|---|---|---|---|---|---|---|
| **Exp. No.** | **IC 50 [uM]** | **Exp. No.** | **IC 50 [uM]** | **Exp. No.** | **IC 50 [uM]** | **Exp. No.** | **IC 50 [uM]** |
| **1** | 0.04 | **32** | 0.027 | **54** | 0.037 | **73** | 0.002 |
| **2** | 0.35 | **34** | 0.007 | **55** | 0.002 | **74** | 0.007 |
| **3** | 0.593 | **35** | 0.057 | **56** | 0.049 | **75** | 0.002 |
| **4** | 0.068 | **36** | 0.011 | **57** | 0.027 | **76** | 0.003 |
| **5** | 0.013 | **37** | 0.034 | **58** | 0.171 | **78** | 0.041 |
| **6** | 1.60 | **39** | 0.012 | **59** | 0.028 | **79** | 0.084 |
| **7** | 1.59 | **40** | 1.04 | **60** | 0.013 | **80** | 0.083 |
| **8** | 6.70 | **43** | 0.009 | **61** | 0.011 | **81** | 0.007 |
| **11** | 3.90 | **44** | 0.006 | **62** | 0.008 | **82** | 0.015 |
| **12** | 13.9 | **45** | 0.006 | **63** | 0.008 | **83** | 0.006 |
| **13** | 0.055 | **46** | 0.0070 | **64** | 0.010 | **84** | 0.012 |
| **14** | 0.033 | **47** | 0.011 | **65** | 0.015 | **85** | 0.011 |
| **15** | 0.033 | **48** | 0.026 | **66** | 0.068 | **86** | 0.009 |
| **16** | 0.443 | **49** | 0.018 | **68** | 0.007 | **87** | 0.014 |
| **18** | 0.053 | **50** | 0.019 | **69** | 0.061 | **88** | 0.012 |
| **19** | 0.006 | **51** | 0.017 | **70** | 0.026 | **89** | 0.010 |
| **20** | 0.189 | **52** | 0.026 | **71** | 0.006 | **90** | 0.0058 |
| **28** | 0.144 | **53** | 0.016 | **72** | 0.002 | | |

### 2. Biophysical Ferroportin-Hepcidin Binding Assay

This biophysical assay was developed to confirm inhibition of hepcidin binding to ferroportin (Fpn) more directly. Incubation of TMR-hepcidin with purified human Fpn isolated from Pichia pastoris yeast cells expressing human Fpn with a C-terminal FLAG affinity tag (Bonaccorsi di Patti, 2014) leads to increased fluorescence polarization (FP) of the TMR-hepcidin ligand. Small molecular weight Fpn inhibitors are tested for inhibition of binding of TMR-hepcidin to Fpn, as detected by dose-dependent decrease of the TMR FP signal, as described in detail below.

A mixture of 1.3 µM human Fpn and 30 nM TMR-hepcidin in FP assay buffer containing 50 mM Tris-HCl pH 7.3, 200 mM NaCl, 0.02% DDM, 0.1% BSA is plated into a 384 well black low volume round bottom plate (Corning, Cat. 3677) at 16 µl per well. 8 µl of serial dilutions of test compounds are added in duplicates to reach final Fpn and TMR-hepcidin concentrations of 1 µM and 20 nM, respectively. Plates are incubated for 90 minutes at room temperature and parallel (S) and perpendicular (P) fluorescence is measured in a Synergy H1 fluorescence reader (BioTek). FP values are calculated in mP according to the following formula. $mP = \frac{{F}_{parallel} - {F}_{perpendicular}}{{F}_{parallel} + {F}_{perpendicular}} \times 1000$

IC₅₀ values are determined with the calculated mP values as described for the hepcidin internalization assay. The IC₅₀ of unlabeled hepcidin in this assay is about 0.37 ± 0.067 µM.

### 3. Inhibition of Ferroportin mediated Iron Export Activity in an Iron Response Assay

Intracellular iron levels are indirectly measured in this assay by monitoring the activity of a beta-lactamase (BLA) reporter gene fused to the human ferritin promoter and the associated iron regulatory element (IRE) contained within the 5' untranslated region of the ferritin mRNA. Expression of ferroportin (Fpn) in such a cell line leads to iron efflux and lower iron levels as reflected by lower activity of the reporter gene. On the other hand, inhibition of Fpn-mediated iron efflux results in elevated cellular iron levels which is detected as increased reporter gene activity. Small molecular weight Fpn inhibitor compounds are tested for dose-dependent effects in this in vitro iron response assay as described below.

The HEK-293 cell line #354 is generated by stable integration of (i) a human Fpn-GFP fusion construct inserted in a derivative of the doxycycline-inducible pTRE-Tight-BI plasmid (Clontech, Cat. 631068) and (ii) a human ferritin promoter-BLA reporter gene into a derivative of the HEK-293 Tet-ON Advanced cell line (Clontech). To generate the ferritin-BLA reporter gene construct, a 1.4 kb fragment of the human ferritin H promoter is amplified by PCR from human genomic DNA (forward primer 5'-CAGGTTTGTGAGCATCCTGAA-3'; reverse primer 5'-GGCGGCGACTAAGGAGAGG-3') and inserted in front of the BLA gene present in the pcDNA^{™}6.2/cGeneBLAzer^{™}-DEST plasmid (Invitrogen, Cat. 12578-043) thereby replacing the original CMV promoter and placing the IRE that regulates translation of the ferritin gene ca. 170 bp upstream of the start codon of the reporter gene. #354 cells are harvested from ca. 80% confluent cultures, seeded at 1.8×10⁵ cells/ml in DMEM/F12 GlutaMAX^{™} medium (Invitrogen, Cat. 31331-028) containing 10% FBS (Clontech, Cat. 631106), 1% Penicillin-Streptomycin, 200 µg/ml Hygromycin B (Invitrogen, Cat. 10687-010), Blasticidin 5 µg/ml, (Invitrogen, Cat. R210-01), 4 µg/ml doxycycline (Clontech, Cat. 631311), 50 µl per well of 384 well PDL-coated plates and grown at 37°C with 5% CO₂. After overnight incubation, 10 µl/well of dilution series of the test compounds are added in quadruplicates and plates are further incubated overnight at 37°C with 5% CO₂. Cells are washed 3 times with HBSS leaving 25 µl per well. BLA activity is detected by adding 5 µl/well of the GeneBlazer reagent CCF4-AM (Invitrogen, Cat. K1085) to the cells. After incubation of the plates in the dark at 18°C for 60 min., blue and green fluorescence signals are measured in a Safire2 fluorescence plate reader (Tecan) with excitation at 410 nm and emissions at 458 nm (blue) and 522 nm (green). The ratio of blue/green fluorescence as a measure for BLA activity is calculated and EC₅₀ values are determined with the calculated blue/green fluorescence ratios as described for the hepcidin internalization assay. The EC₅₀ of hepcidin in this assay is about 0.096 ± 0.063 µM (n=37).

### 4. Ferroportin Internalization and Degradation Assay

HEK-293 cell line #354 (described in example 3) is used to measure the capacity of the compounds to induce internalization and degradation of ferroportin (Fpn) by fluorescence activated cell sorting (FACS). Growing HEK-293 #354 cells in doxycycline containing media induced expression of human Fpn-GFP fusion protein on the cell surface. Data from 10 independent experiments show that cultivation of HEK#354 cells for 48h in the presence of 4 µg/ml doxycycline induce in average 42.6% ± 6.4 % Fpn-GFP-positive cells. Small molecular weight Fpn inhibitor compounds are tested for dose-dependent effects on the Fpn-GFP mean fluorescence intensity (MFI) on HEK-293 cell line #354, as described below.

HEK#354 cells are harvested from ca. 80% confluent cultures, seeded at 0.6×10⁶ cells/ml in DMEM/F12 GlutaMAX^{™} medium (Invitrogen, Cat. 31331-028) containing 10% FBS (Clontech, Cat. 631106), 1% Penicillin-Streptomycin (Invitrogen, Cat. 15140-122), 200 µg/ml Hygromycin B (Invitrogen, Cat. 10687-010), Blasticidin 5 µg/ml, (Invitrogen, Cat. R210-01), 4 µg/ml doxycycline (Clontech, Cat. 631311), 50 µl per well of 384 well plates (Greiner; Cat. 781091) and grown at 37°C with 5% CO₂. After overnight incubation, 10 µl/well of dilution series of the test compounds are added in quadruplicates and plates are further incubated overnight at 37°C with 5% CO₂. Cells are washed once with FACS buffer (PBS containing 1% FBS, 2 mM EDTA and 0.05% NaN₃), harvested in FACS buffer with 0.5 µg/ml propidium iodide (Sigma, Cat. P4864) and analyzed in a flow cytometer (CANTO^{tm} II, BD Biosciences) equipped with high throughput sampler. Live HEK#354 cells are gated as propidium iodide negative population and analyzed for expression of Fpn-GFP. MFI of Fpn-GFP of > 2000 live cells for each compound dilution is calculated using FlowJo (Tree Star's, Oregon) and the potency of the Fpn-inhibitors to induce internalization and degradation of Fpn-GFP is calculated as described for the hepcidin internalization assay. The average EC₅₀ value of hepcidin in this assay is about 0.004 ± 0.002 µM.

### 5. Ferroportin ubiquitination and degradation

Exposure of cells expressing ferroportin (Fpn) to hepcidin is known to trigger ubiquitination and subsequent internalization and degradation of Fpn (Qiao, 2012). The potential of Fpn inhibitors to induce Fpn ubiquitination and degradation is investigated with an immunoprecipitation assay using the J774 mouse macrophage cell line which expresses Fpn upon treatment with iron.

J774 cells (DSMZ, Cat. ACC170) are seeded at 0.8x106 cells/ml in 15 ml of medium (DMEM Gibco Cat. 11971-025, 10% heat inactivated FBS Gibco Cat. 10500-064, 1% Penicillin-Streptomycin Gibco Cat. 15140-122) containing 200µM Fe(Ill)-NTA into 10 cm tissue culture dishes (Greiner Cat. 664160) and grown overnight at 37°C with 5% CO₂. Cells are incubated with synthetic human hepcidin (Bachem, Cat. H-5926) or Fpn inhibitor compounds for 10 min or 120 min. Cells are washed and lysed with ice-cold lysis buffer (Pierce, Life Technoligies, Cat. 87787) including 1X HALT protease inhibitor cocktail (Life technologies, Cat. 78429) and 10 mM iodoacetamide (Sigma, Cat. I6125) to stabilize ubiquitinated proteins. Immunoprecipitation is done using the Pierce Classic IP Kit (Life Technologies, Cat. 26146) following the manufacturer's protocol. Briefly, 2 mg protein in 1.25 ml IP lysis buffer is incubated by mixing for 1h at 4°C with control agarose beads to pre-clear the lysate and reduce nonspecific signal. Unbound lysate is then incubated overnight with 12 µg per reaction of the affinity purified anti-Fpn antibody F308 that is raised against a GST fusion protein of mouse Fpn amino acids 224-308. Immune complexes are captured by pipetting 14µl settled Pierce Protein A/G Plus Agarose beads (Life Technologies, Cat. 20423) per reaction and the slurry is incubated for 1.5 h at 4°C with gentle end-over-end mixing. The beads are washed and immune complexes are eluted directly with 75 µl SDS NuPAGE LDS sample buffer (Life Technologies, Cat. NP0007) containing DTT (Life Technologies, Cat. NP0009).

After immunoprecipitation samples are analyzed by Western blotting using a rabbit anti-mouse MTP1 antiserum (Alpha Diagnostic International, Cat. MTP11-A) and a mouse anti-mono- and polyubiquitinylated conjugates monoclonal antibody (Enzo Lifesciences, Cat. BML-PW8810) for detection of ferroportin and ubiquitin, respectively. Mouse monoclonal anti-rabbit IgG light chain (Abcam, Cat. ab99697) and anti-mouse IgG H&L (Abcam, Cat. ab6789) HRP conjugates are used as secondary antibodies.

### 6. Inhibition of Iron Efflux by Ferroportin Inhibitors

The activity of hepcidin and ferroportin inhibitor compounds regarding their ability to block iron export via ferroportin is tested on T47D cells (ECACC, Cat. 85102201) as described below.

Cells are plated in 24-well plates (Greiner, Cat. 662160) containing 350'000 cells/well and incubated overnight with 100 µM ⁵⁸Fe (⁵⁸Fe(II)-Sulfate, Vifor Pharma Batch No. ROR 3085) in 500 µM L-Ascorbic Acid (Sigma Aldrich, Cat. 795437) containing growth medium. Cells were washed once with 500 µl iron uptake buffer (IUB; PIPES 40mM, Cat. P1851, Glucose Monohydrate 10 mM, Cat. 49158, Sodium Chloride 260 mM, Cat. 71379, Potassium Chloride 20 mM, Cat. P9541, Magnesium Sulfate 2 mM, Cat. 63138, Sigma Aldrich), then once with removal buffer (2 min incubation, BPDS 100 µM, Cat. 11890 and Na₂S₂O₄ 500 µM, Cat. 157953, Sigma Aldrich, in IUB) and again twice with IUB. A serial dilution of hepdicin (Bachem) or ferroportin inhibitors (4 µM-0.0064 µM, 5 fold dilution) is added in a total volume of 0.6 ml per well. Cells are incubated at 37°C with 5% CO2 for 20 h. Supernatants are collected and ⁵⁸Fe is measured using inductively coupled plasma mass spectrometry (ICP-MS, Thermo Scientific, Element 2). Pellets are harvested for protein concentration measurements. Results are plotted as ng ⁵⁸Fe in supernatant per mg protein in cell lysates.

### Preparation of Example Compounds

### General Experimental Details

Commercially available reagents and solvents (HPLC grade) were used without further purification. ¹H NMR spectra were recorded on a Bruker DRX 500 MHz spectrometer, a Bruker DPX 250 MHz spectrometer or a Bruker Avance spectrometer 400 MHz in deuterated solvents. Chemical shifts (δ) are in parts per million.

Compounds were purified by flash column chromatography on normal phase silica on Biotage Isolera systems using the appropriate SNAP cartridge and gradient. Alternatively, compounds were purified on reverse phase using Biotage Isolera systems with the appropriate C18 SNAP cartridge and reverse-phase eluent or by preparative HPLC (if stated otherwise).

### Abbrevations

- EtOAc: Ethylacetate
- CH₂Cl₂: Dichloromethane
- Et₂O: Diethylether
- MeOH: Methanol
- EtOH: Ethanol
- brine: Aqueous saturated sodium chloride solution
- Chloroform-*d*: Deuterated chloroform
- DMSO-*d*₆: Deuterated dimethylsulfoxid
- s: Singlet
- br s: Bright Singlet
- d: Doublet
- dd: Double Doublets
- dt: Doublet of Triplets
- td: Triplet of Doublets
- hept.: Heptett
- m: Multiplet
- q: Quartet
- δ: Chemical shift
- ppm: Parts per million
- M: Molarity
- mm: Millimolar
- umol: Mikromolar
- g: Gram
- mg: Milligram
- l: Liter
- mL: Milliliter
- h: Hours
- min: Minute
- %-w/w: Percentage by mass
- TLC: Thin layer chromatography
- UHPLC: Ultra high pressure liquid chromatography
- MS: Mass spectroscopy
- ESI: Electronic spray ionization
- m/z: mass to charge ratio
- H⁺: Proton
- MHz: Mega Hertz
- s.m.: starting material
- Jones Reagent: CrO₃ in H₂SO₄
- CrO₃: Chromium trioxide
- HCl: Hydrochloric acid
- H₂SO₄: Sulfuric acid
- NH₄Cl: Ammonium chloride
- Na₂SO₄: Sodium sulfate
- NaOH: Sodium hydroxide
- Bn: Benzyl
- MS: Mass spectra
- ESI: Electrospray ionisation
- SNAP: Biotage-column-brandname for flash column chromatography
- R_{f}: Retention Factor
- TLC: Thin Layer Chromatography

### Chemical nomenclature

The chemical names of the intermediates and the final Example Compounds were generated by using Chem Draw Professional 17.0.
All R_{f} values were determined using the following TLC plates: Merck, TLC Silcagel 60 F₂₅₄.

### Intermediates

### A. N-(benzo[f]quinolin-3-ylmethyl)-2-vinyloxazole-4-carboxamide

### (Example Compound No. 6 - Step 1)

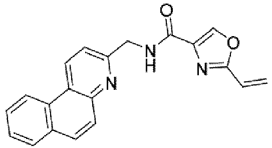

4-Methylmorpholin (0.3 ml, 2.5 mmol) and ethyl chloroformate (0.1 g, 0.89 mmol) were added to a 0°C cold solution of 2-vinyloxazole-4-carboxylic acid (0.1 g, 0.7 mmol) in 10 ml dichloromethane. After stirring for 1 h at 0°C, benzo[f]quinolin-3-ylmethanamine dihydrochloride (0.2 g, 0.7 mmol) (purchased from Chemspace) was added and the resulting mixture was stirred at room temperature for 2 h. The reaction mixture was washed with saturated aqueous NH₄Cl solution (1x 10 ml), brine (1x 10 ml), dried over Na₂SO₄, filtered and concentrated under reduced pressure. The crude material was purified by flash column chromatography (CH₂Cl₂/MeOH) to obtain the titled compound A (0.13 g, 0.4 mmol, 55%) as an off-white solid. UHPLC/MS (ESI): *[m*/*z]:* 330 [M+H]⁺.

### B. N-(phenanthridin-6-ylmethyl)-2-vinyloxazole-4-carboxamide

### (Example Compound No. 7 - Step 1)

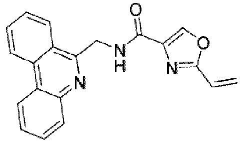

4-Methylmorpholin (0.3 ml, 2.5 mmol) and ethyl chloroformate (0.1 g, 0.89 mmol) were added to a 0°C cold solution of 2-vinyloxazole-4-carboxylic acid (0.1 g, 0.7 mmol) in 10 ml dichloromethane. After stirring for 1 h at 0°C, phenanthridin-6-ylmethanamine dihydrochloride (purchased from Chemspace) (0.2 g, 0.7 mmol) was added and the resulting mixture was stirred at room temperature for 2 h. The reaction mixture was washed with saturated aqueous NH₄Cl solution (1x 10 ml), brine (1x 10 ml), dried over Na₂SO₄, filtered and concentrated under reduced pressure. The crude material was purified by flash column chromatography (CH₂Cl₂/MeOH) to obtain the titled compound B (0.164 g, 0.5 mmol, 71%) as an off-white solid. UHPLC/MS (ESI): *[m*/*z]:* 330 [M+H]⁺.

### C. N-((3-methylpyridin-2-yl)methyl)-2-vinyloxazole-4-carboxamide

### (Example Compound No. 13 - Step 1)

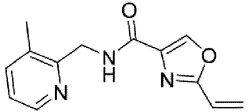

4-Methylmorpholin (0.5 ml, 4.4 mmol) and ethyl chloroformate (0.5 ml, 4.5 mmol) were added to a 0°C cold solution of 2-vinyloxazole-4-carboxylic acid (0.5 g, 3.6 mmol) in 10 ml dichloromethane. After stirring for 1 h at 0°C, (3-methylpyridin-2-yl)methanamine (purchased from Chemspace) (0.5 g, 3.5 mmol) was added and the resulting mixture was stirred at room temperature for 2 h. The reaction mixture was washed with saturated aqueous NH₄Cl solution (1x 10 ml), brine (1x 10 ml), dried over Na₂SO₄, filtered and concentrated under reduced pressure. The crude material was purified by flash column chromatography (CH₂Cl₂/MeOH) to obtain the titled compound C (0.432 g, 1.8 mmol, 49%) as an off-white solid. UHPLC/MS (ESI): [*m*/*z*]: 244 [M+H]⁺.

### D. N-((3-chloropyridin-2-yl)methyl)-2-vinyloxazole-4-carboxamide

### (Example Compound No. 14 - Step 1)

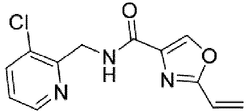

4-Methylmorpholin (0.4 ml, 3.6 mmol) and ethyl chloroformate (0.3 ml, 3.2 mmol) were added to a 0°C cold solution of 2-vinyloxazole-4-carboxylic acid (0.4 g, 2.9 mmol) in 10 ml dichloromethane. After stirring for 1 h at 0°C, (3-chloropyridin-2-yl)methanamine (purchased from Chemspace) (0.4 g, 2.9 mmol) was added and the resulting mixture was stirred at room temperature for 2 h. The reaction mixture was washed with saturated aqueous NH₄Cl solution (1x 10 ml), brine (1x 10 ml), dried over Na₂SO₄, filtered and concentrated under reduced pressure. The crude material was purified by flash column chromatography (CH₂Cl₂/MeOH) to obtain the titled compound D (0.3 g, 1.14 mmol, 39%) as an off-white solid. UHPLC/MS (ESI): *[m*/*z]:* 264 [M+H]⁺.

### E. N-((3-methoxypyridin-2-yl)methyl)-2-vinyloxazole-4-carboxamide

### (Example Compound No. 15 - Step 1)

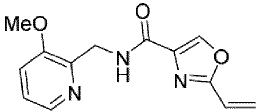

4-Methylmorpholin (0.5 ml, 4.5 mmol) and ethyl chloroformate (0.5 ml, 4.5 mmol) were added to a 0°C cold solution of 2-vinyloxazole-4-carboxylic acid (0.5 g, 3.6 mmol) in 10 ml dichloromethane. After stirring for 1 h at 0°C, (3-methoxypyridin-2-yl)methanamine (purchased from Chemspace) (0.5 g, 3.6 mmol) was added and the resulting mixture was stirred at room temperature for 2 h. The reaction mixture was washed with saturated aqueous NH₄Cl solution (1x 10 ml), brine (1x 10 ml), dried over Na₂SO₄, filtered and concentrated under reduced pressure. The crude material was purified by flash column chromatography (CH₂Cl₂/MeOH) to obtain the titled compound E(0.75 g, 2.9 mmol, 80%) as an off-white solid. UHPLC/MS (ESI): [*m*/*z*]: 260 [M+H]⁺.

### F. N-((3-(trifluoromethyl)pyridin-2-yl)methyl)-2-vinyloxazole-4-carboxamide

### (Example Compound No. 16 - Step 1)

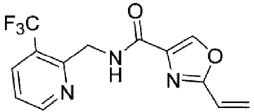

4-Methylmorpholin (0.4 ml, 3.6 mmol) and ethyl chloroformate (0.3 ml, 3.2 mmol) were added to a 0°C cold solution of 2-vinyloxazole-4-carboxylic acid (0.4 g, 2.8 mmol) in 10 ml dichloromethane. After stirring for 1 h at 0°C, (3-(trifluoromethyl)pyridin-2-yl)methanamine (purchased from Chemspace) (0.5 g, 2.8 mmol) was added and the resulting mixture was stirred at room temperature for 2 h. The reaction mixture was washed with saturated aqueous NH₄Cl solution (1x 10 ml), brine (1x 10 ml), dried over Na₂SO₄, filtered and concentrated under reduced pressure. The crude material was purified by flash column chromatography (CH₂Cl₂/MeOH) to obtain the titled compound F (0.51 g, 1.7 mmol, 61%) as an off-white solid. UHPLC/MS (ESI): *[m*/*z]:* 298 [M+H]⁺.

### G. N-(pyridin-2-ylmethyl)-2-vinyloxazole-4-carboxamide

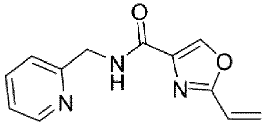

4-Methylmorpholin (0.5 ml, 4.4 mmol) and ethyl chloroformate (0.35 g, 3.3 mmol) were added to a 0°C cold solution of 2-vinyloxazole-4-carboxylic acid (0.5 g, 3.6 mmol) in 10 ml dichloromethane. After stirring for 1 h at 0°C, pyridin-2-ylmethanamine (Chemspace) was added (0.39 g, 3.6 mmol) and the resulting mixture was stirred at room temperature for 2 h. The reaction mixture was washed with saturated aqueous NH4Cl solution (1x 10 ml), brine (1x 10 ml), dried over Na2S04, filtered and concentrated under reduced pressure. The crude material was purified by flash column chromatography (CH₂Cl₂/MeOH) to obtain the titled compound (0.44 g, 1.9 mmol, 53%) as an off-white solid. UHPLC/MS (ESI): *[m*/*z]:* 230 [M+H]⁺.

### Example Compounds

### 1. Example Compound No. 1:

### 2-(2-(((1H-naphtho[2,3-d]imidazol-2-yl)methyl)amino)ethyl)-N-((3-fluoropyridin-2-yl)methyl)oxazole-4-carboxamide

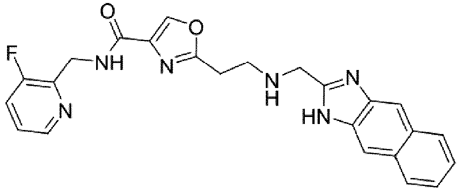

(1H-naphtho[2,3-d]imidazol-2-yl)methanamine dihydrochloride (purchased from Chemspace) (100 mg, 0.37 mmol) and N-((3-fluoropyridin-2-yl)methyl)-2-vinyloxazole-4-carboxamide (92 mg, 0.37 mmol) were added to a solution of NaOH (31 mg, 0.78 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 1 (6.9 mg, 0.016 mmol, 4%) as an off-white solid. ¹H NMR (400 MHz, DMSO-*d*₆) δ 8.50 (m, 2H), 8.35 (m, 1H), 7.95 (m, 4H), 7.65 (m, 1H), 7.35 (m, 3H), 4.60 (m, 2H), 4.00 (m, 2H), 3.00 (m, 2H) ppm. UHPLC/MS (ESI): [*m*/*z*]: 445 [M+H]⁺.

### 2. Example Compound No. 2:

### 2-(2-(((3H-imidazo[4,5-c]quinolin-2-yl)methyl)amino)ethyl)-N-((3-fluoropyridin-2-yl)methyl)oxazole-4-carboxamide

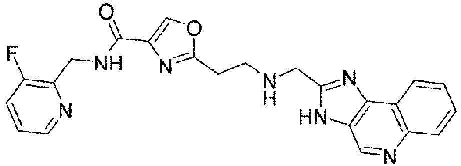

(3H-imidazo[4,5-c]quinolin-2-yl)methanamine dihydrochloride (purchased from Chemspace) (100 mg, 0.37 mmol) and N-((3-fluoropyridin-2-yl)methyl)-2-vinyloxazole-4-carboxamide (92 mg, 0.37 mmol) were added to a solution of NaOH (31 mg, 0.78 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 2(8 mg, 0.018 mmol, 5%) as an off-white solid. ¹H NMR (400 MHz, DMSO-*d*₆) δ 9.10 (m, 1H), 8.50 (m, 1H), 8.40 (m, 2H), 8.05 (m, 1H), 7.65 (m, 3H), 7.4 (m, 1H), 4.60 (m, 2H), 4.10 (m, 2H), 3.00 (m, 4H) ppm. UHPLC/MS (ESI): *[m*/*z]:* 446 [M+H]⁺.

### 3. Example Compound No. 3:

### 2-(2-((2-(3H-imidazo[4,5-c]quinolin-2-yl)ethyl)amino)ethyl)-N-((3-fluoropyridin-2-yl)methyl)oxazole-4-carboxamide

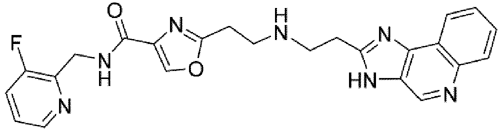

2-(3H-imidazo[4,5-c]quinolin-2-yl)ethan-1-amine dihydrochloride (purchased from Chemspace) (100 mg, 0.35 mmol) and N-((3-fluoropyridin-2-yl)methyl)-2-vinyloxazole-4-carboxamide (87 mg, 0.35 mmol) were added to a solution of NaOH (30 mg, 0.74 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 3 (45.8 mg, 0.1 mmol, 28%) as an off-white solid. ¹H NMR (400 MHz, DMSO-*d*₆) δ 9.1 (s, 1H), 8.5 (m, 1H), 8.3 (m, 2H), 8.2 (s, 1H), 8.1 (m, 1H), 7.6 (m, 3H), 7.4 (m, 1H), 4.6 (d, 2H), 3.2 (m, 4H), 3.0 (m, 4H) ppm. UHPLC/MS (ESI): *[m*/*z]:* 460 [M+H]⁺.

### 4. Example Compound No. 4:

### 2-(2-(((3H-naphtho[1,2-d]imidazol-2-yl)methyl)amino)ethyl)-N-((3-fluoropyridin-2-yl)methyl)oxazole-4-carboxamide

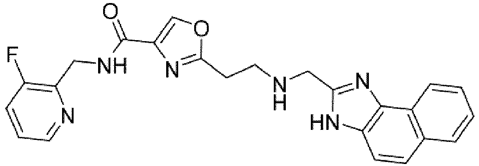

(3H-naphtho[1,2-d]imidazol-2-yl)methanamine dihydrochloride (purchased from Chemspace) (100 mg, 0.37 mmol) and N-((3-fluoropyridin-2-yl)methyl)-2-vinyloxazole-4-carboxamide (92 mg, 0.37 mmol) were added to a solution of NaOH (31 mg, 0.78 mmol) in 10 ml water. The resulting mixture was heated at 80 °C under stirring for 72 hours. After cooling at room temperature the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 4 (5 mg, 0.011 mmol, 3%) as an off-white solid. UHPLC/MS (ESI): *[m*/*z]:* 445 [M+H]⁺. UHPLC/MS (ESI): *[m*/*z]:* 445 [M+H]⁺, 265 [C₁₂H₁₄FN₄O₂]⁺, 181 [C₁₂H₉N₂]⁺, 110 [C₆H₅FN]⁺.

### 5. Example Compound No. 5:

### 2-(2-((2-(3H-naphtho[1,2-d]imidazol-2-yl)ethyl)amino)ethyl)-N-((3-fluoropyridin-2-yl)methyl)oxazole-4-carboxamide

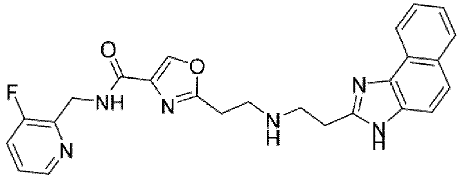

2-(3H-naphtho[1,2-d]imidazol-2-yl)ethan-1-amine dihydrochloride (purchased from Chemspace) (100 mg, 0.35 mmol) and N-((3-fluoropyridin-2-yl)methyl)-2-vinyloxazole-4-carboxamide (87 mg, 0.35 mmol) were added to a solution of NaOH (30 mg, 0.74 mmol) in 10 ml water. The resulting mixture was heated at 80 °C under stirring for 72 hours. After cooling at room temperature the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 5 (40.6 mg, 0.09 mmol, 25%) as an off-white solid. ¹H NMR (400 MHz, DMSO-*d*₆) δ 8.50 (m, 2H), 8.30 (m, 2H), 7.95 (d, 1H), 7.60 (m, 4H), 7.40 (m, 2H), 4.60 (d, 2H), 3.00 (m, 8H) ppm. UHPLC/MS (ESI): *[m*/*z]:* 459 [M+H]⁺.

### 6. Example Compound No. 6:

### 2-(2-((2-(1H-benzo[d]imidazol-2-yl)ethyl)amino)ethyl)-N-(benzo[f]quinolin-3-ylmethyl)oxazole-4-carboxamide

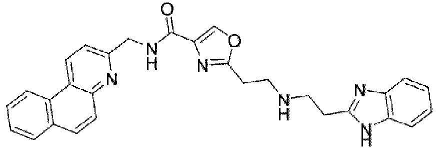

2-(1H-benzo[d]imidazol-2-yl)ethan-1-amine (49 mg, 0.3 mmol) and *N*-(benzo[f]quinolin-3-ylmethyl)-2-vinyloxazole-4-carboxamide (100 mg, 0.3 mmol) were added to a solution of NaOH (1.2 mg, 0.03 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 6 (56 mg, 0.11 mmol, 38%) as an off-white solid. ¹H NMR (400 MHz, DMSO-*d*₆) δ 9.2 (m, 1H), 9.05 (m, 1H), 8.8 (m, 1H), 8.65 (s, 1H), 8.1 (m, 2H), 7.9 (m, 1H), 7.7 (m, 2H), 7.6 (m, 1H), 7.45 (m, 2H), 7.15 (m, 2H), 4.8 (m, 2H), 3.6 (m, 2H), 3.45 (m, 2H), 3.2 (m, 2H), 3.15 (m, 2H) ppm. UHPLC/MS (ESI): *[m*/*z]:* 491 [M+H]⁺.

### 7. Example Compound No. 11:

### 2-(2-((2-(3H-naphtho[1,2-d]imidazol-2-yl)ethyl)amino)ethyl)-N-(benzo[f]isoquinolin-2-ylmethyl)oxazole-4-carboxamide

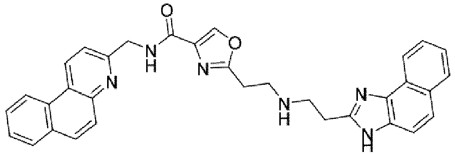

2-(3H-naphtho[1,2-d]imidazol-2-yl)ethan-1-amine dihydrochloride (purchased from Chemspace) (130 mg, 0.46 mmol) and N-(benzo[f]quinolin-3-ylmethyl)-2-vinyloxazole-4-carboxamide (150 mg, 0.46 mmol) were added to a solution of NaOH (55 mg, 1.37 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 11 (7 mg, 0.013 mmol, 3%) as an off-white solid. ¹H NMR (400 MHz, DMSO-*d*₆) δ 9.1 (m, 1H), 8.9 (m, 1H), 8.8 (m, 1H), 8.6 (s, 1H), 8.3 (m, 1H), 8.1 (m, 2H), 8.0 (m, 1H), 7.9 (m, 1H), 7.8 - 7-5 (m, 7H), 7.4 (m, 1H), 4.7 (m, 2H), 3.2 - 3.1 (m, 8H) ppm. UHPLC/MS (ESI): *[m*/*z]:* 541 [M+H]⁺.

### 8. Example Compound No. 12:

### 2-(2-((2-(3H-imidazo[4,5-c]quinolin-2-yl)ethyl)amino)ethyl)-N-(benzo[f]isoquinolin-2-ylmethyl)oxazole-4-carboxamide

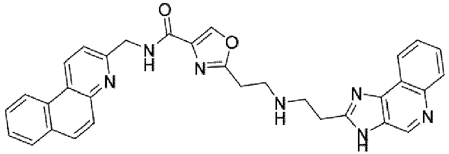

2-(3H-imidazo[4,5-c]quinolin-2-yl)ethan-1-amine dihydrochloride (purchased from Chemspace) (130 mg, 0.46 mmol) and *N*-(benzo[f]quinolin-3-ylmethyl)-2-vinyloxazole-4-carboxamide (150 mg, 0.46 mmol) were added to a solution of NaOH (55 mg, 1.37 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 12 (30.1 mg, 0.056 mmol, 12%) as an off-white solid. ¹H NMR (400 MHz, DMSO-*d*₆) δ 9.2 (m, 1H), 9.1 (s, 1H), 9.0 (m, 1H), 8.8 (m, 1H), 8.6 (s, 1H), 8.3 (m, 1H), 8.1 (m, 3H), 7.9 (m, 1H), 7.8 - 7-6 (m, 5H), 4.7 (m, 2H), 3.2 - 3.1 (m, 8H) ppm. UHPLC/MS (ESI): *[m*/*z]:* 542 [M+H]⁺.

### 9. Example Compound No. 7:

### 2-(2-((2-(1H-benzo[d]imidazol-2-yl)ethyl)amino)ethyl)-N-(phenanthridin-6-ylmethyl)oxazole-4-carboxamide

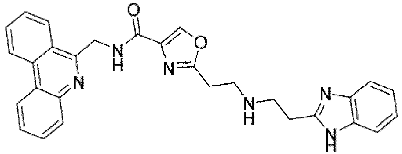

2-(1H-benzo[d]imidazol-2-yl)ethan-1-amine (49 mg, 0.3 mmol) and N-(phenanthridin-6-ylmethyl)-2-vinyloxazole-4-carboxamide (100 mg, 0.3 mmol) were added to a solution of NaOH (1.2 mg, 0.03 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 7 (6 mg, 0.012 mmol, 4%) as an off-white solid. ¹H NMR (400 MHz, DMSO-*d*₆) δ 8.9 - 8.8 (m, 3H), 8.6 (s, 1H), 8.4 (m, 1H), 8.1 - 7.9 (m, 2H), 7.8 - 7.7 (m, 3H), 7.4 (m, 2H), 7.1 (m, 2H), 5.2 (d, 2H), 3.0 (m, 8H) ppm. UHPLC/MS (ESI): [*m*/*z*]: 491 [M+H]⁺.

### 10. Example Compound No. 8:

### 2-(2-((2-(3H-naphtho[1,2-d]imidazol-2-yl)ethyl)amino)ethyl)-N-(phenanthridin-6-ylmethyl)oxazole-4-carboxamide

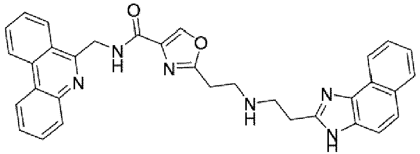

2-(3H-naphtho[1,2-d]imidazol-2-yl)ethan-1-amine dihydrochloride (purchased from Chemspace) (130 mg, 0.46 mmol) and N-(phenanthridin-6-ylmethyl)-2-vinyloxazole-4-carboxamide (150 mg, 0.46 mmol) were added to a solution of NaOH (55 mg, 1.37 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 8 (31 mg, 0.057 mmol, 13%) as an off-white solid. ¹H NMR (400 MHz, DMSO-*d*₆) δ 8.9 (m, 2H), 8.8 (m, 1H), 8.6 (s, 1H), 8.4 (d, 1H), 8.3 (d, 1H), 8.0 - 7.9 (m, 3H), 7.8 - 7.7 (m, 3H), 7.6 - 7.5 (m, 3H), 7.4 (m, 1H), 5.2 (d, 2H), 3.2 (m, 8H) ppm. UHPLC/MS (ESI): *[m*/*z]:* 541 [M+H]⁺.

### 11. Example Compound No. 13:

### 2-(2-((2-(3H-naphtho[1,2-d]imidazol-2-yl)ethyl)amino)ethyl)-N-((3-methylpyridin-2-yl)methyl)oxazole-4-carboxamide

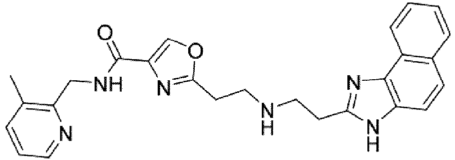

2-(3H-naphtho[1,2-d]imidazol-2-yl)ethan-1-amine dihydrochloride (purchased from Chemspace) (100 mg, 0.35 mmol) and N-((3-methylpyridin-2-yl)methyl)-2-vinyloxazole-4-carboxamide (87 mg, 0.35 mmol) were added to a solution of NaOH (30 mg, 0.74 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 13 (21 mg, 0.046 mmol, 13%) as an off-white solid. ¹H NMR (400 MHz, DMSO-*d*₆) δ 8.50 (m, 2H), 8.30 (m, 2H), 7.95 (d, 1H), 7.60 (m, 4H), 7.40 (m, 2H), 4.60 (d, 2H), 3.00 (m, 8H) ppm. UHPLC/MS (ESI): *[m*/*z]:* 455 [M+H]⁺.

### 12. Example Compound No. 14:

### 2-(2-((2-(3H-naphtho[1,2-d]imidazol-2-yl)ethyl)amino)ethyl)-N-((3-chloropyridin-2-yl)methyl)oxazole-4-carboxamide

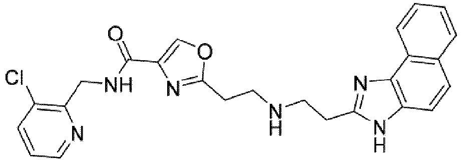

2-(3H-naphtho[1,2-d]imidazol-2-yl)ethan-1-amine dihydrochloride (purchased from Chemspace) (100 mg, 0.35 mmol) and N-((3-chloropyridin-2-yl)methyl)-2-vinyloxazole-4-carboxamide (93 mg, 0.35 mmol) were added to a solution of NaOH (30 mg, 0.74 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 14 (33.7 mg, 0.071 mmol, 20%) as an off-white solid. ¹H NMR (400 MHz, DMSO-*d*₆) δ 8.50 (m, 3H), 8.30 (m, H), 7.95 (m, 2H), 7.60 - 7.40 (m, 5H), 4.60 (m, 2H), 3.00 (m, 8H) ppm. UHPLC/MS (ESI): *[m*/*z]:* 471 [M+H]⁺.

### 13. Example Compound No. 15:

### 2-(2-((2-(3H-naphtho[1,2-d]imidazol-2-yl)ethyl)amino)ethyl)-N-((3-methoxypyridin-2-yl)methyl)oxazole-4-carboxamide

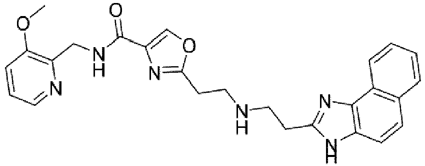

2-(3H-naphtho[1,2-d]imidazol-2-yl)ethan-1-amine dihydrochloride (purchased from Chemspace) (100 mg, 0.35 mmol) and N-((3-methoxypyridin-2-yl)methyl)-2-vinyloxazole-4-carboxamide (91 mg, 0.35 mmol) were added to a solution of NaOH (30 mg, 0.74 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 15 (70 mg,0.15 mmol, 42%) as an off-white solid. ¹H NMR (400 MHz, DMSO-*d*₆) δ 8.50 (s, 1H), 8.3 (m, 2H), 8.1 (m, 1H), 7.95 (m, 1H), 7.6 (m, 3H), 7.4 (m, 2H), 7.3 (m, 1H), 4.5 (d, 2H), 3.95 (s, 3H), 3.1 (m, 8H) ppm. UHPLC/MS (ESI): *[m*/*z]:* 471 [M+H]⁺.

### 14. Example Compound No. 16:

### 2-(2-((2-(3H-naphtho[1,2-d]imidazol-2-yl)ethyl)amino)ethyl)-N-((3-(trifluoromethyl)pyridin-2-yl)methyl)oxazole-4-carboxamide

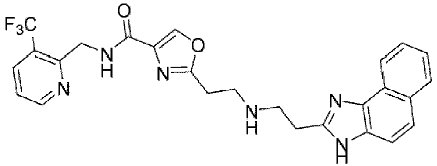

2-(3H-naphtho[1,2-d]imidazol-2-yl)ethan-1-amine dihydrochloride (purchased from Chemspace) (100 mg, 0.35 mmol) and *N*-((3-(trifluoromethyl)pyridin-2-yl)methyl)-2-vinyloxazole-4-carboxamide (105 mg, 0.35 mmol) were added to a solution of NaOH (30 mg, 0.74 mmol) in 10 ml water. The resulting mixture was heated at 80 °C under stirring for 72 hours. After cooling at room temperature, the reaction mixture was adjusted to pH 6 by using 2N HCl and extracted with dichloromethane (3x 10 ml). The combined organic phases were washed with brine, dried over sodium sulfate, filtered and concentrated under reduced pressure. The crude material was purified by flash column chromatography (CH₂Cl₂/MeOH) to obtain the titled compound 16 (43.6 mg, 0.086 mmol, 24 %) as an off-white solid. ¹H NMR (400 MHz, DMSO-*d*₆) δ 8.8 (d, 1H), 8.5 (m, 2H), 8.3 (d, 1H), 8.2 (d, 1H), 7.9 (d, 1H), 7.6 (m, 3H), 7.4 (m, 1H), 4.7 (d, 2H), 3.0 (m, 8H) ppm. UHPLC/MS (ESI): *[m*/*z]:* 509 [M+H]⁺.

### 15. Example Compound No. 19:

### N-((3-fluoropyridin-2-yl)methyl)-2-(2-((2-(5-phenyl-1H-benzo[d]imidazol-2-yl)ethyl)amino)ethyl)oxazole-4-carboxamide

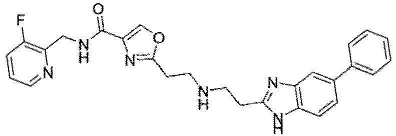

2-(5-phenyl-1H-benzo[d]imidazol-2-yl)ethan-1-amine dihydrochloride (Chemspace) (100 mg, 0.32 mmol) and *N*-((3-fluoropyridin-2-yl)methyl)-2-vinyloxazole-4-carboxamide (80 mg, 0.32 mmol) were added to a solution of NaOH (32 mg, 0.81 mmol) in 10 ml water. The resulting mixture were heated at 80 °C under stirring for 72 hours. After cooling at room temperature the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 19 (22 mg, 0.045 mmol, 14%) as an off-white solid. ¹H NMR (400 MHz, DMSO-*d*₆) δ 8.5 (m, 2H), 8.3 (m, 1H), 7.7 (m, 4H), 7.5 (d, 1H), 7.4 (m, 4H), 7.3 (m, 1H), 4.6 (d, 2H), 3.0 (m, 8H) ppm. UHPLC/MS (ESI): *[m*/*z]:* 485 [M+H]⁺.

### 16. Example Compound No.30:

### 2-(2-((2-(5-(4-fluorophenyl)-1H-benzo[d]imidazol-2-yl)ethyl)amino)ethyl)-N-((3-fluoropyridin-2-yl)methyl)oxazole-4-carboxamide

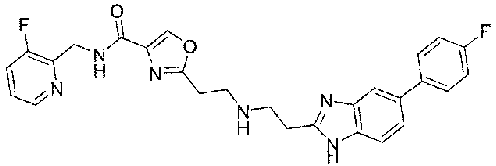

2-(5-(4-fluorophenyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (70 mg, 0.31 mmol) were added to a solution of NaOH (31 mg, 0.77 mmol) in 10 ml water. The resulting mixture were heated at 80 °C under stirring for 72 hours. After cooling at room temperature the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 30 (26 mg, 0.052 mmol, 17%) as an off-white solid. ¹H NMR (400 MHz, DMSO-*d*₆) δ 8.5 (m, 2H), 8.3 (m, 1H), 7.7 (m, 4H), 7.5 (m, 1H), 7.4 (m, 2H), 7.2 (m, 2H), 4.6 (d, 2H), 3.0 (m, 8H) ppm. UHPLC/MS (ESI): *[m*/*z]:* 503 [M+H]⁺.

### 17. Example Compound No. 32:

### 2-(2-((2-(5-(1H-pyrrol-1-yl)-1H-benzo[d]imidazol-2-yl)ethyl)amino)ethyl)-N-((3-fluoropyridin-2-yl)methyl)oxazole-4-carboxamide

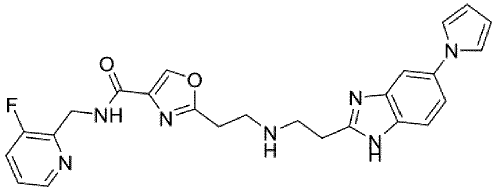

2-(5-(1H-pyrrol-1-yl)-1H-benzo[d]imidazol-2-yl)ethan-1-amine dihydrochloride (Chemspace) (100 mg, 0.33 mmol) and N-((3-fluoropyridin-2-yl)methyl)-2-vinyloxazole-4-carboxamide (83 mg, 0.33 mmol) were added to a solution of NaOH (34 mg, 0.84 mmol) in 10 ml water. The resulting mixture were heated at 80 °C under stirring for 72 hours. After cooling at room temperature the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 30 (54 mg,0.11 mmol, 35%) as an off-white solid. ¹H NMR (400 MHz, DMSO-*d*₆) δ 8.5 (m, 2H), 8.35 (m, 1H), 7.7 (m, 1H), 7.55 (m, 1H), 7.5 (m, 1H), 7.4 (m, 1H), 7.3 (m, 3H), 6.2 (m, 2H), 4.6 (d, 2H), 3.0 (m, 8H) ppm. UHPLC/MS (ESI): [*m*/*z*]: 474 [M+H]⁺.

### 18. Example Compound No. 33:

### N-((3-fluoropyridin-2-yl)methyl)-2-(2-((2-(5,6,7,8-tetrahydro-1H-naphtho[2,3-d]imidazol-2-yl)ethyl)amino)ethyl)oxazole-4-carboxamide

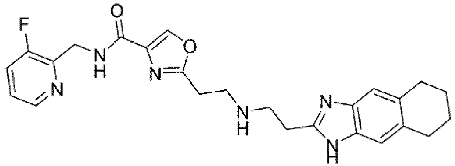

2-(5,6,7,8-tetrahydro-1H-naphtho[2,3-d]imidazol-2-yl)ethan-1-amine dihydrochloride (Chemspace) (100 mg, 0.35 mmol) and N-((3-methoxypyridin-2-yl)methyl)-2-vinyloxazole-4-carboxamide (86 mg, 0.35 mmol) were added to a solution of NaOH (35 mg, 0.87 mmol) in 10 ml water. The resulting mixture were heated at 80 °C under stirring for 72 hours. After cooling at room temperature the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 33 (57 mg, 0.12 mmol, 36%) as an off-white solid. ¹H NMR (400 MHz, DMSO-*d*₆) δ 8.5 (m, 2H), 8.35 (m, 1H), 7.7 (m, 1H), 7.4 (m, 1H), 7.1 (m, 2H), 4.6 (d, 2H), 3.0 (m, 12H), 1.8 (m, 4H) ppm. UHPLC/MS (ESI): *[m*/*z]:* 463 [M+H]⁺.

### 19. Example Compound No. 34:

### 2-(2-((2-(6,7-dihydro-1H-[1,4]dioxino[2',3':4,5]benzo[1,2-d]imidazol-2-yl)ethyl)amino)ethyl)-N-((3-fluoropyridin-2-yl)methyl)oxazole-4-carboxamide

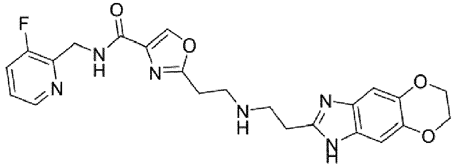

2-(6,7-dihydro-1H-[1,4]dioxino[2',3':4,5]benzo[1,2-d]imidazol-2-yl)ethan-1-amine dihydrochloride (Chemspace) (100 mg, 0.34 mmol) and *N*-((3-fluoropyridin-2-yl)methyl)-2-vinyloxazole-4-carboxamide (85 mg, 0.34 mmol) were added to a solution of NaOH (35 mg, 0.86 mmol) in 10 ml water. The resulting mixture were heated at 80 °C under stirring for 72 hours. After cooling at room temperature the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 34 (25.3 mg, 0.054 mmol, 16%) as an off-white solid. ¹H NMR (400 MHz, DMSO-*d*₆) δ 8.5 (m, 2H), 8.3 (m, 1H), 7.7 (m, 1H), 7.4 (m, 1H), 6.9 (m, 3H), 4.6 (d, 2H), 4.2 (m, 6H), 3.1 (m, 6H) ppm. UHPLC/MS (ESI): *[m*/*z]:* 467 [M+H]⁺.

### 20. Example Compound No. 36:

### 2-(2-((2-(5H-[1,3]dioxolo[4',5':4,5]benzo[1,2-d]imidazol-6-yl)ethyl)amino)ethyl)-N-((3-fluoropyridin-2-yl)methyl)oxazole-4-carboxamide

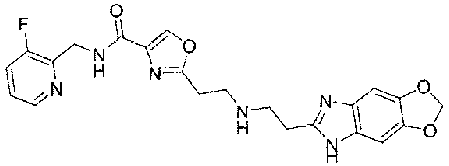

2-(5H-[1,3]dioxolo[4',5':4,5]benzo[1,2-d]imidazol-6-yl)ethan-1-amine hydrochloride (Chemspace) (100 mg, 0.41 mmol) and N-((3-fluoropyridin-2-yl)methyl)-2-vinyloxazole-4-carboxamide (102 mg, 0.41 mmol) were added to a solution of NaOH (25 mg, 0.62 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 67 (13 mg, 0.029 mmol, 7%) as an off-white solid. ¹H NMR (400 MHz, DMSO-*d*₆) δ 8.6 (m, 2H), 8.4 (m, 1H), 7.7 (m, 1H), 7.4 (m, 1H), 7.0 (m, 2H), 6.0 (s, 2H), 4.6 (d, 2H), 3.2 - 3.1 (m, 8H) ppm. UHPLC/MS (ESI): *[m*/*z]:* 453 [M+H]⁺.

### 21. Example Compound No. 37:

### N-((3-fluoropyridin-2-yl)methyl)-2-(2-((2-(7-(phenylethynyl)-1H-benzo[d]imidazol-2-yl)ethyl)amino)ethyl)oxazole-4-carboxamide

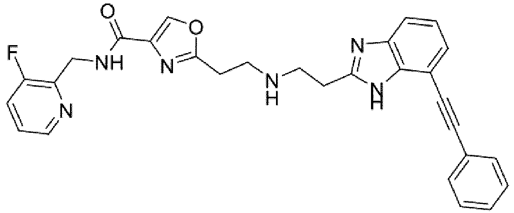

2-(2-(I2-azaneyl)ethyl)-7-(phenylethynyl)-1H-benzo[d]imidazole dihydrochloride (Chemspace) (105 mg, 0.31 mmol) and N-((3-fluoropyridin-2-yl)methyl)-2-vinyloxazole-4-carboxamide (77 mg, 0.31 mmol) were added to a solution of NaOH (25 mg, 0.63 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 77 (49 mg, 0.1 mmol, 31%) as an off-white solid. ¹H NMR (400 MHz, DMSO-*d*₆) δ 8.6 (m, 2H), 8.35 (m, 1H), 7.7 - 7.3 (m, 8H), 7.15 (m, 1H), 4.6 (d, 2H), 3.0 (m, 8H) ppm. UHPLC/MS (ESI): [*m*/*z*]: 509 [M+H]⁺.

### 22. Example Compound No. 39:

### N-((3-fluoropyridin-2-yl)methyl)-2-(2-((2-(6,7,8,9-tetrahydro-3H-naphtho[1,2-d]imidazol-2-yl)ethyl)amino)ethyl)oxazole-4-carboxamide

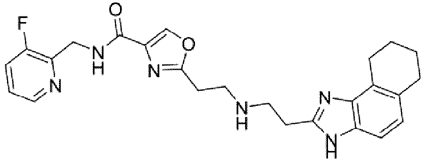

2-(6,7,8,9-tetrahydro-3H-naphtho[1,2-d]imidazol-2-yl)ethan-1-amine dihydrochloride (Chemspace) (100 mg, 0.35 mmol) and N-((3-fluoropyridin-2-yl)methyl)-2-vinyloxazole-4-carboxamide (86 mg, 0.35 mmol) were added to a solution of NaOH (35 mg, 0.87 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 39 (54 mg, 0.12 mmol, 33%) as an off-white solid. ¹H NMR (400 MHz, DMSO-*d*₆) δ 8.5 (m, 2H), 8.3 (m, 1H), 7.7 (m, 1H), 7.4 (m, 1H), 7.1 (d, 1H), 6.8 (d, 1H), 4.6 (d, 2H), 3.0 (m, 8H), 2.85 (m, 2H), 2,75 (m, 2H), 1.8 (m, 4H) ppm. UHPLC/MS (ESI): [*m*/*z*]: 463 [M+H]⁺.

### 23. Example Compound No. 43:

### 2-(2-((2-(1H-naphtho[2,3-d]imidazol-2-yl)ethyl)amino)ethyl)-N-((3-fluoropyridin-2-yl)methyl)oxazole-4-carboxamide

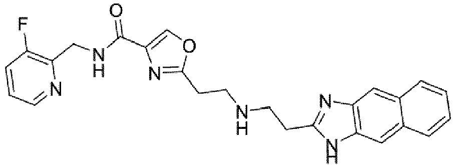

2-(1H-naphtho[2,3-d]imidazol-2-yl)ethan-1-amine dihydrochloride (Chemspace) (100 mg, 0.35 mmol) and N-((3-fluoropyridin-2-yl)methyl)-2-vinyloxazole-4-carboxamide (87 mg, 0.35 mmol) were added to a solution of NaOH (35 mg, 0.88 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 43 (37.6 mg, 0.082 mmol, 23 %) as an off-white solid. ¹H NMR (400 MHz, DMSO-*d*₆) δ 8.5 (m, 2H), 8.3 (m, 1H), 7.9 (m, 4H), 7.7 (m, 1H), 7.3 (m, 3H), 4.6 (d, 2H), 3.0 (m, 8H) ppm. UHPLC/MS (ESI): [*m*/z]: 459 [M+H]⁺.

### 24. Example Compound No. 44:

### 2-(2-((2-(5-(2-chlorophenyl)-1H-benzo[d]imidazol-2-yl)ethyl)amino)ethyl)-N-((3-fluoropyridin-2-yl)methyl)oxazole-4-carboxamide

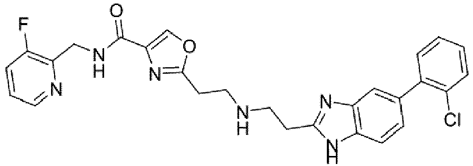

2-(5-(2-chlorophenyl)-1H-benzo[d]imidazol-2-yl)ethan-1-amine dihydrochloride (Chemspace) (100 mg, 0.29 mmol) and *N*-((3-fluoropyridin-2-yl)methyl)-2-vinyloxazole-4-carboxamide (72 mg, 0.29 mmol) were added to a solution of NaOH (29 mg, 0.73 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 44 (32 mg, 0.062 mmol, 21%) as an off-white solid. ¹H NMR (400 MHz, DMSO-*d*₆) δ 8.5 (m, 2H), 8.3 (m, 1H), 7.7 (m, 1H), 7.6 (s, 1H), 7.5 (m, 2H), 7.4 (m, 2H), 7.3 (m, 3H), 4.6 (d, 2H), 3.0 (m, 8H) ppm. UHPLC/MS (ESI): [*m*/*z*]: 520 [M+H]⁺.

### 25. Example Compound No. 45

### N-((3-fluoropyridin-2-yl)methyl)-2-(2-((2-(5-(2-methoxyphenyl)-1H-benzo[d]imidazol-2-yl)ethyl)amino)ethyl)oxazole-4-carboxamide

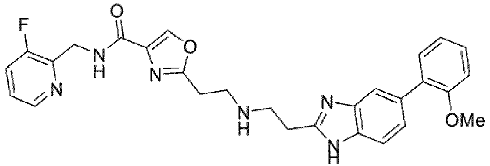

2-(5-(2-methoxyphenyl)-1H-benzo[d]imidazol-2-yl)ethan-1-amine dihydrochloride (Chemspace) (100 mg, 0.29 mmol) and *N*-((3-fluoropyridin-2-yl)methyl)-2-vinyloxazole-4-carboxamide (73 mg, 0.29 mmol) were added to a solution of NaOH (29 mg, 0.73 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 45 (64 mg, 0.12 mmol, 42%) as an off-white solid. ¹H NMR (400 MHz, DMSO-*d*₆) δ 8.5 (m, 2H), 8.3 (m, 1H), 7.7 (m, 1H), 7.5 (s, 1H), 7.45 (d, 1H), 7.35 (m, 1H), 7.3 (m, 2H), 7.2 (dd, 1H), 7.1 (d, 1H), 7.0 (t, 1H), 4.6 (d, 2H), 3.0 (m, 8H) ppm. UHPLC/MS (ESI): [*m*/*z*]: 515 [M+H]⁺.

### 26. Example Compound No. 46:

### 2-(2-((2-(5-(2-fluorophenyl)-1H-benzo[d]imidazol-2-yl)ethyl)amino)ethyl)-N-((3-fluoropyridin-2-yl)methyl)oxazole-4-carboxamide

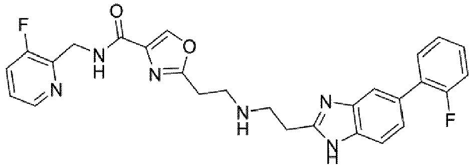

2-(5-(2-fluorophenyl)-1H-benzo[d]imidazol-2-yl)ethan-1-amine dihydrochloride (Chemspace) (100 mg, 0.3 mmol) and *N*-((3-fluoropyridin-2-yl)methyl)-2-vinyloxazole-4-carboxamide (75 mg, 0.3 mmol) were added to a solution of NaOH (31 mg, 0.76 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 46 (49.1 mg, 0.098 mmol, 32%) as an off-white solid. ¹H NMR (400 MHz, DMSO-*d*₆) δ 8.5 (m, 2H), 8.3 (m, 1H), 7.7 (m, 1H), 7.6 (s, 1H), 7.5 (m, 2H), 7.4 (m, 2H), 7.3 (m, 3H), 4.6 (d, 2H), 3.0 (m, 8H) ppm. UHPLC/MS (ESI): [*m*/*z*]: 503 [M+H]⁺.

### 27. Example Compound No. 47:

### 2-(2-((2-(6,7-dihydro-1H-[1,4]dioxino[2',3':4,5]benzo[1,2-d]imidazol-2-yl)ethyl)amino)ethyl)-N-((3-methylpyridin-2-yl)methyl)oxazole-4-carboxamide

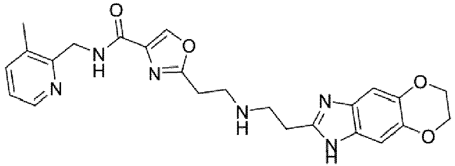

2-(6,7-dihydro-1H-[1,4]dioxino[2',3':4,5]benzo[1,2-d]imidazol-2-yl)ethan-1-amine dihydrochloride (Chemspace) (100 mg, 0.34 mmol) and N-((3-methylpyridin-2-yl)methyl)-2-vinyloxazole-4-carboxamide (84 mg, 0.34 mmol) were added to a solution of NaOH (35 mg, 0.86 mmol) in 10 ml water. The resulting mixture were heated at 80 °C under stirring for 72 hours. After cooling at room temperature the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 47 (18 mg, 0.039 mmol, 12%) as an off-white solid. ¹H NMR (400 MHz, DMSO-*d*₆) δ 8.5 (m, 2H), 8.3 (m, 1H), 7.7 (m, 1H), 7.4 (m, 1H), 6.9 (m, 3H), 4.6 (d, 2H), 4.2 (m, 6H), 3.1 (m, 6H) ppm. UHPLC/MS (ESI): [*m*/*z*]: 463 [M+H]⁺.

### 28. Example Compound No. 48:

### 2-(2-((2-(6,7-dihydro-1H-[1,4]dioxino[2',3':4,5]benzo[1,2-d]imidazol-2-yl)ethyl)amino)ethyl)-N-((3-methoxypyridin-2-yl)methyl)oxazole-4-carboxamide

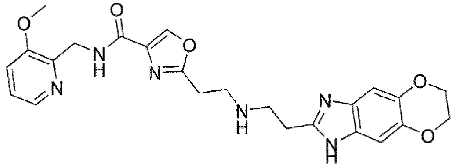

2-(6,7-dihydro-1H-[1,4]dioxino[2',3':4,5]benzo[1,2-d]imidazol-2-yl)ethan-1-amine dihydrochloride (Chemspace) (100 mg, 0.34 mmol) and N-((3-methoxypyridin-2-yl)methyl)-2-vinyloxazole-4-carboxamide (89 mg, 0.34 mmol) were added to a solution of NaOH (35 mg, 0.86 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 48 (33 mg, 0.069 mmol, 20%) as an off-white solid. ¹H NMR (400 MHz, DMSO-*d*₆) δ 8.5 (m, 1H), 8.3 (m, 1H), 8.1 (m, 1H), 7.4 (m, 1H), 7.3 (m, 1H), 6.85 (m, 2H), 4.5 (d, 2H), 4.2 (m, 4H), 3.9 (m, 3H), 3.0 (m, 8H) ppm. UHPLC/MS (ESI): *[m*/*z]:* 479 [M+H]⁺.

### 29. Example Compound No. 49:

### 2-(2-((2-(5-(2-methoxyphenyl)-1H-benzo[d]imidazol-2-yl)ethyl)amino)ethyl)-N-((3-methylpyridin-2-yl)methyl)oxazole-4-carboxamide

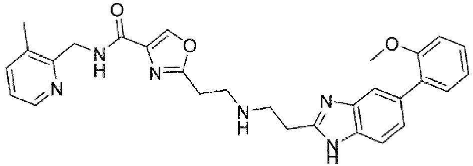

2-(5-(2-methoxyphenyl)-1H-benzo[d]imidazol-2-yl)ethan-1-amine dihydrochloride (Chemspace) (100 mg, 0.29 mmol) and N-((3-methylpyridin-2-yl)methyl)-2-vinyloxazole-4-carboxamide (72 mg, 0.29 mmol) were added to a solution of NaOH (29 mg, 0.74 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 49 (53 mg, 0.11 mmol, 38%) as an off-white solid. ¹H NMR (400 MHz, DMSO-*d*₆) δ 8.5 (m, 2H), 8.35 (m, 1H), 7.6 (d, 1H), 7.5 (s, 1H), 7.4 (d, 1H), 7.3 (m, 2H), 7.2 (m, 2H), 7.1 (m, 1H), 7.0 (m, 1H), 4.5 (d, 2H), 3.7 (s, 3H), 3.0 (m, 8H), 2.3 (s, 3H) ppm. UHPLC/MS (ESI): [*m*/*z*]: 481 [M+H]⁺.

### 30. Example Compound No. 50:

### (3-methoxypyridin-2-yl)methyl 2-(2-((2-(6,7,8,9-tetrahydro-3H-naphtho[1,2-d]imidazol-2-yl)ethyl)amino)ethyl)oxazole-4-carboxylate

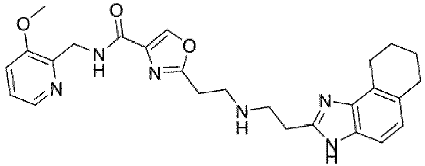

2-(6,7,8,9-tetrahydro-3H-naphtho[1,2-d]imidazol-2-yl)ethan-1-amine dihydrochloride (Chemspace) (100 mg, 0.35 mmol) and N-((3-methoxypyridin-2-yl)methyl)-2-vinyloxazole-4-carboxamide (90 mg, 0.35 mmol) were added to a solution of NaOH (35 mg, 0.87 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 50 (58 mg, 0.12 mmol, 35%) as an off-white solid. ¹H NMR (400 MHz, DMSO-*d*₆) δ 8.5 (m, 1H), 8.3 (m, 1H), 8.1 (m, 1H), 7.4 (d, 1H), 7.3 (m, 1H), 7.1 (d, 2H), 6.8 (d, 1H), 4.5 (d, 2H), 3.9 (s, 3H), 3.0 (m, 8H), 2.85 (m, 2H), 2,75 (m, 2H), 1.8 (m, 4H) ppm. UHPLC/MS (ESI): [*m*/*z*]: 475 [M+H]⁺.

### 31. Example Compound No. 51:

### 2-(2-((2-(5-(2-methoxyphenyl)-1H-benzo[d]imidazol-2-yl)ethyl)amino)ethyl)-N-((3-methoxypyridin-2-yl)methyl)oxazole-4-carboxamide

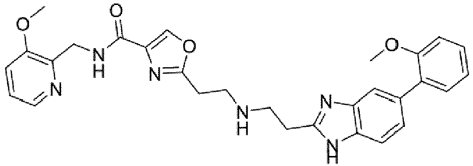

2-(5-(2-methoxyphenyl)-1H-benzo[d]imidazol-2-yl)ethan-1-amine dihydrochloride (Chemspace) (100 mg, 0.29 mmol) and N-((3-methoxypyridin-2-yl)methyl)-2-vinyloxazole-4-carboxamide (76 mg, 0.29 mmol) were added to a solution of NaOH (29 mg, 0.74 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 51 (66 mg, 0.13 mmol, 43%) as an off-white solid. ¹H NMR (400 MHz, DMSO-*d*₆) δ 8.5 (s, 1H), 8.3 (m, 1H), 8.1 (m, 1H), 7.5 (d, 1H), 7.4 (m, 2H), 7.3 (m, 3H), 7.2 (m, 1H), 7.1 (m, 1H), 7.0 (m, 1H), 4.5 (d, 2H), 3.9 (s, 3H), 3.7 (s, 3H), 3.0 (m, 8H) ppm. UHPLC/MS (ESI): *[m*/*z]:* 527 [M+H]⁺.

### 32. Example Compound No. 52:

### N-((3-methylpyridin-2-yl)methyl)-2-(2-((2-(5-phenyl-1H-benzo[d]imidazol-2-yl)ethyl)amino)ethyl)oxazole-4-carboxamide

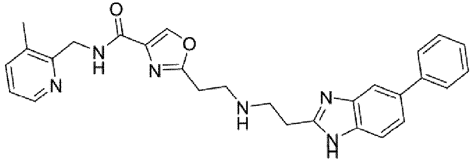

2-(5-phenyl-1H-benzo[d]imidazol-2-yl)ethan-1-amine dihydrochloride (Chemspace) (100 mg, 0.30 mmol) and N-((3-methylpyridin-2-yl)methyl)-2-vinyloxazole-4-carboxamide (75 mg, 0.30 mmol) were added to a solution of NaOH (30 mg, 0.75 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 52 (58 mg, 0.12 mmol, 40%) as an off-white solid. ¹H NMR (400 MHz, DMSO-*d*₆) δ 8.5 (m, 2H), 8.35 (m, 1H), 7.65 (m, 2H), 7.6 (m, 1H), 7.5 (m, 1H), 7.4 (m, 3H), 7.3 (m, 1H), 7.2 (m, 1H), 4.5 (d, 2H), 3.0 (m, 8H), 2.3 (s, 3H) ppm. UHPLC/MS (ESI): *[m*/*z]:* 481 [M+H]⁺.

### 33. Example Compound No. 53:

### N-((3-methoxypyridin-2-yl)methyl)-2-(2-((2-(5-phenyl-1H-benzo[d]imidazol-2-yl)ethyl)amino)ethyl)oxazole-4-carboxamide

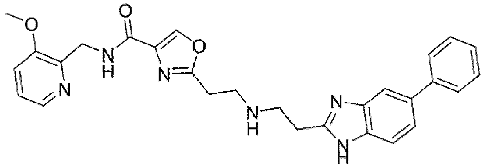

2-(5-phenyl-1H-benzo[d]imidazol-2-yl)ethan-1-amine dihydrochloride (Chemspace) (100 mg, 0.33 mmol) and N-((3-methoxypyridin-2-yl)methyl)-2-vinyloxazole-4-carboxamide (84 mg, 0.32 mmol) were added to a solution of NaOH (32 mg, 0.81 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 53 (87 mg, 0.18 mmol, 55%) as an off-white solid. ¹H NMR (400 MHz, DMSO-*d*₆) δ 8.5 (m, 1H), 8.35 (m, 1H), 8.1 (m, 1H), 7.7 (m, 2H), 7.5 (m, 1H), 7.4 (m, 3H), 7.3 (m, 2H), 4.5 (d, 2H), 3.8 (s, 3H), 3.0 (m, 8H) ppm. UHPLC/MS (ESI): *[m*/*z]:* 497 [M+H]⁺.

### 34. Example Compound No. 54:

### 2-(2-((2-(5-(1H-pyrrol-1-yl)-1H-benzo[d]imidazol-2-yl)ethyl)amino)ethyl)-N-((3-methoxypyridin-2-yl)methyl)oxazole-4-carboxamide

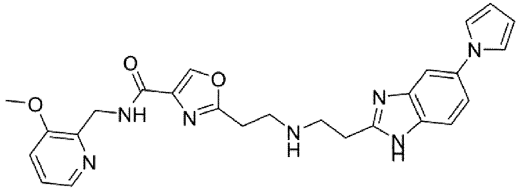

2-(5-(1H-pyrrol-1-yl)-1H-benzo[d]imidazol-2-yl)ethan-1-amine dihydrochloride (Chemspace) (100 mg, 0.33 mmol) and N-((3-methoxypyridin-2-yl)methyl)-2-vinyloxazole-4-carboxamide (87 mg, 0.33 mmol) were added to a solution of NaOH (34 mg, 0.84 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 54 (63 mg, 0.13 mmol, 39%) as an off-white solid. ¹H NMR (400 MHz, DMSO-*d*₆) δ 8.5 (m, 1H), 8.35 (m, 1H), 8.1 (m, 1H), 7.55 (m, 1H), 7.5 (m, 1H), 7.4 (m, 1H), 7.3 (m, 4H), 6.2 (m, 2H), 4.6 (d, 2H), 3.0 (m, 8H) ppm. UHPLC/MS (ESI): [*m*/*z*]: 486 [M+H]⁺.

### 35. Example Compound No. 55:

### N-((3-chloropyridin-2-yl)methyl)-2-(2-((2-(6,7-dihydro-1H-[1,4]dioxino[2',3':4,5]benzo[1,2-d]imidazol-2-yl)ethyl)amino)ethyl)oxazole-4-carboxamide

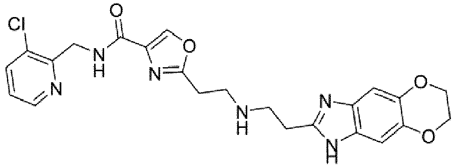

2-(6,7-dihydro-1H-[1,4]dioxino[2',3':4,5]benzo[1,2-d]imidazol-2-yl)ethan-1-amine dihydrochloride (Chemspace) (100 mg, 0.34 mmol) and N-((3-chloropyridin-2-yl)methyl)-2-vinyloxazole-4-carboxamide (90 mg, 0.34 mmol) were added to a solution of NaOH (35 mg, 0.86 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 55 (61 mg, 0.13 mmol, 37%) as an off-white solid. ¹H NMR (400 MHz, DMSO-*d*₆) δ 8.5 (m, 3H), 7.9 (m, 1H), 7.4 (m, 1H), 6.9 (m, 2H), 4.6 (d, 2H), 4.2 (m, 4H), 3.0 - 2.8 (m, 8H) ppm. UHPLC/MS (ESI): [*m*/*z*]: 483 [M+H]⁺.

### 36. Example Compound No. 56:

### 2-(2-((2-(6,7-dihydro-1H-[1,4]dioxino[2',3':4,5]benzo[1,2-d]imidazol-2-yl)ethyl)amino)ethyl)-N-((3-(trifluoromethyl)pyridin-2-yl)methyl)oxazole-4-carboxamide

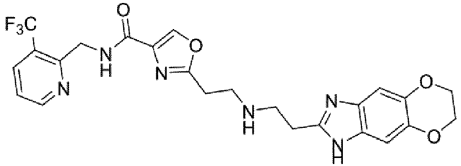

2-(6,7-dihydro-1H-[1,4]dioxino[2',3':4,5]benzo[1,2-d]imidazol-2-yl)ethan-1-amine dihydrochloride (Chemspace) (100 mg, 0.34 mmol) and N-((3-(trifluoromethyl)pyridin-2-yl)methyl)-2-vinyloxazole-4-carboxamide (102 mg, 0.34 mmol) were added to a solution of NaOH (35 mg, 0.86 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 56 (13 mg, 0.025 mmol, 7%) as an off-white solid. ¹H NMR (400 MHz, DMSO-*d*₆) δ 8.8 (m, 1H), 8.5 (m, 2H), 8.2 (m, 1H), 7.5 (m, 1H), 6.9 (m, 1H), 4.6 (d, 2H), 4.2 (m, 4H), 3.0 - 2.8 (m, 8H) ppm. UHPLC/MS (ESI): *[m*/*z]:* 517 [M+H]⁺.

### 37. Example Compound No. 57:

### 2-(2-((2-(5-(4-chlorophenyl)-1H-benzo[d]imidazol-2-yl)ethyl)amino)ethyl)-N-((3-fluoropyridin-2-yl)methyl)oxazole-4-carboxamide

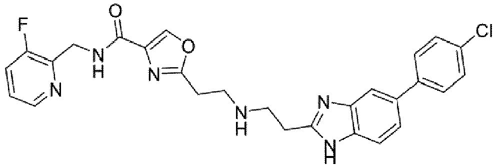

2-(5-(4-chlorophenyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (75 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 57 (11 mg, 0.02 mmol, 3%) as an off-white solid. ¹H NMR (400 MHz, DMSO-*d*₆) δ 8.5 (m, 2H), 8.35 (m, 1H), 7.7 (m, 4H), 7.5 - 7.3 (m, 5H), 4.6 (d, 2H), 3.0 (m, 8H) ppm. UHPLC/MS (ESI): [*m*/*z*]: 520 [M+H]⁺.

### 38. Example Compound No. 58:

### 2-(2-((2-(5-(1H-pyrrol-1-yl)-1H-benzo[d]imidazol-2-yl)ethyl)amino)ethyl)-N-((3-(trifluoromethyl)pyridin-2-yl)methyl)oxazole-4-carboxamide

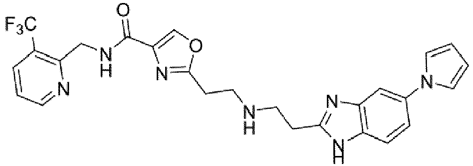

2-(5-(1H-pyrrol-1-yl)-1H-benzo[d]imidazol-2-yl)ethan-1-amine dihydrochloride (Chemspace) (100 mg, 0.33 mmol) and N-((3-trifluoromethylpyridin-2-yl)methyl)-2-vinyloxazole-4-carboxamide (100 mg, 0.33 mmol) were added to a solution of NaOH (34 mg, 0.84 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 58 (63 mg, 0.12 mmol, 37%) as an off-white solid. ¹H NMR (400 MHz, DMSO-*d*₆) δ 8.8 (m, 1H), 8.5 (m, 2H), 8.1 (m, 1H), 7.5 (m, 3H), 7.3 (m, 3H), 6.2 (m, 2H), 4.6 (d, 2H), 3.0 (m, 8H) ppm. UHPLC/MS (ESI): [*m*/*z*]: 524 [M+H]⁺.

### 39. Example Compound No. 59:

### 2-(2-((2-(5-(1H-pyrrol-1-yl)-1H-benzo[d]imidazol-2-yl)ethyl)amino)ethyl)-N-((3-chloropyridin-2-yl)methyl)oxazole-4-carboxamide

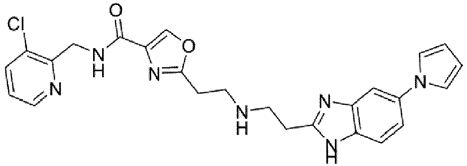

2-(5-(1H-pyrrol-1-yl)-1H-benzo[d]imidazol-2-yl)ethan-1-amine dihydrochloride (Chemspace) (100 mg, 0.33 mmol) and N-((3-chloropyridin-2-yl)methyl)-2-vinyloxazole-4-carboxamide (100 mg, 0.33 mmol) were added to a solution of NaOH (34 mg, 0.84 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 59 (66 mg, 0.14 mmol, 41%) as an off-white solid. ¹H NMR (400 MHz, DMSO-*d*₆) δ 8.5 (m, 3H), 7.9 (m, 1H), 7.55 (m, 1H), 7.5 (m, 1H), 7.4 (m, 1H), 7.3 (m, 3H), 6.2 (m, 2H), 4.6 (d, 2H), 3.0 (m, 8H) ppm. UHPLC/MS (ESI): [*m*/*z*]: 491 [M+H]⁺.

### 40. Example Compound No. 60:

### N-((3-fluoropyridin-2-yl)methyl)-2-(2-((2-(5-(p-tolyl)-1H-benzo[d]imidazol-2-yl)ethyl)amino)ethyl)oxazole-4-carboxamide

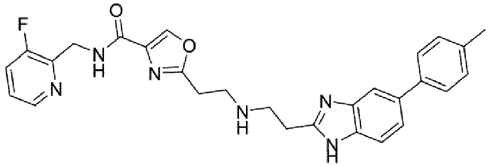

2-(5-(p-tolyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (77 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 60 (36 mg, 0.07 mmol, 23%) as an off-white solid. ¹H NMR (400 MHz, DMSO-*d*₆) δ 8.5 (m, 2H), 8.35 (m, 1H), 7.7 (m, 2H), 7.5 (m, 3H), 7.4 (m, 2H), 7.2 (m, 2H), 4.6 (d, 2H), 3.0 (m, 8H), 2.3 (s, 3H) ppm. UHPLC/MS (ESI): [*m*/*z*]: 499 [M+H]⁺.

### 41. Example Compound No. 61:

### 2-(2-((2-(5-(3-fluorophenyl)-1H-benzo[d]imidazol-2-yl)ethyl)amino)ethyl)-N-((3-fluoropyridin-2-yl)methyl)oxazole-4-carboxamide

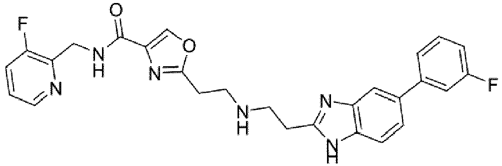

2-(5-(3-fluorophenyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (77 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 61 (44 mg, 0.088 mmol, 28%) as an off-white solid. ¹H NMR (400 MHz, DMSO-*d*₆) δ 8.5 (m, 2H), 8.3 (m, 1H), 7.7 (m, 2H), 7.5 (m, 5H), 7.1 (m, 1H), 4.6 (d, 2H), 3.0 (m, 8H) ppm. UHPLC/MS (ESI): [*m*/*z*]: 503 [M+H]⁺.

### 42. Example Compound No. 62:

### N-((3-fluoropyridin-2-yl)methyl)-2-(2-((2-(5-(o-tolyl)-1H-benzo[d]imidazol-2-yl)ethyl)amino)ethyl)oxazole-4-carboxamide

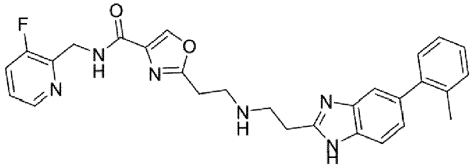

2-(5-(o-tolyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (77 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 62 (34 mg, 0.068 mmol, 22%) as an off-white solid. ¹H NMR (400 MHz, DMSO-*d*₆) δ 8.5 (m, 2H), 8.3 (m, 1H), 8.1 (s, 1H), 7.7 (m, 1H), 7.5 (m, 1H), 7.4 (m, 2H), 7.2 (m, 4 H), 7.0 (m, 1H), 4.6 (d, 2H), 3.0 (m, 8H), 2.2 (s, 3H) ppm. UHPLC/MS (ESI): [*m*/*z*]: 499 [M+H]⁺.

### 43. Example Compound No. 63

### N-((3-fluoropyridin-2-yl)methyl)-2-(2-((2-(5-(3-methoxyphenyl)-1H-benzo[d]imidazol-2-yl)ethyl)amino)ethyl)oxazole-4-carboxamide

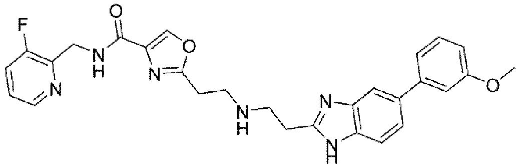

2-(5-(3-methoxyphenyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (70 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 63 (23 mg, 0.045 mmol, 15%) as an off-white solid. ¹H NMR (400 MHz, DMSO-*d*₆) δ 8.5 (m, 2H), 8.3 (m, 1H), 7.7 (m, 2H), 7.5 (m, 1H), 7.4 (m, 3H), 7.2 (m, 2H), 6.9 (m, 1H), 4.6 (d, 2H), 3.8 (s, 3H), 3.0 (m, 8H) ppm. UHPLC/MS (ESI): [*m*/*z*]: 515 [M+H]⁺.

### 44. Example Compound No. 64:

### N-((3-fluoropyridin-2-yl)methyl)-2-(2-((2-(5-(4-methoxyphenyl)-1H-benzo[d]imidazol-2-yl)ethyl)amino)ethyl)oxazole-4-carboxamide

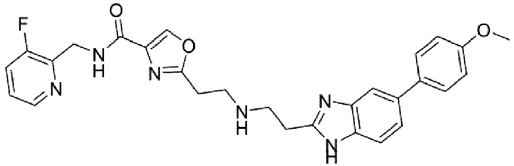

2-(5-(4-methoxyphenyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (70 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 64 (41 mg, 0.08 mmol, 26%) as an off-white solid. ¹H NMR (400 MHz, DMSO-*d*₆) δ 8.5 (m, 2H), 8.3 (m, 1H), 7.7 (m, 1H), 7.6 (m, 3H), 7.5 (m, 1H), 7.4 (m, 2H), 7.0 (m, 2H), 4.6 (d, 2H), 3.8 (s, 3H), 3.0 (m, 8H) ppm. UHPLC/MS (ESI): [*m*/*z*]: 515 [M+H]⁺.

### 45. Example Compound No. 65:

### N-((3-fluoropyridin-2-yl)methyl)-2-(2-((2-(5-(m-tolyl)-1H-benzo[d]imidazol-2-yl)ethyl)amino)ethyl)oxazole-4-carboxamide

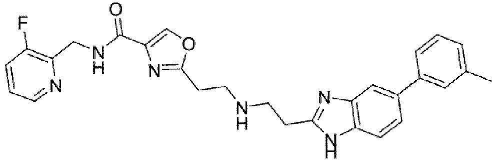

2-(5-(m-tolyl)-1H-benzo[d]imidazol-2-yl)ethan-1-amine dihydrochloride (Chemspace) (100 mg, 0.31 mmol) and N-((3-fluoropyridin-2-yl)methyl)-2-vinyloxazole-4-carboxamide (70 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 65 (10 mg, 0.02 mmol, 7%) as an off-white solid. ¹H NMR (400 MHz, DMSO-*d*₆) δ 8.5 (m, 2H), 8.3 (m, 1H), 7.7 (m, 2H), 7.5 - 7.3 (m, 5H), 7.1 (m, 1H), 4.6 (d, 2H), 3.0 (m, 8H), 2.3 (s, 3H) ppm. UHPLC/MS (ESI): *[m*/*z]:* 499 [M+H]⁺.

### 46. Example Compound No. 66:

### 2-(2-((2-(5-(4-fluorophenyl)-1H-benzo[d]imidazol-2-yl)ethyl)amino)ethyl)-N-(pyridin-2-ylmethyl)oxazole-4-carboxamide

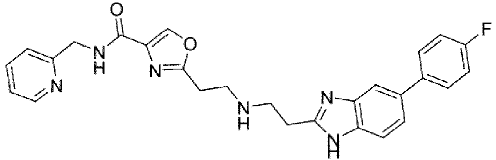

2-(5-(4-fluorophenyl)-1H-benzo[d]imidazol-2-yl)ethan-1-amine dihydrochloride (Chemspace) (100 mg, 0.31 mmol) and N-(pyridin-2-ylmethyl)-2-vinyloxazole-4-carboxamide (70 mg, 0.31 mmol) were added to a solution of NaOH (31 mg, 0.77 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 66 (27 mg, 0.056 mmol, 18%) as an off-white solid. ¹H NMR (400 MHz, DMSO-*d*₆) δ 8.8 (m, 1H), 8.5 (m, 2H), 7.7 (m, 4H), 7.5 (m, 1H), 7.4 (m, 1H), 7.2 (m, 5H), 4.5 (d, 2H), 3.0 (m, 8H) ppm. UHPLC/MS (ESI): *[m*/*z]:* 485 [M+H]⁺.

### 47. Example Compound No. 68:

### N-((3-fluoropyridin-2-yl)methyl)-2-(2-((2-(6-(2-methoxyethoxy)-1H-benzo[d]imidazol-2-yl)ethyl)amino)ethyl)oxazole-4-carboxamide

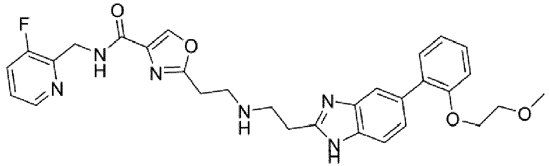

2-(6-(2-methoxyethoxy)-1H-benzo[d]imidazol-2-yl)ethan-1-amine hydrochloride (Chemspace) (100 mg, 0.37 mmol) and N-((3-fluoropyridin-2-yl)methyl)-2-vinyloxazole-4-carboxamide (91 mg, 0.37 mmol) were added to a solution of NaOH (22 mg, 0.55 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 68 (40 mg, 0.072 mmol, 19%) as an off-white solid. ¹H NMR (400 MHz, DMSO-*d*₆) δ 8.5 (m, 2H), 8.4 (m, 1H), 7.7 (m, 1H), 7.6 (s, 1H), 7.5 - 7.2 (m, 5H), 7.1 - 7.0 (m, 2H), 4.6 (d, 2H), 4.1 (d, 2H), 3.6 (d, 2H), 3.4 - 3.3 (m, 5H), 3.2 (s, 3H), 3.0 (m, 7H) ppm. UHPLC/MS (ESI): [*m*/*z*]: 559 [M+H]⁺.

### 48. Example Compound No. 69:

### 2-(2-((2-(5-(3-fluorophenyl)-1H-benzo[d]imidazol-2-yl)ethyl)amino)ethyl)-N-(pyridin-2-ylmethyl)oxazole-4-carboxamide

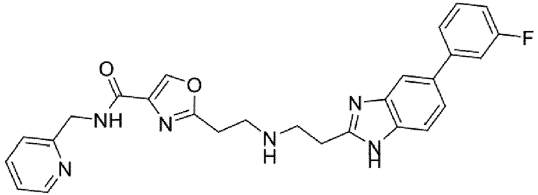

2-(5-(3-fluorophenyl)-1H-benzo[d]imidazol-2-yl)ethan-1-amine dihydrochloride (Chemspace) (100 mg, 0.31 mmol) and N-(pyridin-2-ylmethyl)-2-vinyloxazole-4-carboxamide (70 mg, 0.31 mmol) were added to a solution of NaOH (31 mg, 0.76 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 69 (19 mg, 0.039 mmol, 13%) as an off-white solid. ¹H NMR (400 MHz, DMSO-*d*₆) δ 8.8 (m, 1H), 8.5 (s, 1H), 8.45 (m, 2H), 7.8 (m, 2H), 7.5 (m, 5H), 7.3 (m, 2H), 7.1 (m, 1H), 4.5 (d, 2H), 3.0 (m, 8H) ppm. UHPLC/MS (ESI): [*m*/*z*]: 485 [M+H]⁺.

### 49. Example Compound No. 70:

### 2-(2-((2-(5-(3-methoxyphenyl)-1H-benzo[d]imidazol-2-yl)ethyl)amino)ethyl)-N-(pyridin-2-ylmethyl)oxazole-4-carboxamide

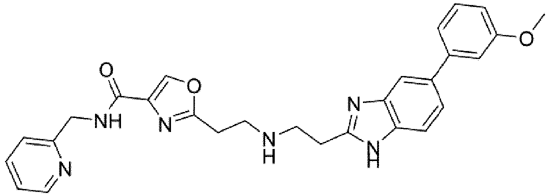

2-(5-(3-methoxyphenyl)-1H-benzo[d]imidazol-2-yl)ethan-1-amine dihydrochloride (Chemspace) (100 mg, 0.30 mmol) and N-(pyridin-2-ylmethyl)-2-vinyloxazole-4-carboxamide (70 mg, 0.30 mmol) were added to a solution of NaOH (31 mg, 0.76 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 70 (40 mg, 0.08 mmol, 27%) as an off-white solid. ¹H NMR (400 MHz, DMSO-*d*₆) δ 8.7 (m, 1H), 8.55 (s, 1H), 8.5 (m, 1H), 7.7 (m, 2H), 7.5 (m, 1H), 7.4 (m, 2H), 7.2 (m, 3H), 7.15 (m, 1H), 6.9 (m, 1H), 4.5 (d, 2H), 3.0 (m, 8H) ppm. UHPLC/MS (ESI): [*m*/*z*]: 497 [M+H]⁺.

### 50. Example Compound No. 71:

### 2-(2-((2-(5-(2-ethylphenyl)-1H-benzo[d]imidazol-2-yl)ethyl)amino)ethyl)-N-((3-fluoropyridin-2-yl)methyl)oxazole-4-carboxamide

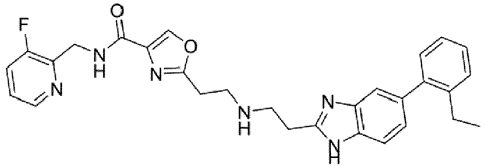

2-(5-(2-ethylphenyl)-1H-benzo[d]imidazol-2-yl)ethan-1-amine dihydrochloride (Chemspace) (100 mg, 0.30 mmol) and N-((3-fluoropyridin-2-yl)methyl)-2-vinyloxazole-4-carboxamide (73 mg, 0.31 mmol) were added to a solution of NaOH (30 mg, 0.74 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 71 (65 mg, 0.13 mmol, 42%) as an off-white solid. ¹H NMR (400 MHz, DMSO-*d*₆) δ 8.5 (m, 2H), 8.4 (m, 1H), 7.7 (m, 1H), 7.5 (m, 1H), 7.4 - 7.2 (m, 6H), 7.0 (m, 1H), 4.6 (d, 2H), 3.0 (m, 8H), 2.6 (m, 2H), 1.0 (t, 3H) ppm. UHPLC/MS (ESI): [*m*/*z*]: 513 [M+H]⁺.

### 51. Example Compound No. 72:

### N-((3-fluoropyridin-2-yl)methyl)-2-(2-((2-(5-(phenylethynyl)-1H-benzo[d]imidazol-2-yl)ethyl)amino)ethyl)oxazole-4-carboxamide

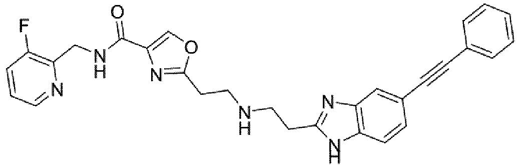

2-(5-(phenylethynyl)-1H-benzo[d]imidazol-2-yl)ethan-1-amine dihydrochloride (Chemspace) (100 mg, 0.30 mmol) and N-((3-fluoropyridin-2-yl)methyl)-2-vinyloxazole-4-carboxamide (74 mg, 0.26 mmol) were added to a solution of NaOH (30 mg, 0.75 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 72 (56 mg, 0.11 mmol, 37%) as an off-white solid. ¹H NMR (400 MHz, DMSO-*d*₆) δ 8.5 (m, 2H), 8.4 (m, 1H), 7.7 (m, 2H), 7.6 (m, 2H), 7.5 (m, 1H), 7.4 (m, 4H), 7.3 (m, 1H), 4.6 (d, 2H), 4.1 (d, 2H), 3.0 (m, 8H), 3.2 (s, 3H), 3.0 (m, 7H) ppm. UHPLC/MS (ESI): [*m*/*z*]: 509 [M+H]⁺.

### 52. Example Compound No. 73:

### N-((3-fluoropyridin-2-yl)methyl)-2-(2-((2-(5-(pyridin-3-ylethynyl)-1H-benzo[d]imidazol-2-yl)ethyl)amino)ethyl)oxazole-4-carboxamide

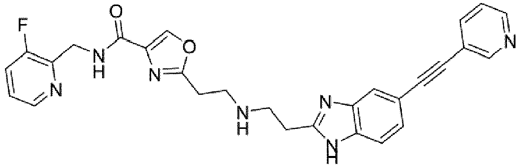

2-(5-(pyridin-3-ylethynyl)-1H-benzo[d]imidazol-2-yl)ethan-1-amine trihydrochloride (Chemspace) (100 mg, 0.27 mmol) and N-((3-fluoropyridin-2-yl)methyl)-2-vinyloxazole-4-carboxamide (67 mg, 0.27 mmol) were added to a solution of NaOH (38 mg, 0.94 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 73 (42 mg, 0.083 mmol, 30%) as an off-white solid. ¹H NMR (400 MHz, DMSO-*d*₆) δ 8.8 (s, 1H), 8.6 (m, 1H), 8.5 (m, 2H), 8.4 (m, 1H), 8.0 (m, 1H), 7.7 (m, 2H), 7.5 - 7.3 (m, 4H), 4.5 (d, 2H), 3.0 (m, 8H) ppm. UHPLC/MS (ESI): [*m*/*z*]: 510 [M+H]⁺.

### 53. Example Compound No. 74:

### N-((3-methoxypyridin-2-yl)methyl)-2-(2-((2-(5-(p-tolyl)-1H-benzo[d]imidazol-2-yl)ethyl)amino)ethyl)oxazole-4-carboxamide

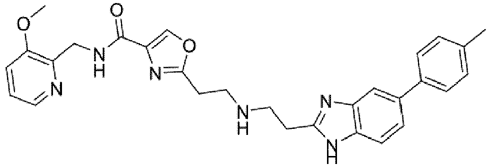

2-(5-(p-tolyl)-1H-benzo[d]imidazol-2-yl)ethan-1-amine dihydrochloride (Chemspace) (100 mg, 0.31 mmol) and N-((3-methoxypyridin-2-yl)methyl)-2-vinyloxazole-4-carboxamide (79 mg, 0.31 mmol) were added to a solution of NaOH (31 mg, 0.76 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 74 (48 mg, 0.09 mmol, 30%) as an off-white solid. ¹H NMR (400 MHz, DMSO-*d*₆) δ 8.5 (s, 1H), 8.3 (m, 1H), 8.1 (m, 1H), 7.65 (s, 1H), 7.5 - 7.2 (m, 8H), 4.5 (d, 2H), 3.9 (s, 3H), 3.0 (m, 8H) ppm. UHPLC/MS (ESI): [*m*/*z*]: 510 [M+H]⁺.

### 54. Example Compound No. 75:

### N-((3-fluoropyridin-2-yl)methyl)-2-(2-((2-(5-phenethyl-1H-benzo[d]imidazol-2-yl)ethyl)amino)ethyl)oxazole-4-carboxamide

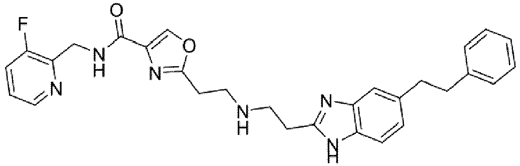

2-(5-phenethyl-1H-benzo[d]imidazol-2-yl)ethan-1-amine dihydrochloride (Chemspace) (100 mg, 0.3 mmol) and N-((3-methoxypyridin-2-yl)methyl)-2-vinyloxazole-4-carboxamide (73 mg, 0.3 mmol) were added to a solution of NaOH (30 mg, 0.74 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 75 (65 mg, 0.13 mmol, 42%) as an off-white solid. ¹H NMR (400 MHz, DMSO-*d*₆) δ 8.5 (m, 2H), 8.35 (m, 1H), 7.7 (m, 1H), 7.4 (m, 1H), 7.3 (m, 1H), 7.2 (m, 5H), 7.1 (m, 1H), 7.0 (m, 1H), 4.6 (d, 2H), 3.0 (m, 12H) ppm. UHPLC/MS (ESI): [*m*/*z*]: 513 [M+H]⁺.

### 55. Example Compound No. 76:

### 2-(2-((2-(5-((3-(dimethylcarbamoyl)phenyl)ethynyl)-1H-benzo[d]imidazol-2-yl)ethyl)amino)ethyl)-N-((3-fluoropyridin-2-yl)methyl)oxazole-4-carboxamide

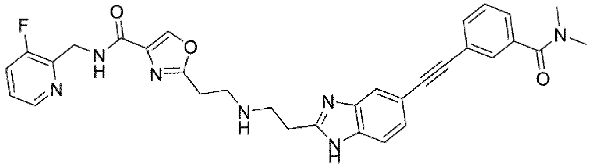

3-((2-(2-aminoethyl)-1H-benzo[d]imidazol-5-yl)ethynyl)-N,N-dimethylbenzamide dihydrochloride (Chemspace) (100 mg, 0.25 mmol) and N-((3-methoxypyridin-2-yl)methyl)-2-vinyloxazole-4-carboxamide (61 mg, 0.25 mmol) were added to a solution of NaOH (25 mg, 0.62 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 76 (21 mg, 0.036 mmol, 15%) as an off-white solid. ¹H NMR (400 MHz, DMSO-*d*₆) δ 8.5 (m, 2H), 8.35 (m, 1H), 7.7 (m, 2H), 7.6 (m, 1H), 7.55 (s, 1H), 7.45 (m, 2H), 7.4 (m, 2H), 7.3 (m, 1H), 4.6 (d, 2H), 3.0 (m, 14H) ppm. UHPLC/MS (ESI): [*m*/*z*]: 580 [M+H]⁺.

### 56. Example Compound No. 78:

### N-((3-fluoropyridin-2-yl)methyl)-2-(2-((2-(7-((3-methoxyphenyl)ethynyl)-1H-benzo[d]imidazol-2-yl)ethyl)amino)ethyl)oxazole-4-carboxamide

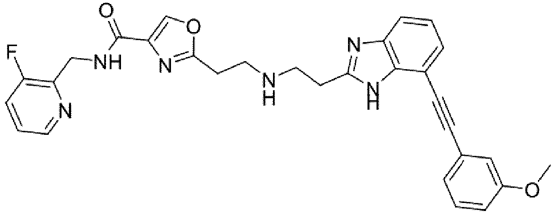

2-(2-(I2-azaneyl)ethyl)-7-((3-methoxyphenyl)ethynyl)-1H-benzo[d]imidazole dihydrochloride (Chemspace) (100 mg, 0.28 mmol) and N-((3-fluoropyridin-2-yl)methyl)-2-vinyloxazole-4-carboxamide (68 mg, 0.28 mmol) were added to a solution of NaOH (27 mg, 0.69 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 78 (78 mg, 0.15 mmol, 52%) as an off-white solid. ¹H NMR (400 MHz, DMSO-*d*₆) δ 8.6 (m, 2H), 8.35 (m, 1H), 7.7 - 7.0 (m, 9H), 4.6 (d, 2H), 3.8 (s, 3H), 3.0 (m, 8H) ppm. UHPLC/MS (ESI): [*m*/*z*]: 539 [M+H]⁺.

### 57. Example Compound No. 79:

### N-((3-fluoropyridin-2-yl)methyl)-2-(2-((2-(7-((3-(oxazol-2-yl)phenyl)ethynyl)-1H-benzo[d]imidazol-2-yl)ethyl)amino)ethyl)oxazole-4-carboxamide

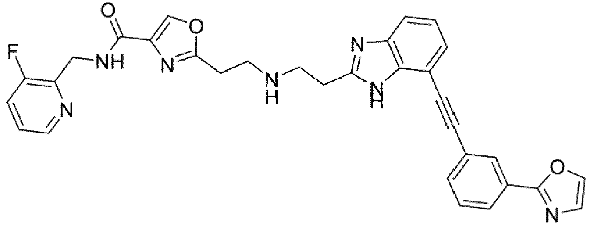

2-(3-((2-(2-(I2-azaneyl)ethyl)-1H-benzo[d]imidazol-7-yl)ethynyl)phenyl)oxazole dihydrochloride (Chemspace) (100 mg, 0.25 mmol) and N-((3-fluoropyridin-2-yl)methyl)-2-vinyloxazole-4-carboxamide (62 mg, 0.25 mmol) were added to a solution of NaOH (25 mg, 0.62 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 79 (34 mg, 0.06 mmol, 24%) as an off-white solid. ¹H NMR (400 MHz, DMSO-*d*₆) δ 8.5 (m, 2H), 8.35 (m, 1H), 8.25 (m, 1H), 8.2 (m, 1H), 8.0 (m, 1H), 7.7 - 7.1 (m, 8H), 4.6 (d, 2H), 3.0 (m, 8H) ppm. UHPLC/MS (ESI): [*m*/*z*]: 576 [M+H]⁺.

### 58. Example Compound No. 80:

### N-((3-fluoropyridin-2-yl)methyl)-2-(2-((2-(7-(pyridin-3-ylethynyl)-1H-benzo[d]imidazol-2-yl)ethyl)amino)ethyl)oxazole-4-carboxamide

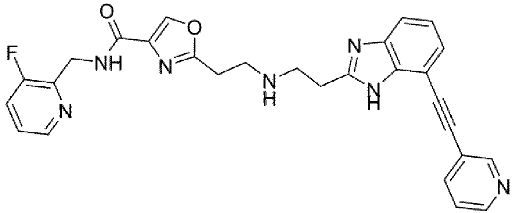

2-(2-(I2-azaneyl)ethyl)-7-(pyridin-3-ylethynyl)-1H-benzo[d]imidazole trihydrochloride (Chemspace) (100 mg, 0.27 mmol) and N-((3-fluoropyridin-2-yl)methyl)-2-vinyloxazole-4-carboxamide (67 mg, 0.27 mmol) were added to a solution of NaOH (38 mg, 0.94 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 80 (102 mg, 0.2 mmol, 74%) as an off-white solid. ¹H NMR (400 MHz, DMSO-*d*₆) δ 8.8 (s, 1H), 8.6 - 8.3 (m, 4H), 8.0 (m, 1H), 7.7 - 7.1 (m, 6H), 4.6 (d, 2H), 3.0 (m, 8H) ppm. UHPLC/MS (ESI): [*m*/*z*]: 510 [M+H]⁺.

### 59. Example Compound No. 81:

### 2-(2-((2-(5-(2,3-dihydrobenzo[b][1,4]dioxin-5-yl)-1H-benzo[d]imidazol-2-yl)ethyl)amino)ethyl)-N-((3-fluoropyridin-2-yl)methyl)oxazole-4-carboxamide

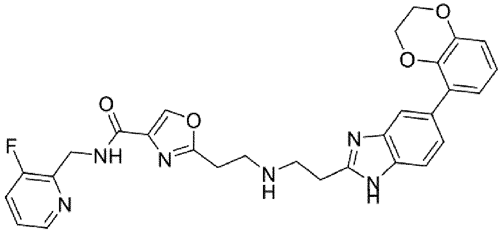

2-(5-(2,3-dihydrobenzo[b][1,4]dioxin-5-yl)-1H-benzo[d]imidazol-2-yl)ethan-1-amine dihydrochloride (Chemspace) (100 mg, 0.27 mmol) and N-((3-fluoropyridin-2-yl)methyl)-2-vinyloxazole-4-carboxamide (68 mg, 0.27 mmol) were added to a solution of NaOH (27 mg, 0.69 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 81 (58 mg, 0.1 mmol, 36%) as an off-white solid. ¹H NMR (400 MHz, DMSO-*d*₆) δ 8.55 (m, 2H), 8.35 (m, 1H), 7.7 (m, 1H), 7.55 (m, 1H), 7.45 (m, 1H), 7.3 (m, 1H), 7.2 (m, 1H), 6.85 (m, 3H), 4.6 (d, 2H), 4.3 (m, 4H), 3.0 (m, 8H) ppm. UHPLC/MS (ESI): [*m*/*z*]: 543 [M+H]⁺.

### 60. Example Compound No. 82:

### N-((3-fluoropyridin-2-yl)methyl)-2-(2-((2-(5-(5,6,7,8-tetrahydronaphthalen-1-yl)-1H-benzo[d]imidazol-2-yl)ethyl)amino)ethyl)oxazole-4-carboxamide

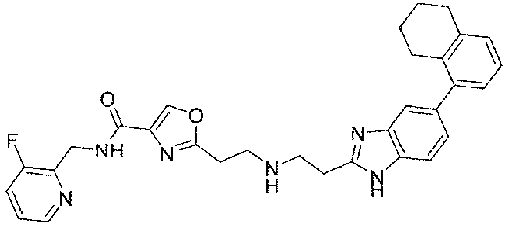

2-(5-(5,6,7,8-tetrahydronaphthalen-1-yl)-1H-benzo[d]imidazol-2-yl)ethan-1-amine dihydrochloride (Chemspace) (100 mg, 0.27 mmol) and N-((3-fluoropyridin-2-yl)methyl)-2-vinyloxazole-4-carboxamide (68 mg, 0.27 mmol) were added to a solution of NaOH (27 mg, 0.69 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 82 (52 mg, 0.1 mmol, 36%) as an off-white solid. ¹H NMR (400 MHz, DMSO-*d*₆) δ 8.5 (m, 2H), 8.35 (m, 1H), 7.7 (m, 1H), 7.5 (m, 1H), 7.4 (m, 1H), 7.3 (m, 1H), 7.1 - 7.0 (m, 4H), 4.6 (d, 2H), 3.0 (m, 8H), 2.8 (m, 2H), 1.7 (m, 4H), 1.2 (m, 2H) ppm. UHPLC/MS (ESI): [*m*/*z*]: 539 [M+H]⁺.

### 61. Example Compound No. 83:

### 2-(2-((2-(5-(2,3-dimethoxyphenyl)-1H-benzo[d]imidazol-2-yl)ethyl)amino)ethyl)-N-((3-fluoropyridin-2-yl)methyl)oxazole-4-carboxamide

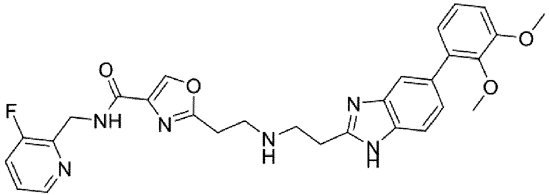

2-(5-(2,3-dimethoxyphenyl)-1H-benzo[d]imidazol-2-yl)ethan-1-amine dihydrochloride (Chemspace) (100 mg, 0.27 mmol) and N-((3-fluoropyridin-2-yl)methyl)-2-vinyloxazole-4-carboxamide (67 mg, 0.27 mmol) were added to a solution of NaOH (27 mg, 0.68 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 83 (28 mg, 0.05 mmol, 19%) as an off-white solid. ¹H NMR (400 MHz, DMSO-*d*₆) δ 8.5 (m, 2H), 8.35 (m, 1H), 7.7 (m, 1H), 7.5 (m, 2H), 7.4 (m, 1H), 7.2 (m, 1H), 7.1 (m, 1H), 7.0 (m, 1H), 6.9 (m, 1H), 4.6 (d, 2H), 3.8 (s, 3H), 3.5 (s, 3H), 3.0 (m, 8H) ppm. UHPLC/MS (ESI): [*m*/*z*]: 545 [M+H]⁺.

### 62. Example Compound No. 84:

### 2-(2-((2-(5-(2,3-dimethylphenyl)-1H-benzo[d]imidazol-2-yl)ethyl)amino)ethyl)-N-((3-fluoropyridin-2-yl)methyl)oxazole-4-carboxamide

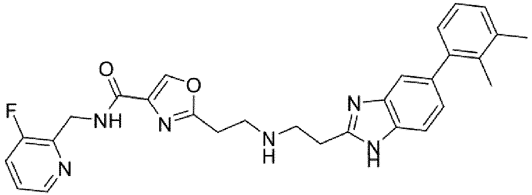

2-(5-(2,3-dimethylphenyl)-1H-benzo[d]imidazol-2-yl)ethan-1-amine dihydrochloride (Chemspace) (100 mg, 0.3 mmol) and N-((3-fluoropyridin-2-yl)methyl)-2-vinyloxazole-4-carboxamide (73 mg, 0.3 mmol) were added to a solution of NaOH (30 mg, 0.74 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 84 (46 mg, 0.09 mmol, 30%) as an off-white solid. ¹H NMR (400 MHz, DMSO-*d*₆) δ 8.5 (m, 2H), 8.35 (m, 1H), 7.7 (m, 1H), 7.5 (m, 1H), 7.4 (m, 1H), 7.3 (s, 1H), 7.1 - 7.0 (m, 4H), 4.6 (d, 2H), 3.0 (m, 8H), 2.3 (s, 3H), 2.1 (s, 3H) ppm. UHPLC/MS (ESI): [*m*/*z*]: 513 [M+H]⁺.

### 63. Example Compound No. 85:

### 2-(2-((2-(5-(3-chlorophenyl)-1H-benzo[d]imidazol-2-yl)ethyl)amino)ethyl)-N-((3-fluoropyridin-2-yl)methyl)oxazole-4-carboxamide

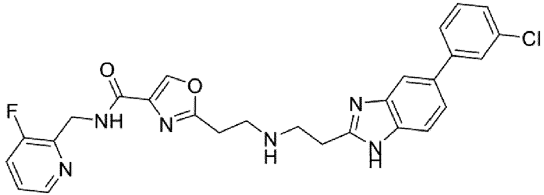

2-(5-(3-chlorophenyl)-1H-benzo[d]imidazol-2-yl)ethan-1-amine dihydrochloride (Chemspace) (100 mg, 0.29 mmol) and N-((3-fluoropyridin-2-yl)methyl)-2-vinyloxazole-4-carboxamide (72 mg, 0.29 mmol) were added to a solution of NaOH (29 mg, 0.73 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 85 (108 mg, 0.2 mmol, 72%) as an off-white solid. ¹H NMR (400 MHz, DMSO-*d*₆) δ 8.5 (m, 2H), 8.35 (m, 1H), 7.75 - 7.6 (m, 4), 7.55 - 7.35 (m, 5H), 4.6 (d, 2H), 3.0 (m, 8H) ppm. UHPLC/MS (ESI): [*m*/*z*]: 520 [M+H]⁺.

### 64. Example Compound No 86:

### N-((3-fluoropyridin-2-yl)methyl)-2-(2-((2-(5-(2-(oxazol-2-yl)phenyl)-1H-benzo[d]imidazol-2-yl)ethyl)amino)ethyl)oxazole-4-carboxamide

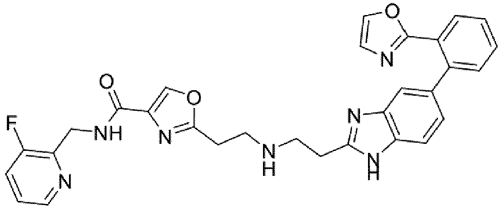

2-(5-(2-(oxazol-2-yl)phenyl)-1H-benzo[d]imidazol-2-yl)ethan-1-amine (Chemspace) (100 mg, 0.33 mmol) and N-((3-methoxypyridin-2-yl)methyl)-2-vinyloxazole-4-carboxamide (81 mg, 0.33 mmol) were added to a solution of NaOH (7 mg, 0.2 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 86 (50 mg, 0.09 mmol, 27%) as an off-white solid. ¹H NMR (400 MHz, DMSO-*d*₆) δ 8.5 (m, 2H), 8.35 (m, 1H), 7.9 (s, 1H), 7.8 - 7.2 (m, 8H), 6.9 (m, 1H), 4.6 (d, 2H), 3.0 (m, 8H) ppm. UHPLC/MS (ESI): [*m*/*z*]: 552 [M+H]⁺.

### 65. Example Compound No. 87:

### N-((3-fluoropyridin-2-yl)methyl)-2-(2-((2-(5-(3-(oxazol-2-yl)phenyl)-1H-benzo[d]imidazol-2-yl)ethyl)amino)ethyl)oxazole-4-carboxamide

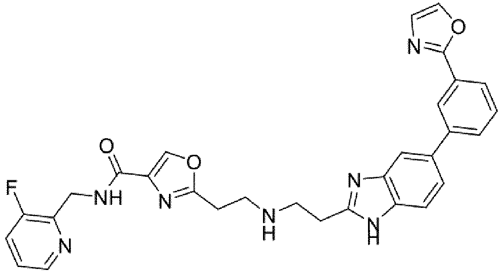

2-(5-(3-(oxazol-2-yl)phenyl)-1H-benzo[d]imidazol-2-yl)ethan-1-amine (Chemspace) (100 mg, 0.33 mmol) and N-((3-methoxypyridin-2-yl)methyl)-2-vinyloxazole-4-carboxamide (81 mg, 0.33 mmol) were added to a solution of NaOH (7 mg, 0.2 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 87 (54 mg, 0.1 mmol, 30%) as an off-white solid. ¹H NMR (400 MHz, DMSO-*d*₆) δ 8.5 (m, 2H), 8.35 (m, 1H), 8.2 (m, 2H), 7.95 (m, 1H), 7.8 (m, 2H), 7.7 - 7.55 (m, 3H), 7.45 - 7.35 (m, 3H), 4.6 (d, 2H), 3.0 (m, 8H) ppm. UHPLC/MS (ESI): [*m*/*z*]: 552 [M+H]⁺.

### 66. Example Compound No. 88:

### 2-(2-((2-(5-(2,3-dihydrobenzo[b][1,4]dioxin-6-yl)-1H-benzo[d]imidazol-2-yl)ethyl)amino)ethyl)-N-((3-fluoropyridin-2-yl)methyl)oxazole-4-carboxamide

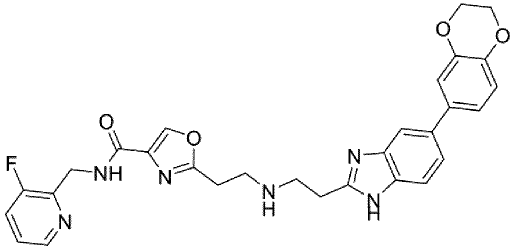

2-(5-(2,3-dihydrobenzo[b][1,4]dioxin-6-yl)-1H-benzo[d]imidazol-2-yl)ethan-1-amine dihydrochloride (Chemspace) (100 mg, 0.27 mmol) and N-((3-methoxypyridin-2-yl)methyl)-2-vinyloxazole-4-carboxamide (67 mg, 0.27 mmol) were added to a solution of NaOH (27 mg, 0.68 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 88 (10 mg, 0.02 mmol, 7%) as an off-white solid. ¹H NMR (400 MHz, DMSO-*d*₆) δ 8.5 (m, 2H), 8.35 (m, 1H), 7.7 (m, 1H), 7.6 (s, 1H), 7.45 - 7.3 (m, 3H), 7.1 (m, 2H), 6.9 (m, 1H), 4.6 (d, 2H), 4.3 (s, 4H), 3.0 (m, 8H), 2.7 (m, 4H), 1.7 (m, 4H) ppm. UHPLC/MS (ESI): [*m*/*z*]: 543 [M+H]⁺.

### 67. Example Compound No. 89:

### N-((3-fluoropyridin-2-yl)methyl)-2-(2-((2-(5-(5,6,7,8-tetrahydronaphthalen-2-yl)-1H-benzo[d]imidazol-2-yl)ethyl)amino)ethyl)oxazole-4-carboxamide

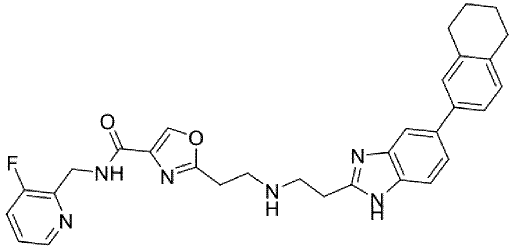

2-(5-(5,6,7,8-tetrahydronaphthalen-2-yl)-1H-benzo[d]imidazol-2-yl)ethan-1-amine dihydrochloride (Chemspace) (100 mg, 0.27 mmol) and N-((3-methoxypyridin-2-yl)methyl)-2-vinyloxazole-4-carboxamide (68 mg, 0.27 mmol) were added to a solution of NaOH (27 mg, 0.69 mmol) in 10 ml water. The resulting mixture were heated at 80 °C under stirring for 72 hours. After cooling at room temperature, the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 89 (30 mg, 0.06 mmol, 20%) as an off-white solid. ¹H NMR (400 MHz, DMSO-*d*₆) δ 8.5 (m, 2H), 8.35 (m, 1H), 7.65 (m, 2H), 7.5 (m, 1H), 7.3 (m, 4H), 7.1 (m, 1H), 4.6 (d, 2H), 3.0 (m, 8H), 2.7 (m, 4H), 1.7 (m, 4H) ppm. UHPLC/MS (ESI): [*m*/*z*]: 539 [M+H]⁺.

### 68. Example Compound No. 90:

### N-((3-fluoropyridin-2-yl)methyl)-2-(2-((2-(5-(4-(oxazol-2-yl)phenyl)-1H-benzo[d]imidazol-2-yl)ethyl)amino)ethyl)oxazole-4-carboxamide

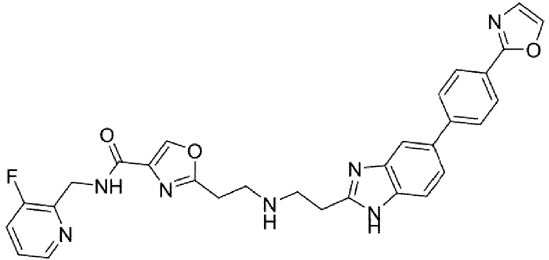

2-(5-(4-(oxazol-2-yl)phenyl)-1H-benzo[d]imidazol-2-yl)ethan-1-amine dihydrochloride (Chemspace) (100 mg, 0.29 mmol) and N-((3-methoxypyridin-2-yl)methyl)-2-vinyloxazole-4-carboxamide (73 mg, 0.29 mmol) were added to a solution of NaOH (29 mg, 0.73 mmol) in 10 ml water. The resulting mixture were heated at 80 °C under stirring for 72 hours. After cooling at room temperature,y the reaction mixture was adjusted to pH 6 by using 2N HCl and extracted with dichloromethane (3x 10 ml). The combined organic phase was washed with brine, dried over sodium sulfate, filtered and concentrated under reduced pressure. The crude material was purified by flash column chromatography (CH₂Cl₂/MeOH) to obtain the titled compound 90 (30 mg, 0.05 mmol, 19%) as an off-white solid. ¹H NMR (400 MHz, DMSO-*d*₆) δ 8.5 (m, 2H), 8.35 (m, 1H), 8.25 (m, 1H), 8.05 (m, 2H), 7.85 (m, 3H), 7.7 (m, 1H), 7.5 (m, 2H), 7.35 (m, 2H), 4.6 (d, 2H), 3.0 (m, 8H) ppm. UHPLC/MS (ESI): [*m*/*z*]: 552 [M+H]⁺.

## Claims

1. Compounds according to formula (I) 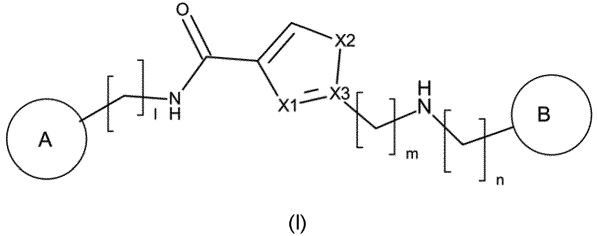 wherein
I is an integer of 1 or 2;
m and n are independently an integer of 1, 2 or 3;
X¹ is N, S or O;
X² is N, S, O or CR⁴;
X³ is C or N;
with the proviso that one of X¹ and X² is N
and if X³ is N, then X² is CR⁴;
and wherein
R⁴ represents
- H,
- halogen,
- linear or branched C₁-C₃-alkyl,
- linear or branched C₁-C₃-haloalkyl, or
A represents one of the following groups (a-1), (a-2), (a-3), (a-4) and (a-5) 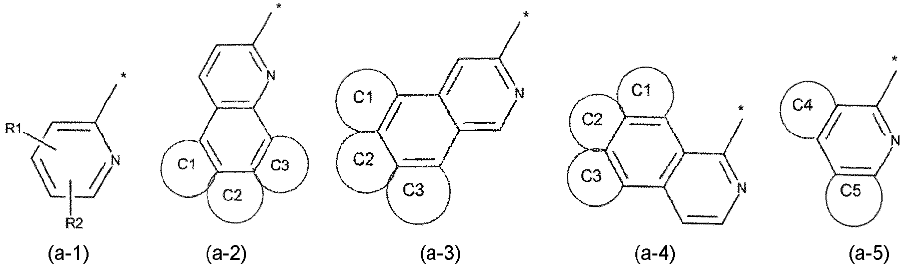
wherein * indicates the binding position;
R¹ and R² independently represent
- H,
- halogen,
- linear or branched C₁-C₃-alkyl,
- linear or branched C₁-C₃-haloalkyl,
- linear or branched C₁-C₃-alkoxy;
in formulae (a-2), (a-3) and (a-4) one of C1, C2 and C3 is present and in formula (a-5) both C4 and C5 are present, and
C1, C2, C3, C4 and C5 independently respresent
- a fused 6-membered aryl ring,
- a fused 5- or 6-membered heteroaryl ring,
- a fused 5- or 6-membered cycloalkyl ring,
- a fused 5- or 6-membered heterocyclyl ring; and
wherein the groups (a-2), (a-3), (a-4) and (a-5) carry 0, 1, 2 or 3 substituents, which are independently selected from
- halogen,
- linear or branched C₁-C₃-alkyl,
- linear or branched C₁-C₃-haloalkyl,
- linear or branched C₁-C₃-alkoxy;
B represents one of the following groups (b-1), (b-2) and (b-3) 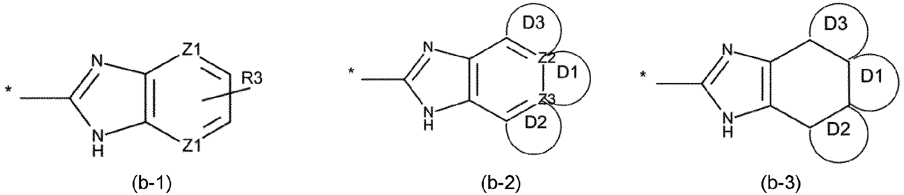
wherein * indicates the binding position;
R³ represents
- H,
- unsubstituted or substituted 6-membered aryl,
- unsubstituted or substituted 5- or 6-membered heteroaryl,
- unsubstituted or substituted bicyclic heteroaryl,
- 5- or 6-membered cycloalkyl,
- 5- or 6-membered heterocyclyl,
- 5- or 6-membered heterocyclylalkyl,
- 6-membered arylalkyl,
- unsubstituted or substituted 6-membered arylalkinyl
- unsubstituted or substituted 5- or 6-membered heteroarylalkinyl, or
- a phenyl group, which forms a fused bicyclic ring with a 5- or 6-membered cycloalkyl- or heterocyclyl group;
Z¹ represents N or C,
with the proviso that not more than one Z¹ represents N;
in formulae (b-2) and (b-3) one of D1, D2 and D3 is present and respresents
- a fused 6-membered aryl ring,
- a fused 5- or 6-membered heteroaryl ring,
- a fused 5- or 6-membered cycloalkyl ring,
- a fused 5- or 6-membered heterocyclyl ring; and
wherein the groups (b-2) and (b-3) carry 0, 1, 2 or 3 substituents, which are independently selected from
- halogen,
- linear or branched C₁-C₃-alkyl,
- linear or branched C₁-C₃-haloalkyl,
- linear or branched C₁-C₃-alkoxy;
Z² and Z³ represent N or C,
with the proviso that Z² may represent N when D2 is present and Z² may represent N when D3 is present;
and wherein the compounds (I) are **characterized in that** at least one of the groups A and B contains at least 3 rings;
and pharmaceutically acceptable salts thereof.

2. Compounds according to claim 1, wherein
R³ represents
- H,
- unsubstituted or substituted 6-membered aryl,
- unsubstituted or substituted 5- or 6-membered heteroaryl,
- unsubstituted or substituted bicyclic heteroaryl,
- 5- or 6-membered cycloalkyl,
- 5- or 6-membered heterocyclyl,
- 5- or 6-membered heterocyclylalkyl,
- 6-membered arylalkinyl.

3. Compounds according to claim 1 or 2,
wherein
I is an integer of 1 or 2;
m and n are independently an integer of 1, 2 or 3;
X¹ is N, S or O;
X² is N, S, O or CR⁴;
X³ is C or N;
with the proviso that one of X¹ and X² is N
and if X³ is N, then X² is CR⁴;
and wherein
R⁴ represents H;
A represents one of the following groups (a-1), (a-2), (a-3), (a-4) and (a-5) 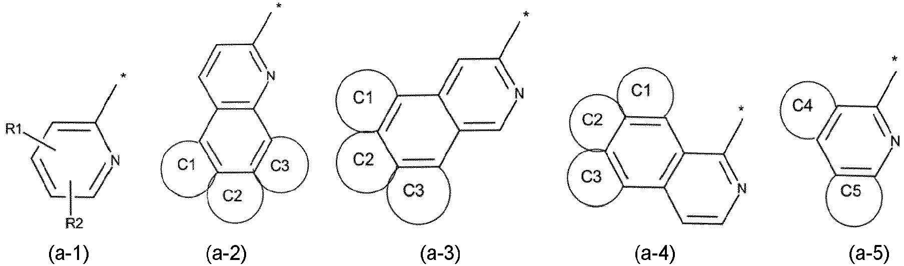
wherein * indicates the binding position;
R¹ and R² independently represent
- hydrogen,
- halogen,
- linear or branched C₁-C₃-alkyl,
- linear or branched C₁-C₃-haloalkyl,
- linear or branched C₁-C₃-alkoxy;
in formulae (a-2), (a-3) and (a-4) one of C1, C2 and C3 is present and in formula (a-5) both C4 and C5 are present, and
C1, C2, C3, C4 and C5 respresent a fused phenyl ring; and
wherein the groups (a-2), (a-3), (a-4) and (a-5) carry 0 or 1 substituent, which is selected from
- halogen,
- linear or branched C₁-C₃-alkyl,
- linear or branched C₁-C₃-haloalkyl,
- linear or branched C₁-C₃-alkoxy;
B represents one of the following groups (b-1), (b-2) and (b-3) 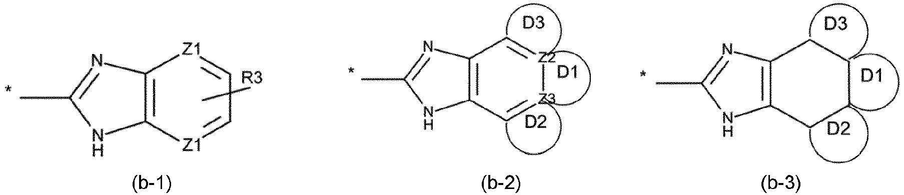
wherein * indicates the binding position;
R³ represents
- H,
- unsubstituted or substituted phenyl,
- unsubstituted or substituted 5- or 6-membered heteroaryl,
- unsubstituted or substituted bicyclic heteroaryl,
- 6-membered heterocyclyl,
- 6-membered heterocyclylalkyl,
- phenylalkyl,
- unsubstituted or substituted phenylethinyl or unsubstituted or substituted 6-membered heteroarylethinyl, or
- a phenyl group, which forms a fused bicyclic ring with a 5- or 6-membered cycloalkyl- or heterocyclyl group;
Z¹ represents N or C,
with the proviso that not more than one Z¹ represents N;
in formulae (b-2) and (b-3) one of D1, D2 and D3 is present and respresents
- a fused phenyl ring,
- a fused 6-membered heteroaryl ring,
- a fused 6-membered cycloalkyl ring,
- a fused 5- or 6-membered heterocyclyl ring; and
wherein the groups (b-2) and (b-3) carry 0 or 1 substituent, which is selected from
- halogen,
- linear or branched C₁-C₃-alkyl,
- linear or branched C₁-C₃-haloalkyl,
- linear or branched C₁-C₃-alkoxy;
Z² and Z³ represent N or C,
with the proviso that Z² may represent N when D2 is present and Z² may represent N when D3 is present;
and wherein the compounds (I) are **characterized in that** at least one of the groups A and B contains at least 3 rings;
and pharmaceutically acceptable salts thereof.

4. Compounds according to any one of claims 1 to 3, wherein
R¹ and R² independently represent
- halogen,
- linear or branched C₁-C₃-alkyl,
- linear or branched C₁-C₃-haloalkyl,
- linear or branched C₁-C₃-alkoxy; and
R³ represents
- H,
- unsubstituted or substituted phenyl,
- unsubstituted or substituted 5- or 6-membered heteroaryl,
- unsubstituted or substituted bicyclic heteroaryl,
- 6-membered heterocyclyl,
- 6-membered heterocyclylalkyl, and
- phenylethinyl.

5. Compounds according to any one of claims 1 to 4, which are **characterized in that** one of the groups A and B contains 3 rings; and pharmaceutically acceptable salts thereof.

6. Compounds according to any one of the claims 1 to 5, wherein
- the group A is a group (a-1); and/or
- the group A is a group (a-2), (a-3) or (a-5); and/or
- the group A is a group (a-5); and/or
- the group B is a group (b-1) or (b-2);
and pharmaceutically acceptable salts thereof.

7. Compounds according to any one of claims 1 to 6, wherein possible substituents are selected from the following groups
- halogen substituents represent F, Cl and Br,
- linear or branched C₁-C₃-alkyl substituents represent methyl and ethyl,
- linear or branched C₁-C₃-haloalkyl substituents represent trifluoromethyl (CF₃),
- linear or branched C₁-C₃-alkoxy substituents represent methoxy;
- 5- or 6-membered heteroaryl substituents represent oxazolyl,
- an alkoxyalkylether substituent represents a methoxyethylether group,
- a dialkylaminocarbonyl substituent represents dimethylaminocarbonyl,
preferably possible substituents are selected from the following groups
- halogen substituents represent F, Cl and Br,
- linear or branched C₁-C₃-alkyl substituents represent methyl,
- linear or branched C₁-C₃-haloalkyl substituents represent trifluoromethyl (CF₃),
- linear or branched C₁-C₃-alkoxy substituents represent methoxy;
and pharmaceutically acceptable salts thereof.

8. Compounds according to any one of claims 1 to 7, wherein X¹, X² and X³ are selected to form one of the following groups: 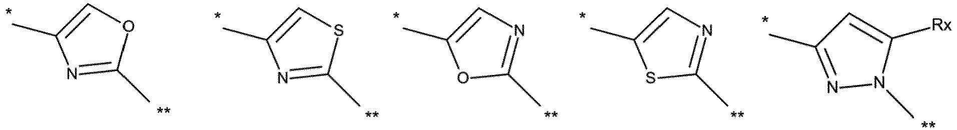
wherein * indicates the binding site to the aminocarbonyl-group and ** indicates the binding site to the - [(CH₂)]ₘ-amino-[(CH₂)]ₙ- group;
and wherein
R⁴ represents
- H,
- halogen,
- linear or branched C₁-C₃-alkyl,
- linear or branched C₁-C₃-haloalkyl; or
wherein preferably X¹, X² and X³ are selected to form one of the following groups: 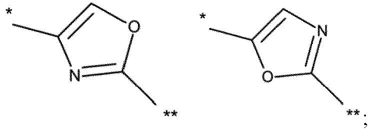 or
wherein preferably X¹, X² and X³ are selected to form one of the following groups: 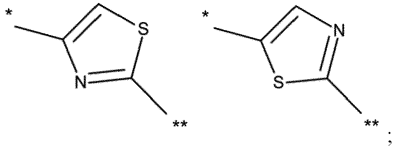
and pharmaceutically acceptable salts thereof.

9. Compounds according to anyone of the preceding claims, wherein for group A the following among groups (a-1), (a-2), (a-3) and (a-5) are selected: 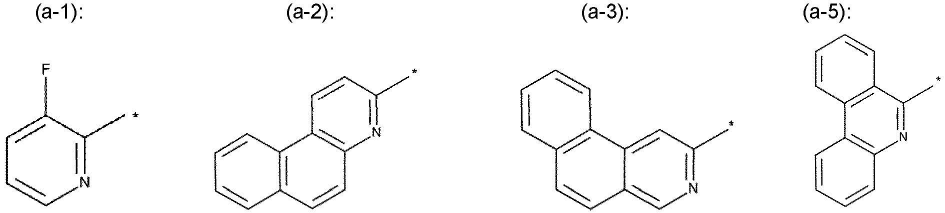 and pharmaceutically acceptable salts thereof.

10. Compounds according to any one of the preceding claims, which are selected from
| **No.** | Chemical Structure |
|---|---|
| **1** | 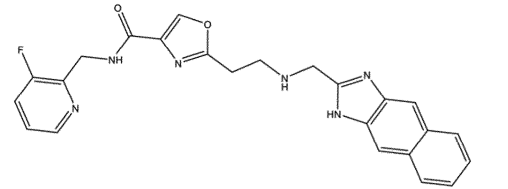 |
| **2** | 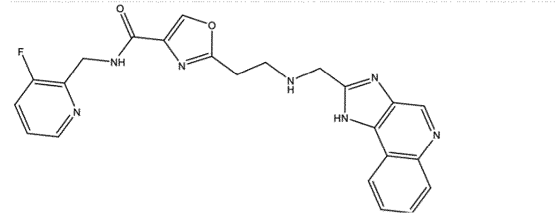 |
| **3** | 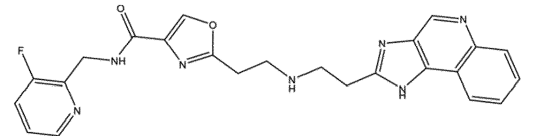 |
| **4** | 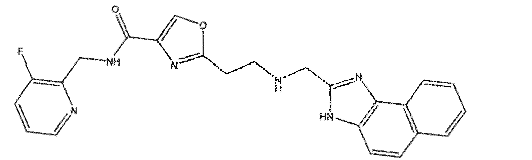 |
| **5** | 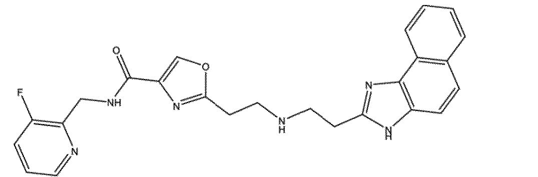 |
| **6** | 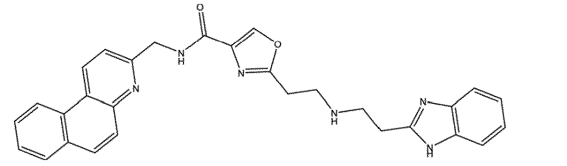 |
| **7** | 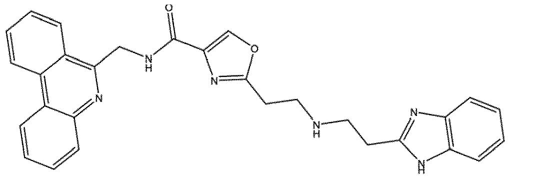 |
| **8** | 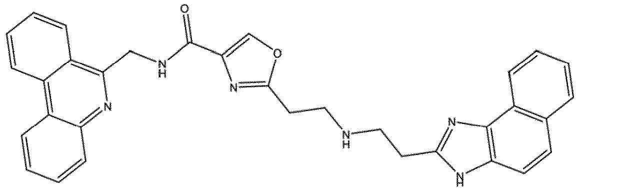 |
| **9** | 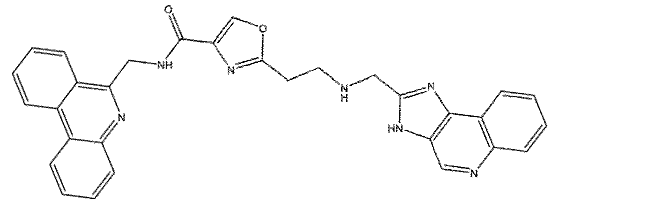 |
| **10** | 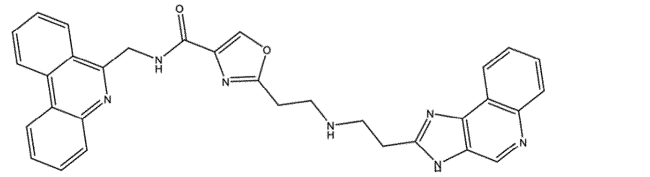 |
| **11** | 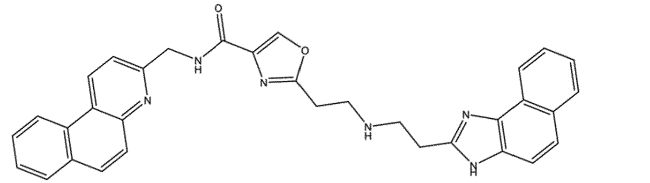 |
| **12** | 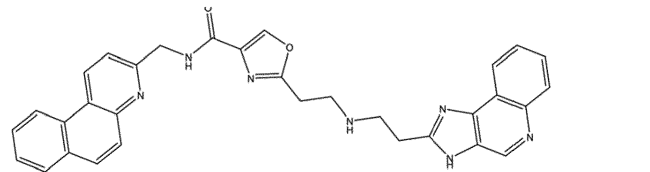 |
| **13** | 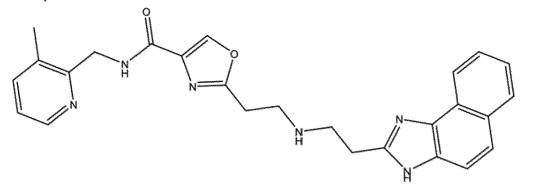 |
| **14** | 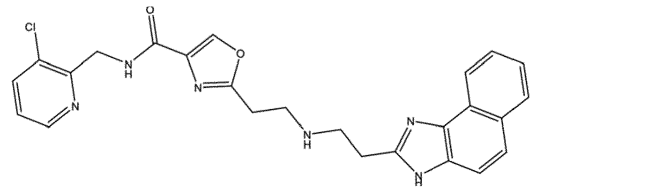 |
| **15** | 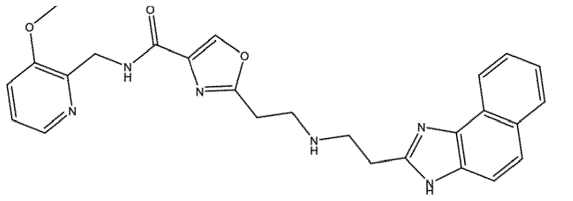 |
| **16** | 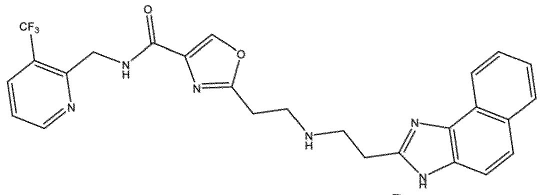 |
| **17** | 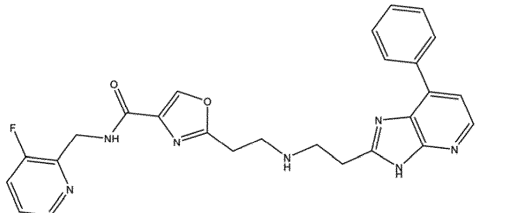 |
| **18** | 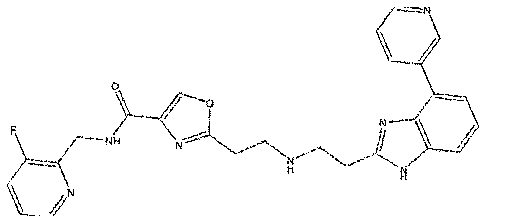 |
| **19** | 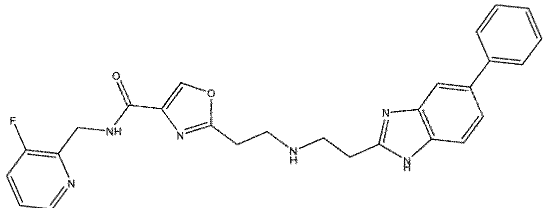 |
| **20** | 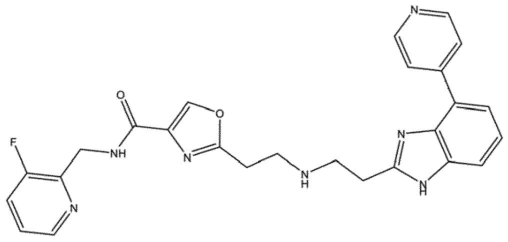 |
| **21** | 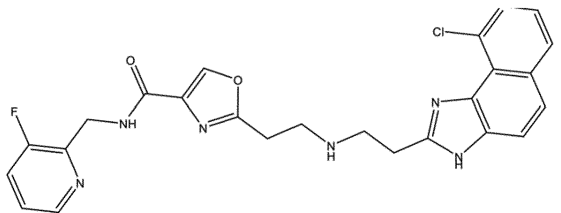 |
| **22** | 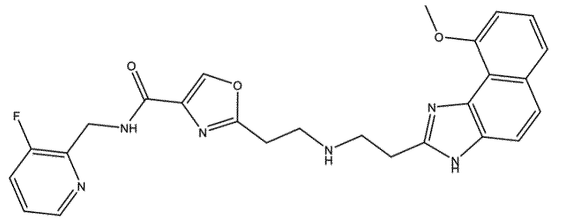 |
| **23** | 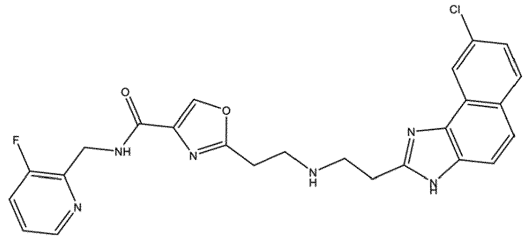 |
| **24** | 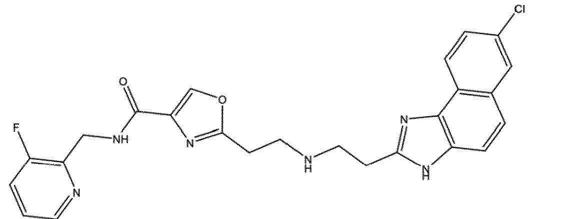 |
| **25** | 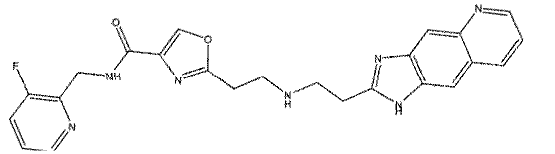 |
| **26** | 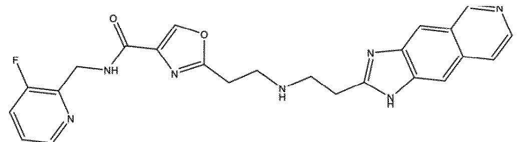 |
| **27** | 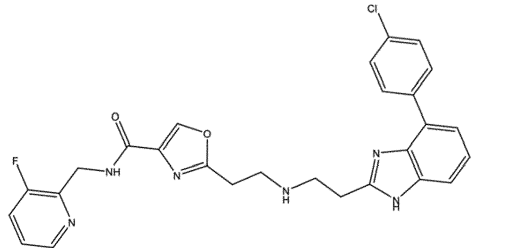 |
| **28** | 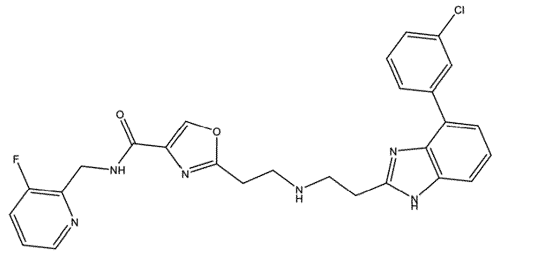 |
| **29** | 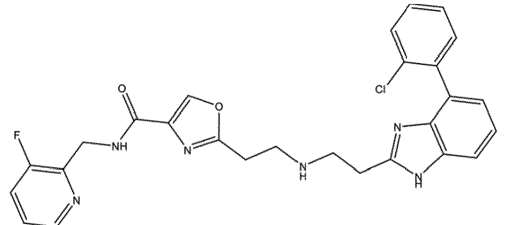 |
| **30** | 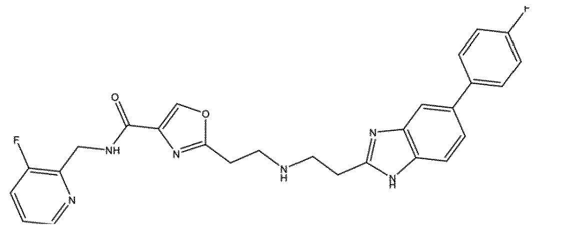 |
| **31** | 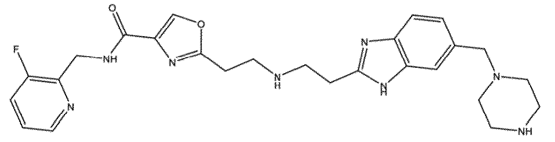 |
| **32** | 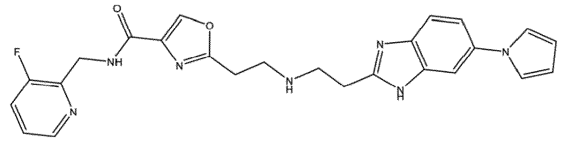 |
| **33** | 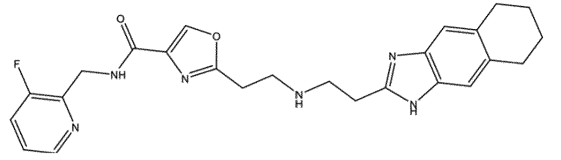 |
| **34** | 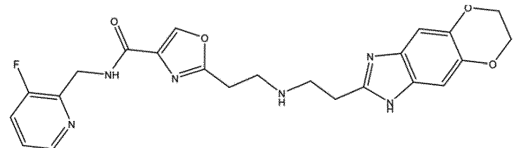 |
| **35** | 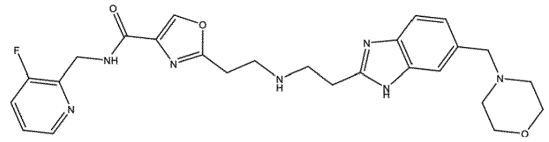 |
| **36** | 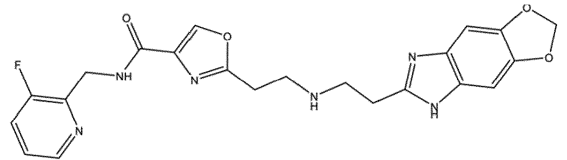 |
| **37** | 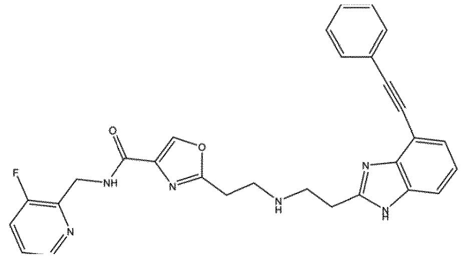 |
| **38** | 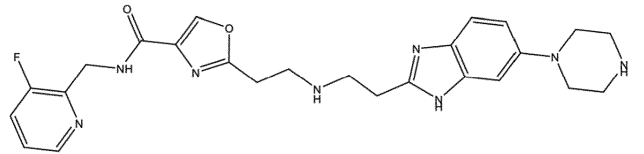 |
| **39** | 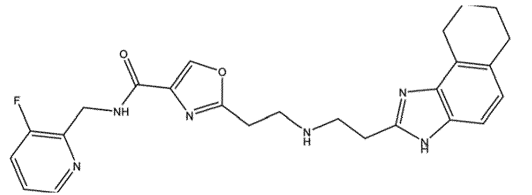 |
| **40** | 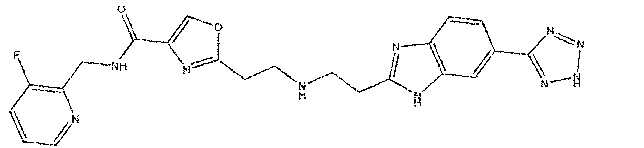 |
| **41** | 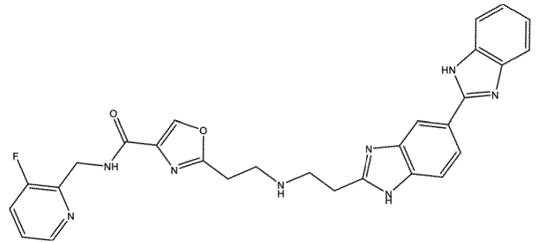 |
| **42** | 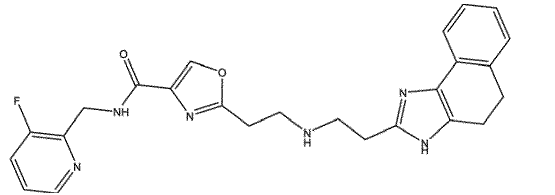 |
| **43** | 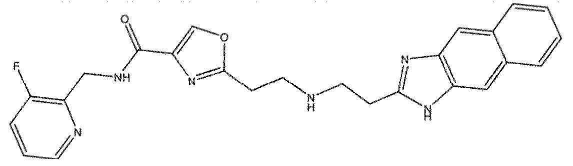 |
| **44** | 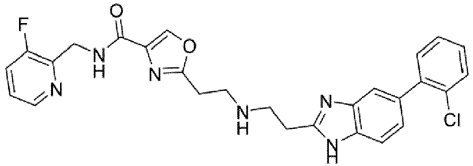 |
| **45** | 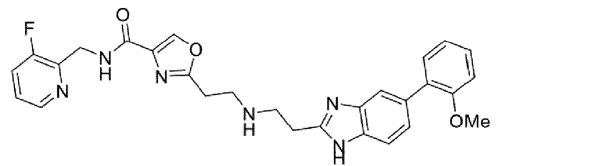 |
| **46** | 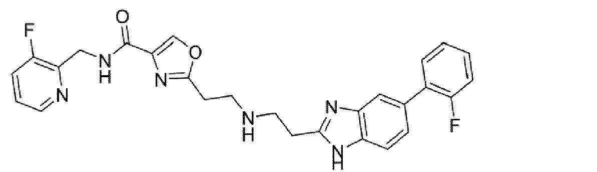 |
| **47** | 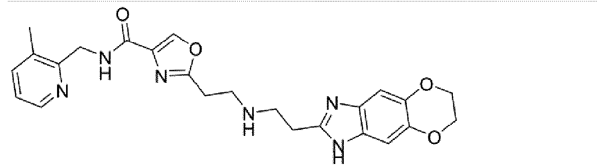 |
| **48** | 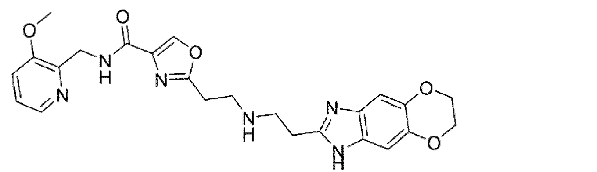 |
| **49** | 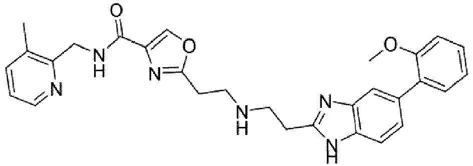 |
| **50** | 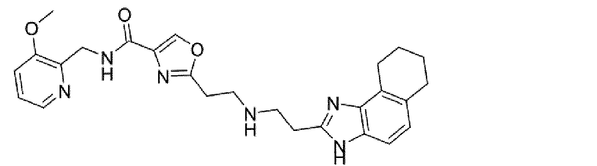 |
| **51** | 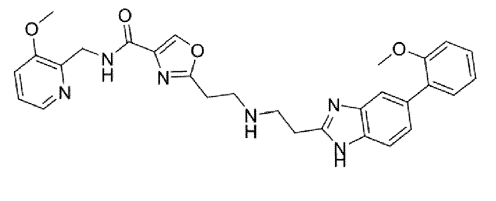 |
| **52** | 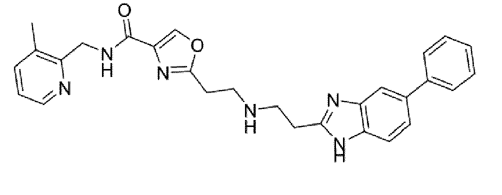 |
| **53** | 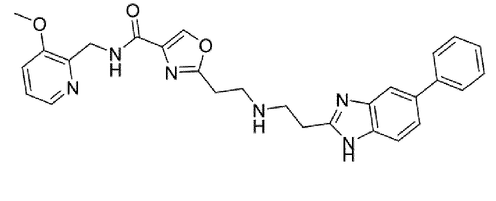 |
| **54** | 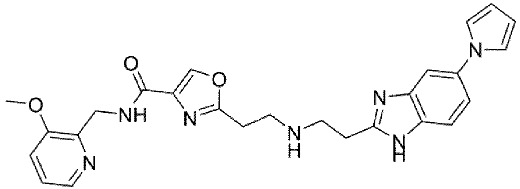 |
| **55** | 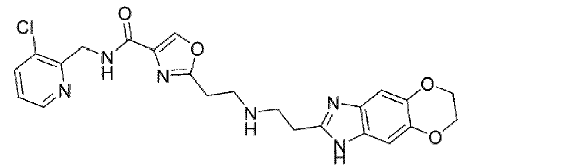 |
| **56** | 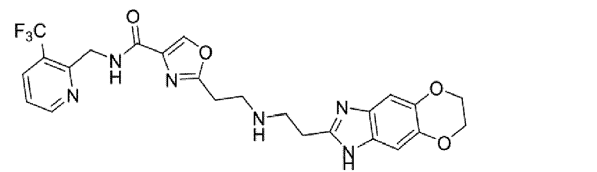 |
| **57** | 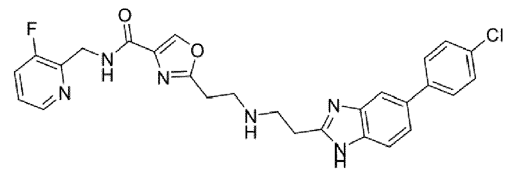 |
| **58** | 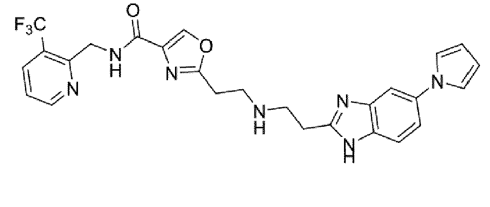 |
| **59** | 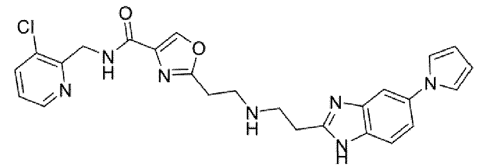 |
| **60** | 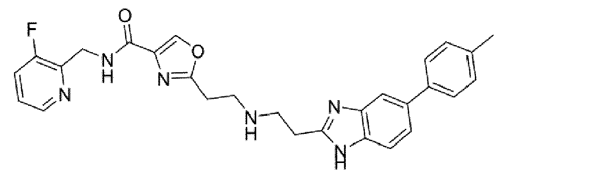 |
| **61** | 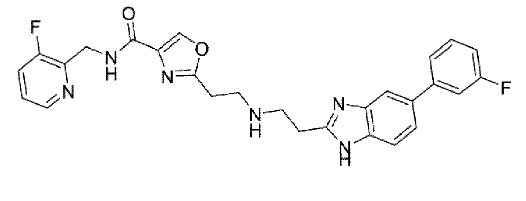 |
| **62** | 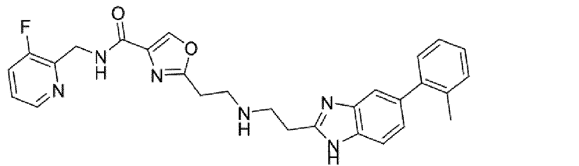 |
| **63** | 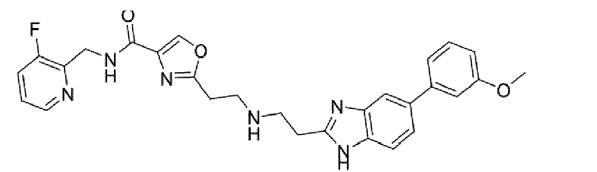 |
| **64** | 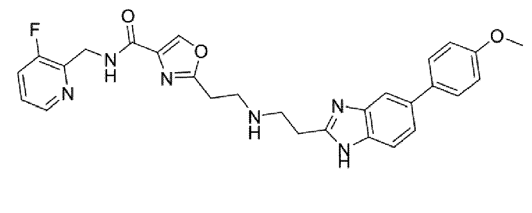 |
| **65** | 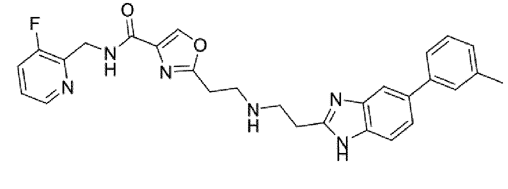 |
| **66** | 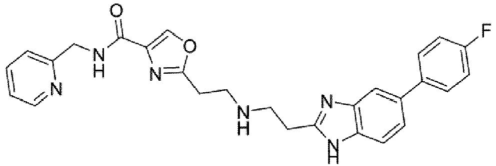 |
| **68** | 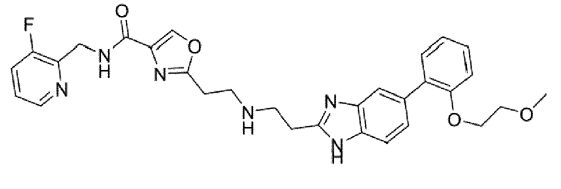 |
| **69** | 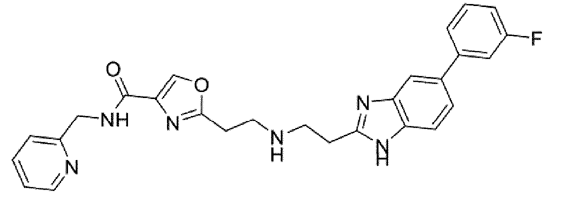 |
| **70** | 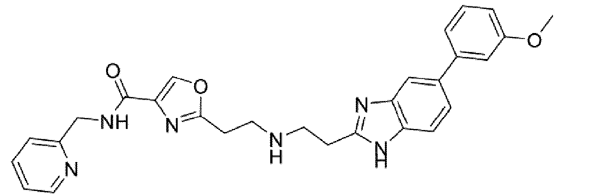 |
| **71** | 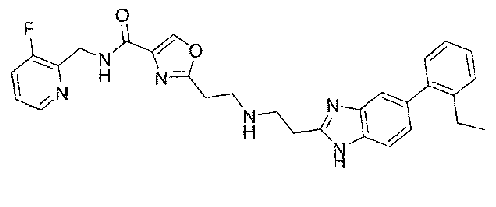 |
| **72** | 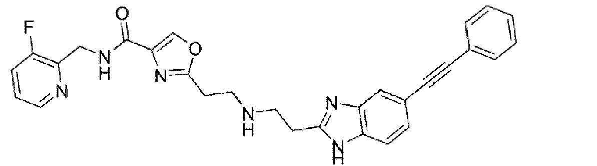 |
| **73** | 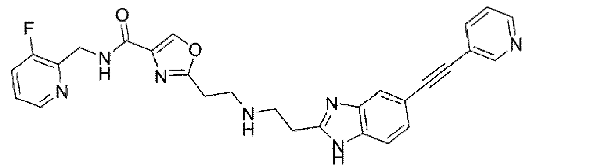 |
| **74** | 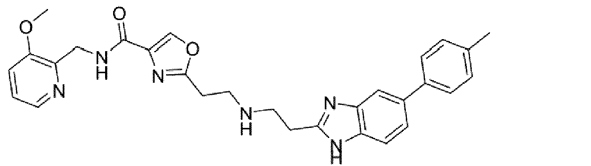 |
| **75** | 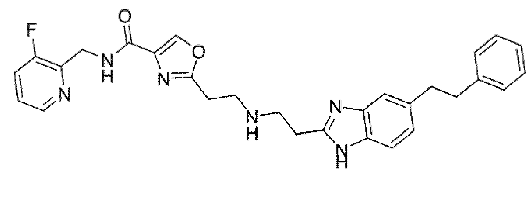 |
| **76** | 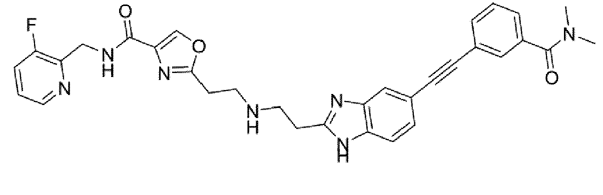 |
| **78** | 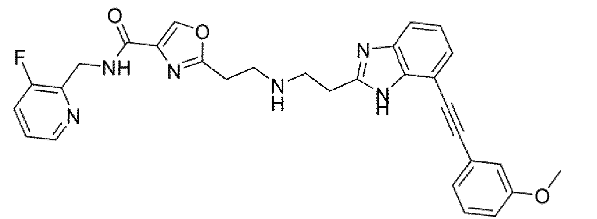 |
| **79** | 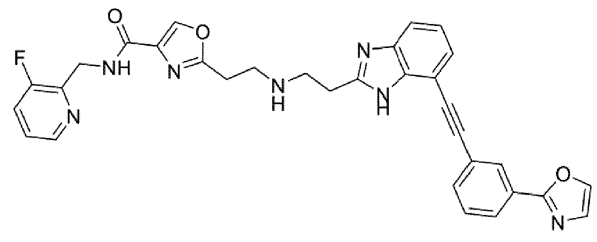 |
| **80** | 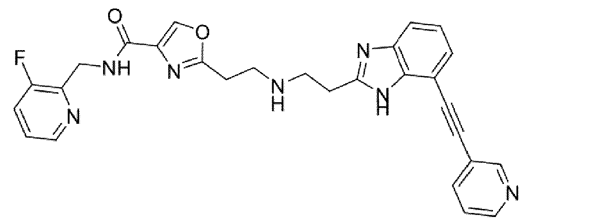 |
| **81** | 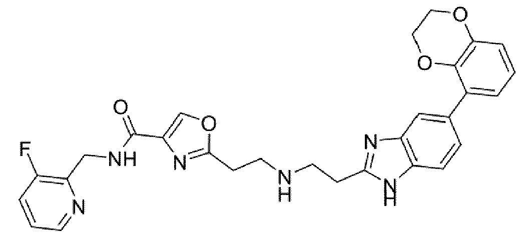 |
| **82** | 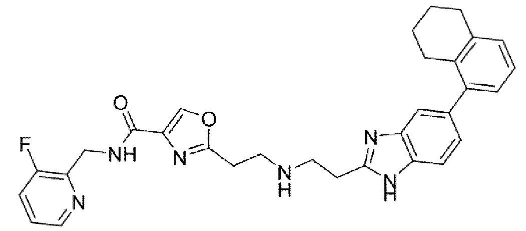 |
| **83** | 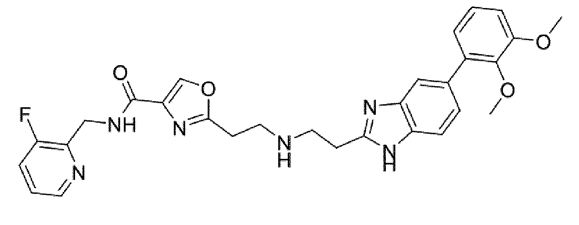 |
| **84** | 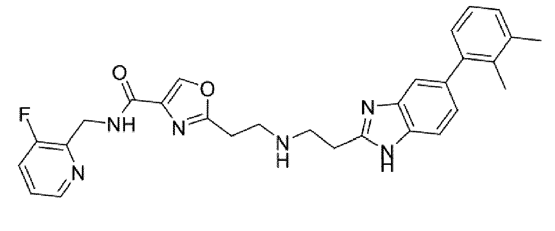 |
| **85** | 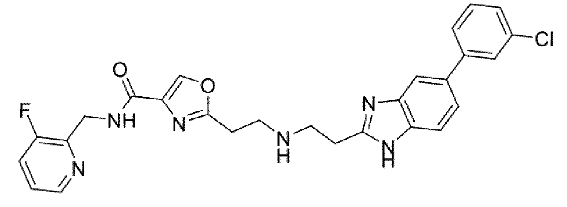 |
| **86** | 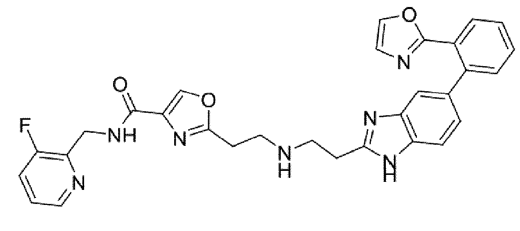 |
| **87** | 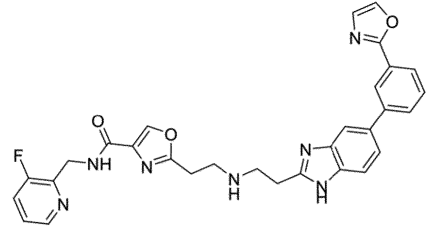 |
| **88** | 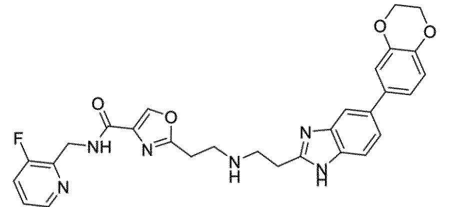 |
| **89** | 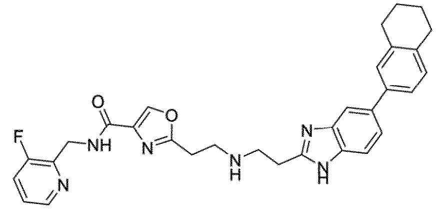 |
| **90** | 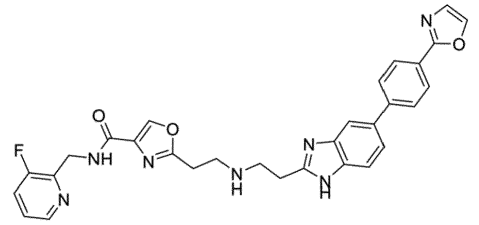 |
and pharmaceutically acceptable salts thereof

11. Compounds according to any one of the preceding claims for the use as a medicament.

12. Compounds according to any one of the claims 1 to 10 for the use as ferroportin inhibitor or for the use in the inhibition of iron transport mediated by ferroportin or for the use in the prophylaxis and/or treatment of iron metabolism disorders leading to increased iron levels or increased iron absorption, and/or iron overload.

13. Compounds as defined in any one of the claims 1 to 10 for the use in the prophylaxis and/or treatment of
- diseases related to or caused by increased iron levels, increased iron absorption or iron overload, selected from thalassemia, including alpha-thalassemia, beta-thalassemia and delta-thalassemia, hemoglobinopathy, hemoglobin E disease, hemoglobin H disease, haemochromatosis, hemolytic anemia, including in particular sickle cell anemia or congenital dyserythropoietic anemia; and/or-diseases associated with ineffective erythropoiesis, such as myelodysplastic syndromes (MDS, myelodysplasia), congenital dyserythropoietic anemia, and myeloproliferative neoplasms, such as polycythemia vera; and/or
- diseases caused by reduced levels of hepcidin; and/or
- infections caused by pathogenic microorganisms, such as the bacterium Vibrio vulnificus, in an adjunctive therapy by limiting the amount of iron available to said pathogenic microorganisms; and/or
- neurodegenerative diseases such as Alzheimer's disease and Parkinson's disease by limiting the deposition or increase of iron in tissue or cells; and/or
- formation of radicals, reactive oxygen species (ROS) and oxidative stress; and/or
- cardiac, liver and endocrine damage caused by iron overload; and/or
- inflammation triggered by excess iron.

14. A medicament containing one or more of the compounds as defined in any one of the claims 1 to 10, which may further contain
- one or more pharmaceutical carriers and/or auxiliaries and/or
- solvents, and/or
- at least one additional pharmaceutically active compound, which is preferably selected from active compounds for the prophylaxis and treatment of iron overload, thalassemia, or haemochromatosis, active compounds for the prophylaxis and treatment of neurodegenerative diseases, such as Alzheimer's disease or Parkinson's disease, and the associated symptoms, and iron-chelating compounds;
and wherein said medicament is preferably in the form of a formulation for oral or parenteral administration.

15. Compounds as defined in any one of the claims 1 to 10 for the use in a combination therapy, comprising co-administration of the compounds as defined in any of the preceding claims with at least one additional pharmaceutically active compound, wherein
said co-administration of the combination therapy may be carried out in a fixed dose combination therapy by co-administration of the compounds as defined in any of the preceding claims with at least one additional pharmaceutically active compound in a fixed-dose formulation; or
said co-administration of the combination therapy may be carried out in a free dose combination therapy by co-administration of the compounds as defined in any of the preceding claims and the at least one additional pharmaceutically active compound in free doses of the respective compounds, either by simultaneous administration of the individual compounds or by sequential use of the individual compounds distributed over a time period; and
wherein the one or more other pharmaceutically active compounds are preferably active compounds for reducing iron overload, which are selected from Tmprss6-ASO, iron chelators, curcumin, SSP-004184, Deferitrin, deferasirox, deferoxamine and/or deferiprone; and/or pharmaceutically active compounds which are selected from antioxidants, such as n-acetyl cysteine; anti-diabetics, such as GLP-1 receptor agonists; antibiotics, such as vancomycin (Van) or tobramycin; drugs for the treatment of malaria; anticancer agents; antifungal drugs; drugs for the treatment of neurodegenerative diseases such as Alzheimer's disease and Parkinson's disease, comprising dopamine agonists such as Levodopa; anti-viral drugs, such as interferon-a or ribavirin; immunosuppressents, such as cyclosporine A or cyclosporine A derivatives; iron supplements; vitamin supplements; red cell production stimulators; anti-inflammatory biologies; anti-thrombolytics; statins; vasopressors; and inotropic compounds.

## Patentansprüche

1. Verbindungen der Formel (I) 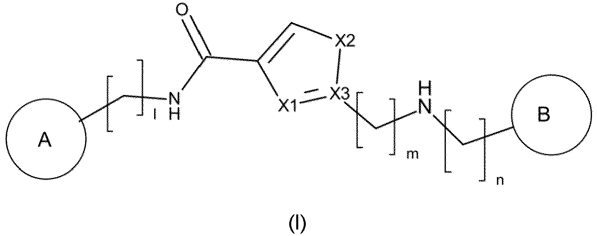 wobei
I ist eine ganze Zahl von 1 oder 2;
m und n sind unabhängig voneinander eine ganze Zahl von 1, 2 oder 3;
X¹ ist N, S oder O;
X² ist N, S, O oder CR⁴;
X³ ist C oder N;
mit der Maßgabe, dass eines von X¹ und X² N ist
und wenn X³ N ist, dann ist X² CR⁴;
und wobei
R⁴ steht für
- H,
- Halogen,
- lineares oder verzweigtes C₁-C₃-Alkyl,
- lineares oder verzweigtes C₁-C₃-Halogenalkyl, oder
A steht für eine der folgenden Gruppen (a-1), (a-2), (a-3), (a-4) und (a-5) 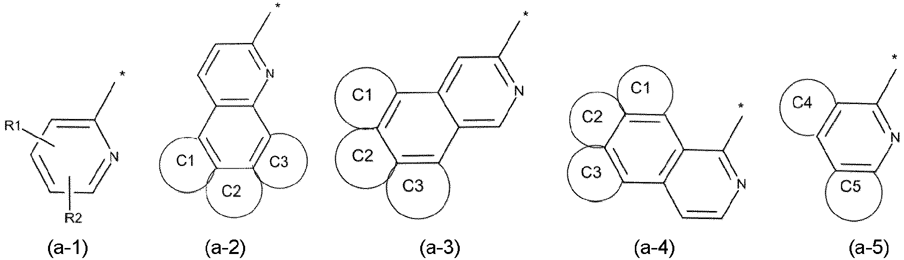
wobei * die Bindungsposition angibt;
R¹ und R² stehen unabhängig voneinander für
- H,
- Halogen,
- lineares oder verzweigtes C₁-C₃-Alkyl,
- lineares oder verzweigtes C₁-C₃-Halogenalkyl,
- lineares oder verzweigtes C₁-C₃-Alkoxy;
in den Formeln (a-2), (a-3) und (a-4) is eines von C1, C2 und C3 vorhanden und in der Formel (a-5) sind sowohl C4 als auch C5 vorhanden, und
C1, C2, C3, C4 und C5 stellen unabhängig voneinander dar
- einen kondensierten 6-gliedrigen Arylring,
- einen kondensierten 5- oder 6-gliedrigen Heteroarylring,
- einen kondensierten 5- oder 6-gliedrigen Cycloalkylring,
- einen kondensierten 5- oder 6-gliedrigen Heterocyclylring; und
wobei die Gruppen (a-2), (a-3), (a-4) und (a-5) 0, 1, 2 oder 3 Substituenten tragen, die unabhängig voneinander ausgewählt sind aus
- Halogen,
- lineares oder verzweigtes C₁-C₃-Alkyl,
- lineares oder verzweigtes C₁-C₃-Halogenalkyl,
- lineares oder verzweigtes C₁-C₃-Alkoxy;
B steht für eine der folgenden Gruppen (b-1), (b-2) und (b-3) 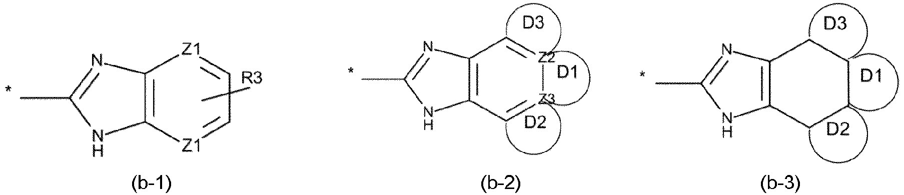
wobei * die Bindungsposition angibt;
R³ steht für
- H,
- unsubstituiertes oder substituiertes 6-gliedriges Aryl,
- unsubstituiertes oder substituiertes 5- oder 6-gliedriges Heteroaryl,
- unsubstituiertes oder substituiertes bicyclisches Heteroaryl,
- 5- oder 6-gliedriges Cycloalkyl,
- 5- oder 6-gliedriges Heterocyclyl,
- 5- oder 6-gliedriges Heterocyclylalkyl,
- 6-gliedriges Arylalkyl,
- unsubstituiertes oder substituiertes 6-gliedriges Arylalkinyl
- unsubstituiertes oder substituiertes 5- oder 6-gliedriges Heteroarylalkinyl, oder
- eine Phenylgruppe, die mit einer 5- oder 6-gliedrigen Cycloalkyl- oder Heterocyclylgruppe einen kondensierten bicyclischen Ring bildet;
Z¹ steht für N oder C,
mit der Maßgabe, dass nicht mehr als ein Z¹ für N steht;
in den Formeln (b-2) und (b-3) ist eines von D1, D2 und D3 vorhanden und steht für
- einen kondensierten 6-gliedrigen Arylring,
- einen kondensierten 5- oder 6-gliedrigen Heteroarylring,
- einen kondensierten 5- oder 6-gliedrigen Cycloalkylring,
- einen kondensierten 5- oder 6-gliedrigen Heterocyclylring; und
wobei die Gruppen (b-2) und (b-3) 0, 1, 2 oder 3 Substituenten tragen, die unabhängig voneinander ausgewählt sind aus
- Halogen,
- lineares oder verzweigtes C₁-C₃-Alkyl,
- lineares oder verzweigtes C₁-C₃-Halogenalkyl,
- lineares oder verzweigtes C₁-C₃-Alkoxy;
Z² und Z³ stehen für N oder C,
mit der Maßgabe, dass Z² für N stehen kann, wenn D2 vorhanden ist, und Z³ für N stehen kann, wenn D3 vorhanden ist;
und wobei die Verbindungen (I) **dadurch gekennzeichnet sind, dass** mindestens eine der Gruppen A und B mindestens 3 Ringe enthält;
und deren pharmazeutisch akzeptable Salze.

2. Verbindungen nach Anspruch 1, wobei
R³ steht für
- H,
- unsubstituiertes oder substituiertes 6-gliedriges Aryl,
- unsubstituiertes oder substituiertes 5- oder 6-gliedriges Heteroaryl,
- unsubstituiertes oder substituiertes bicyclisches Heteroaryl,
- 5- oder 6-gliedriges Cycloalkyl,
- 5- oder 6-gliedriges Heterocyclyl,
- 5- oder 6-gliedriges Heterocyclylalkyl,
- 6-gliedriges Arylalkinyl.

3. Verbindungen nach Anspruch 1 oder 2,
wobei
I ist eine ganze Zahl von 1 oder 2;
m und n sind unabhängig voneinander eine ganze Zahl von 1, 2 oder 3;
X¹ ist N, S oder O;
X² ist N, S, O oder CR⁴;
X³ ist C oder N;
mit der Maßgabe, dass eines von X¹ und X² N ist
und wenn X³ N ist, dann ist X² CR⁴;
und wobei
R⁴ für H steht;
A steht für eine der folgenden Gruppen (a-1), (a-2), (a-3), (a-4) und (a-5) 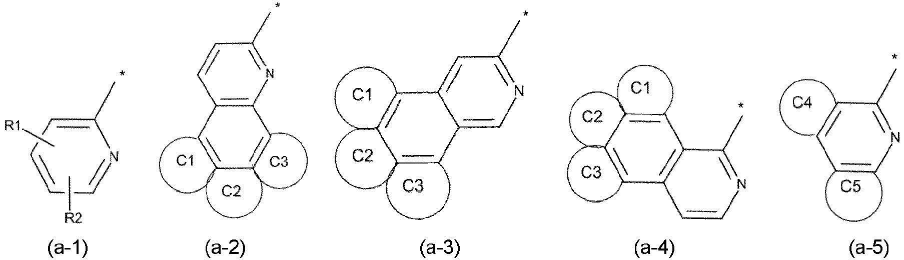 wobei * die Bindungsposition angibt;
R¹ und R² stehen unabhängig voneinander für
- Wasserstoff,
- Halogen,
- lineares oder verzweigtes C₁-C₃-Alkyl,
- lineares oder verzweigtes C₁-C₃-Halogenalkyl,
- lineares oder verzweigtes C₁-C₃-Alkoxy;
in den Formeln (a-2), (a-3) und (a-4) ist eines von C1, C2 und C3 vorhanden und in der Formel (a-5) sind sowohl C4 als auch C5 vorhanden, und
C1, C2, C3, C4 und C5 stellen einen kondensierten Phenylring dar; und
wobei die Gruppen (a-2), (a-3), (a-4) und (a-5) 0 oder 1 Substituent tragen, der ausgewählt ist aus
- Halogen,
- lineares oder verzweigtes C₁-C₃-Alkyl,
- lineares oder verzweigtes C₁-C₃-Halogenalkyl,
- lineares oder verzweigtes C₁-C₃-Alkoxy;
B steht für eine der folgenden Gruppen (b-1), (b-2) und (b-3) 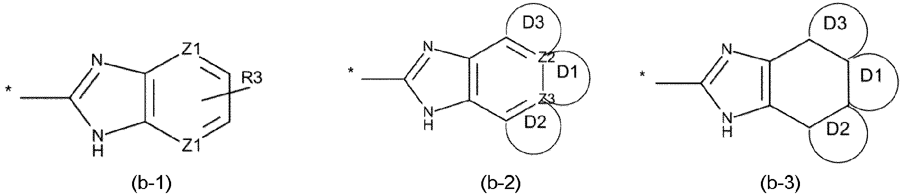
wobei * die Bindungsposition angibt;
R³ steht für
- H,
- unsubstituiertes oder substituiertes Phenyl,
- unsubstituiertes oder substituiertes 5- oder 6-gliedriges Heteroaryl,
- unsubstituiertes oder substituiertes bicyclisches Heteroaryl,
- 6-gliedriger Heterocyclyl,
- 6-gliedriges Heterocyclylalkyl,
- Phenylalkyl,
- unsubstituiertes oder substituiertes Phenylethinyl oder unsubstituiertes oder substituiertes 6-gliedriges Heteroarylethinyl, oder
- eine Phenylgruppe, die mit einer 5- oder 6-gliedrigen Cycloalkyl- oder Heterocyclylgruppe einen kondensierten bicyclischen Ring bildet;
Z¹ steht für N oder C,
mit der Maßgabe, dass nicht mehr als ein Z¹ für N steht;
in den Formeln (b-2) und (b-3) ist eines von D1, D2 und D3 vorhanden und steht für
- einen kondensierten Phenylring,
- einen kondensierten 6-gliedrigen Heteroarylring,
- einen kondensierten 6-gliedrigen Cycloalkylring,
- einen kondensierten 5- oder 6-gliedrigen Heterocyclylring; und
wobei die Gruppen (b-2) und (b-3) 0 oder 1 Substituent tragen, der ausgewählt ist aus
- Halogen,
- lineares oder verzweigtes C₁-C₃-Alkyl,
- lineares oder verzweigtes C₁-C₃-Halogenalkyl,
- lineares oder verzweigtes C₁-C₃-Alkoxy;
Z² und Z³ stehen für N oder C,
mit der Maßgabe, dass Z² N darstellen kann, wenn D2 vorhanden ist, und Z³ N darstellen kann, wenn D3 vorhanden ist;
und wobei die Verbindungen (I) **dadurch gekennzeichnet sind, dass** mindestens eine der Gruppen A und B mindestens 3 Ringe enthält;
und deren pharmazeutisch akzeptable Salze.

4. Verbindungen nach einem der Ansprüche 1 bis 3, wobei
R¹ und R² unabhängig voneinander darstellen
- Halogen,
- lineares oder verzweigtes C₁-C₃-Alkyl,
- lineares oder verzweigtes C₁-C₃-Halogenalkyl,
- lineares oder verzweigtes C₁-C₃-Alkoxy und
R³ steht für
- H,
- unsubstituiertes oder substituiertes Phenyl,
- unsubstituiertes oder substituiertes 5- oder 6-gliedriges Heteroaryl,
- unsubstituiertes oder substituiertes bicyclisches Heteroaryl,
- 6-gliedriger Heterocyclyl,
- 6-gliedriges Heterocyclylalkyl, und
- Phenylethinyl.

5. Verbindungen nach einem der Ansprüche 1 bis 4, **dadurch gekennzeichnet, dass** eine der Gruppen A und B 3 Ringe enthält; und pharmazeutisch akzeptable Salze davon.

6. Verbindungen nach einem der Ansprüche 1 bis 5, wobei
- die Gruppe A eine Gruppe (a-1) ist; und/oder
- die Gruppe A eine Gruppe (a-2), (a-3) oder (a-5) ist; und/oder
- die Gruppe A eine Gruppe (a-5) ist; und/oder
- die Gruppe B eine Gruppe (b-1) oder (b-2) ist;
und deren pharmazeutisch akzeptable Salze.

7. Verbindungen nach einem der Ansprüche 1 bis 6, wobei mögliche Substituenten aus den folgenden Gruppen ausgewählt sind
- Halogensubstituenten stehen für F, Cl und Br,
- lineare oder verzweigte C₁-C₃-Alkylsubstituenten stellen Methyl und Ethyl dar,
- lineare oder verzweigte C₁-C₃-Halogenalkylsubstituenten stellen Trifluormethyl (CF₃) dar,
- lineare oder verzweigte C₁-C₃-Alkoxysubstituenten stellen Methoxy dar;
- 5- oder 6-gliedrige Heteroaryl-Substituenten stehen für Oxazolyl,
- ein Alkoxyalkylether-Substituent stellt eine Methoxyethylether-Gruppe dar,
- ein Dialkylaminocarbonyl-Substituent steht für Dimethylaminocarbonyl,
bevorzugt sind mögliche Substituenten aus den folgenden Gruppen ausgewählt
- Halogensubstituenten stehen für F, Cl und Br,
- lineare oder verzweigte C₁-C₃-Alkylsubstituenten stellen Methyl dar,
- lineare oder verzweigte C₁-C₃-Halogenalkylsubstituenten stellen Trifluormethyl (CF₃) dar,
- lineare oder verzweigte C₁-C₃-Alkoxysubstituenten stellen Methoxy dar;
und deren pharmazeutisch akzeptable Salze.

8. Verbindungen nach einem der Ansprüche 1 bis 7, wobei X¹, X² und X³ ausgewählt werden, um eine der folgenden Gruppen zu bilden: 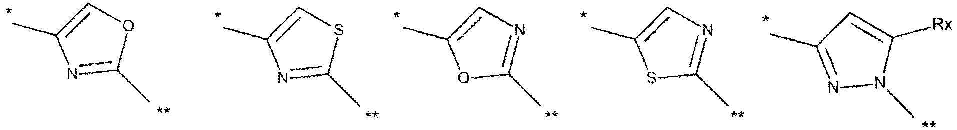
wobei * die Bindungsstelle an die Aminocarbonylgruppe und ** die Bindungsstelle an die -[(CH₂)]ₘ-Amino-[(CH₂)]₍ₙ)-Gruppe angibt;
und wobei
R⁴ steht für
- H,
- Halogen,
- lineares oder verzweigtes C₁-C₃-Alkyl,
- lineares oder verzweigtes C₁-C₃-Halogenalkyl; oder
worin vorzugsweise X¹, X² und X³ so ausgewählt sind, dass sie eine der folgenden Gruppen bilden: 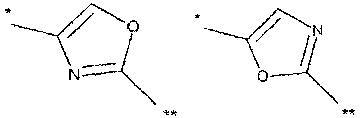 oder
worin vorzugsweise X¹, X² und X³ ausgewählt sind, um eine der folgenden Gruppen zu bilden: 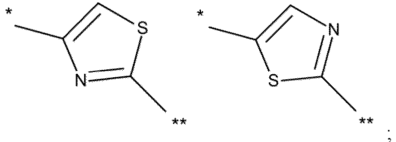
und deren pharmazeutisch akzeptable Salze.

9. Verbindungen nach einem der vorhergehenden Ansprüche, wobei für die Gruppe A die folgenden aus den Gruppen (a-1), (a-2), (a-3) und (a-5) ausgewählt werden: 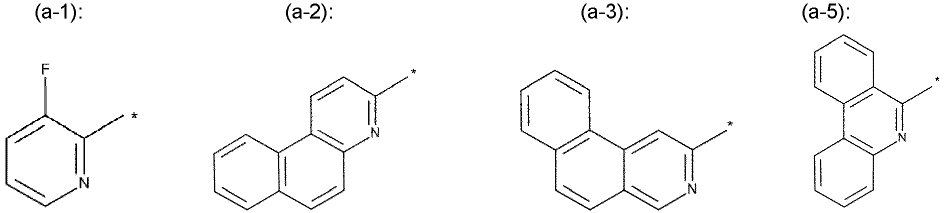 und deren pharmazeutisch akzeptable Salze.

10. Verbindungen nach einem der vorangehenden Ansprüche, die ausgewählt sind aus
| **Nr.** | **Chemische Struktur** |
|---|---|
| **1** | 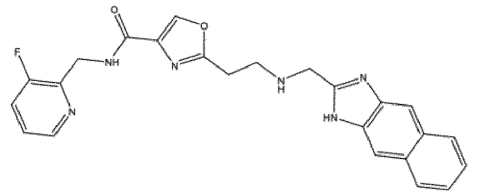 |
| **2** | 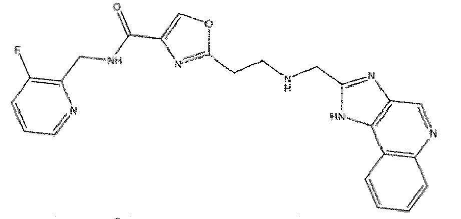 |
| **3** | 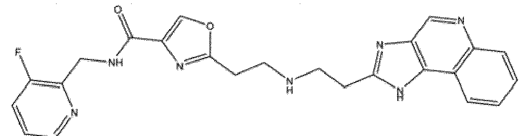 |
| **4** | 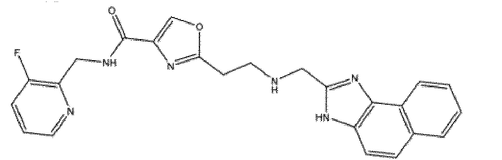 |
| **5** | 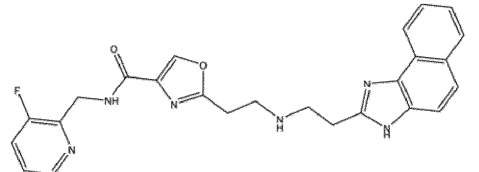 |
| **6** | 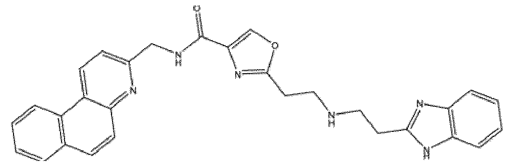 |
| **7** | 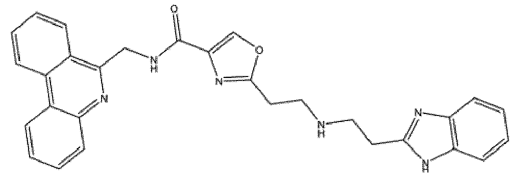 |
| **8** | 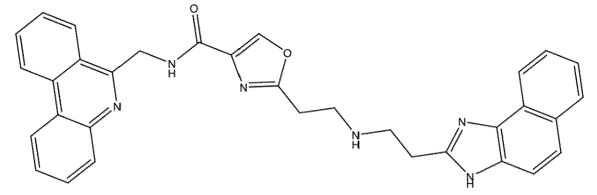 |
| **9** | 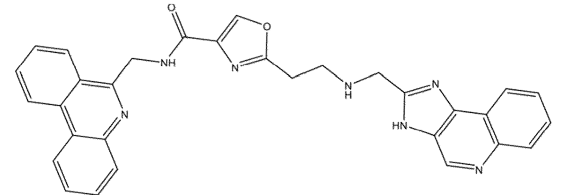 |
| **10** | 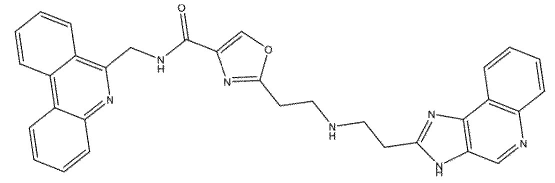 |
| **11** | 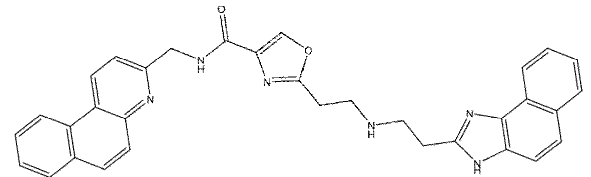 |
| **12** | 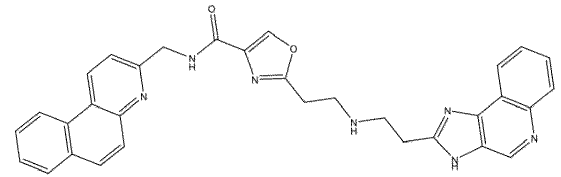 |
| **13** | 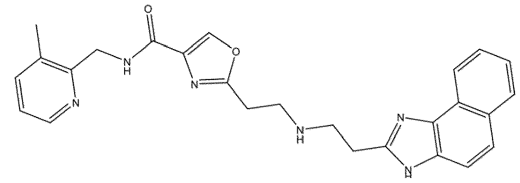 |
| **14** | 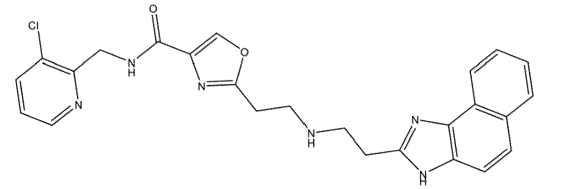 |
| **15** | 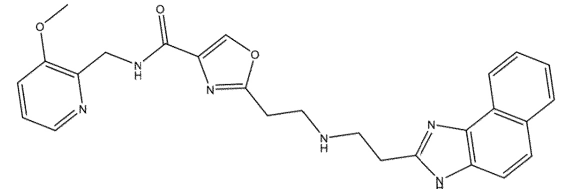 |
| **16** | 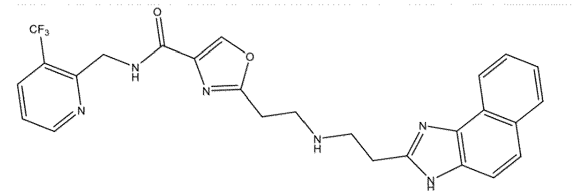 CF3 O N H O N N N H N N H |
| **17** | 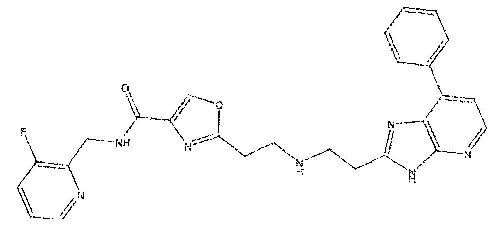 |
| **18** | 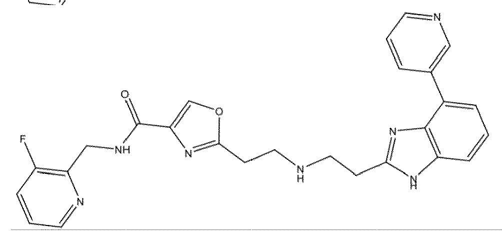 |
| **19** | 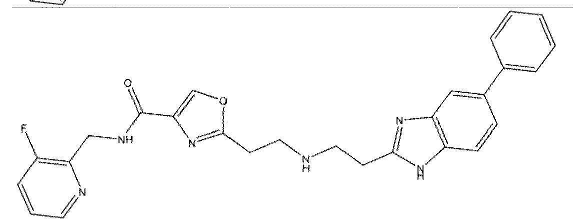 |
| **20** | 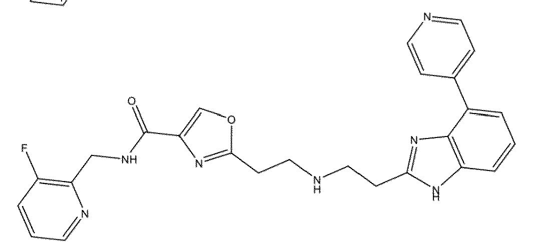 F O NH N O N H N N N H |
| **21** | 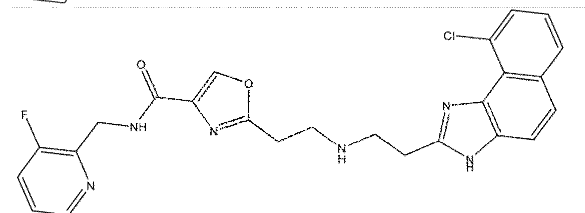 |
| **22** | 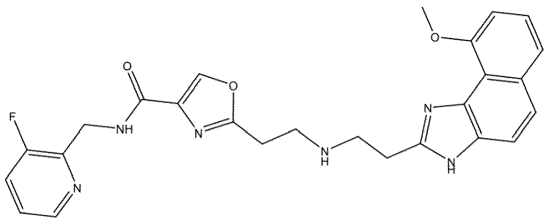 |
| **23** | 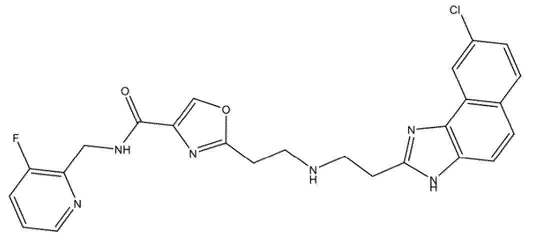 |
| **24** | 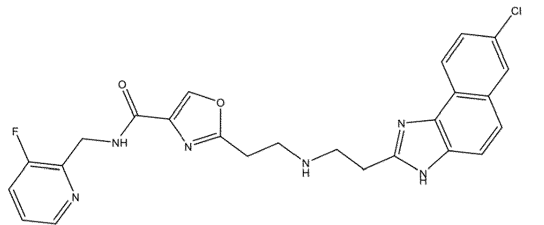 |
| **25** | 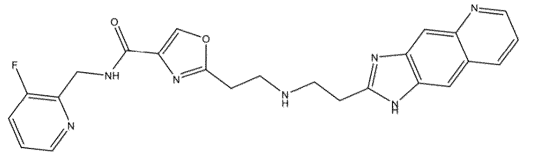 |
| **26** | 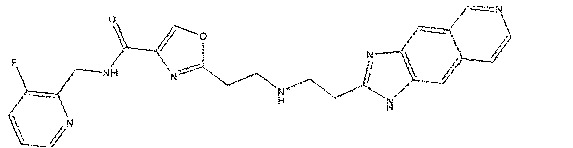 |
| **27** | 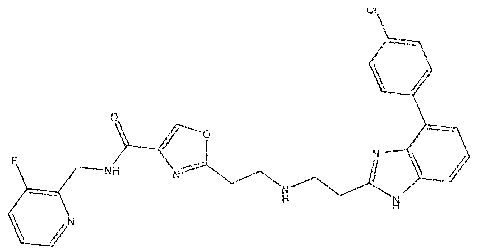 |
| **28** | 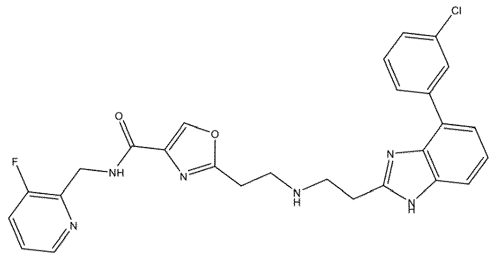 |
| **29** | 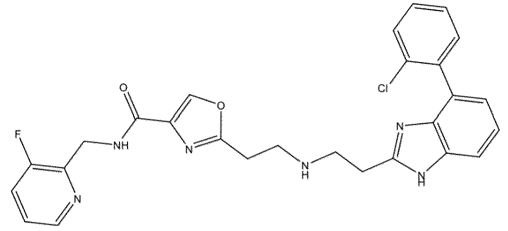 |
| **30** | 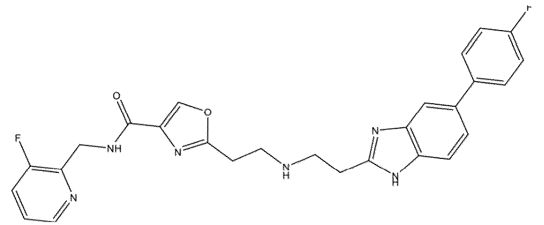 |
| **31** | 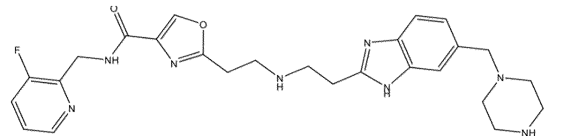 |
| **32** | 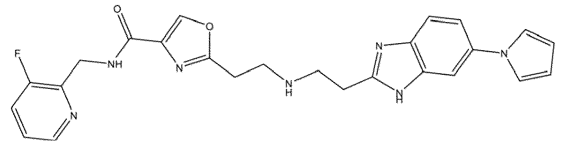 |
| **33** | 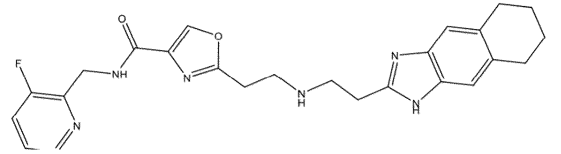 |
| **34** | 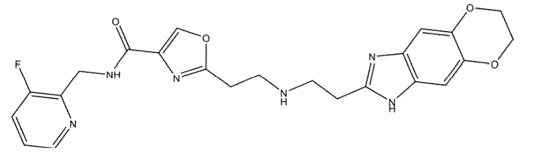 |
| **35** | 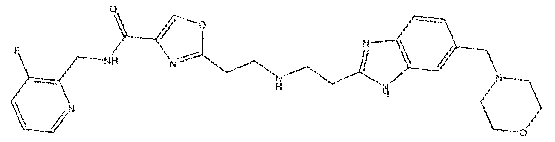 |
| **36** | 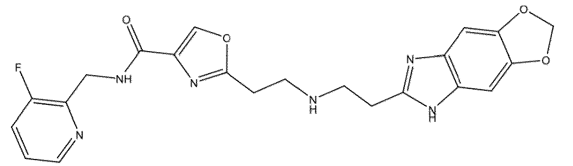 |
| **37** | 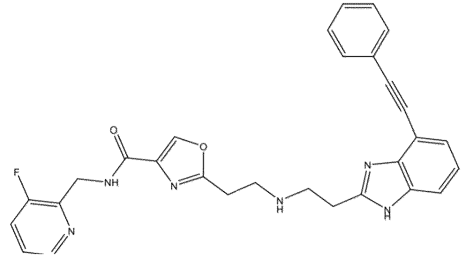 |
| **38** | 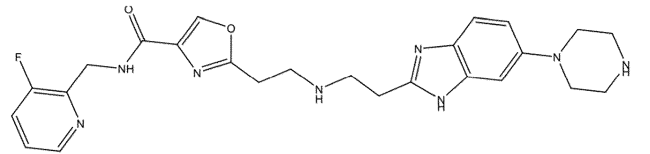 |
| **39** | 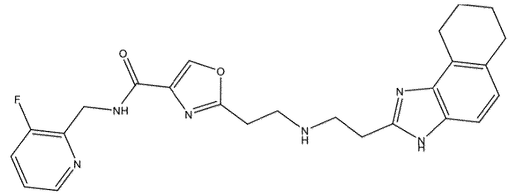 |
| **40** | 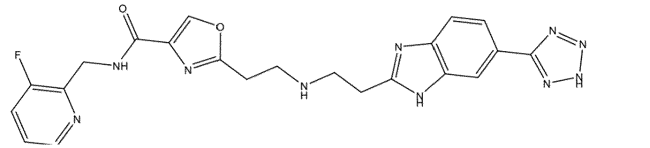 |
| **41** | 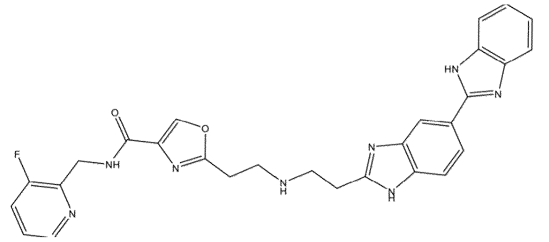 |
| **42** | 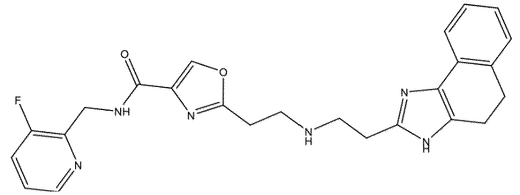 |
| **43** | 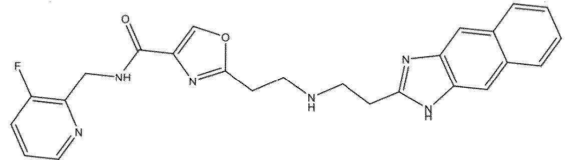 |
| **44** | 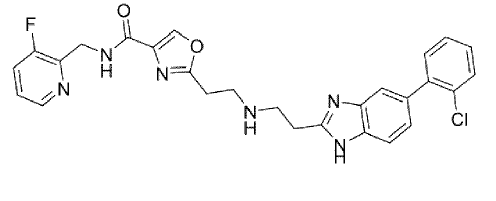 |
| **45** | 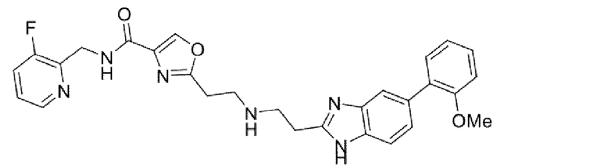 |
| **46** | 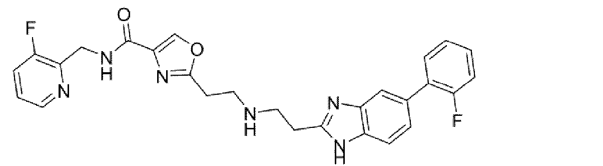 |
| **47** | 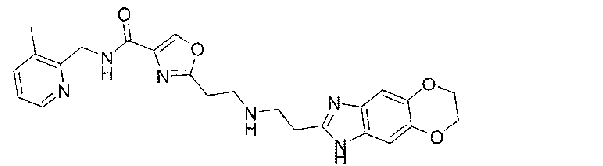 |
| **48** | 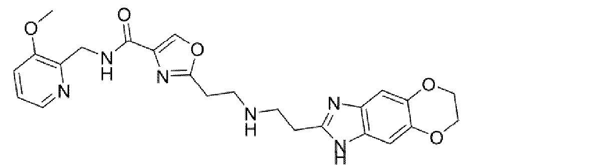 |
| **49** | 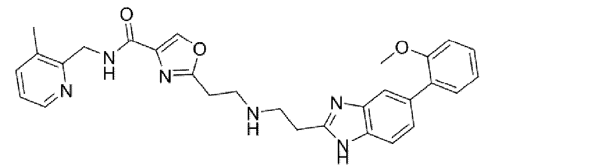 |
| **50** | 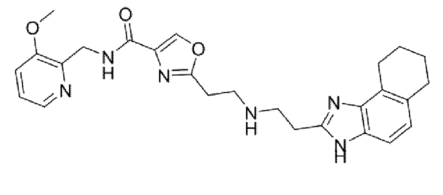 |
| **51** | 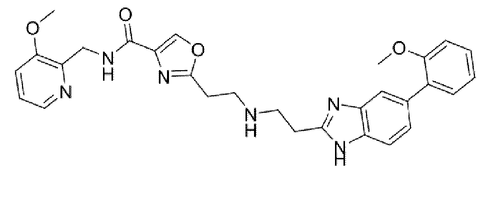 |
| **52** | 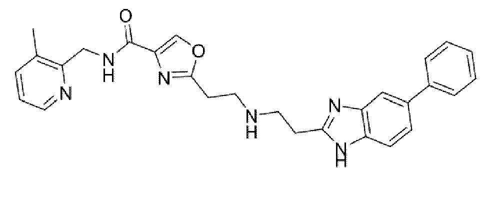 |
| **53** | 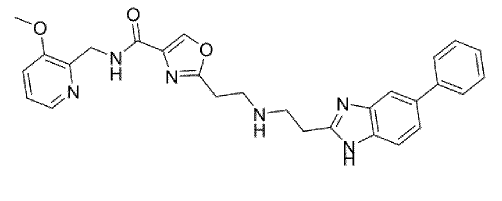 |
| **54** | 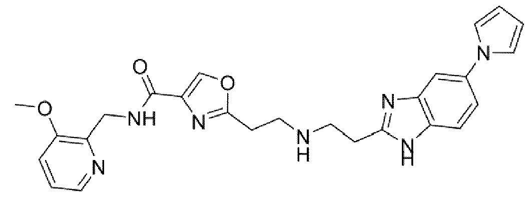 |
| **55** | 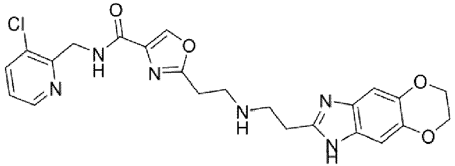 |
| **56** | 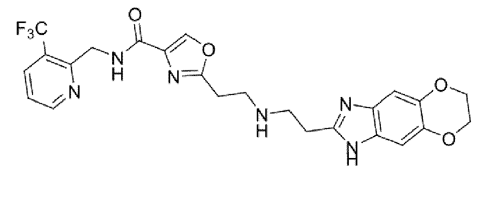 |
| **57** | 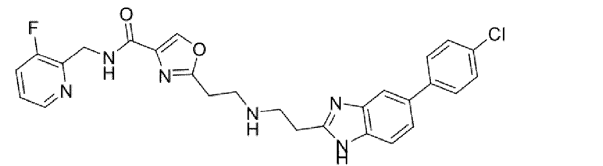 |
| **58** | 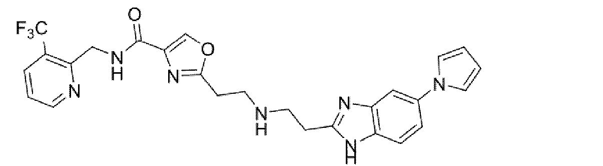 |
| **59** | 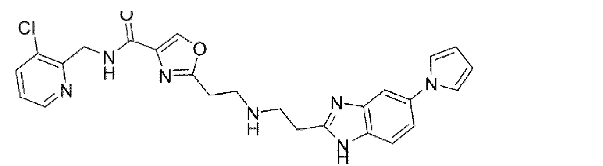 |
| **60** | 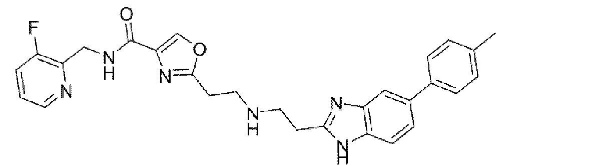 |
| **61** | 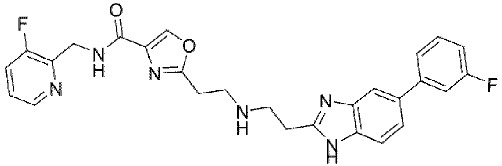 |
| **62** | 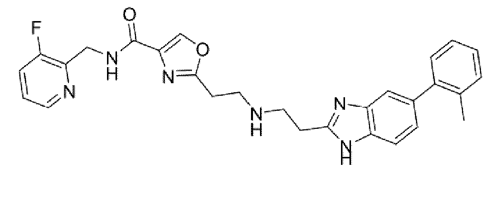 |
| **63** | 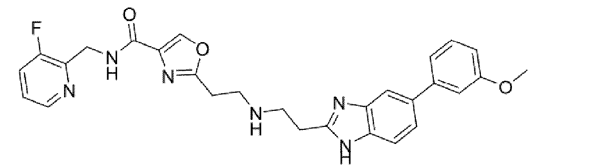 |
| **64** | 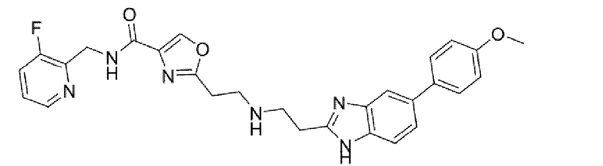 |
| **65** | 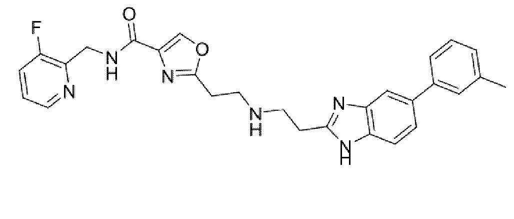 |
| **66** | 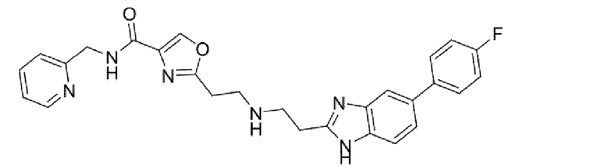 |
| **68** | 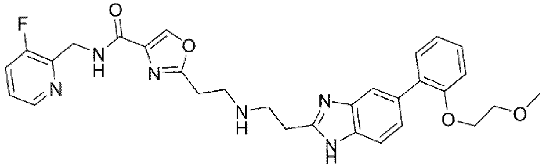 |
| **69** | 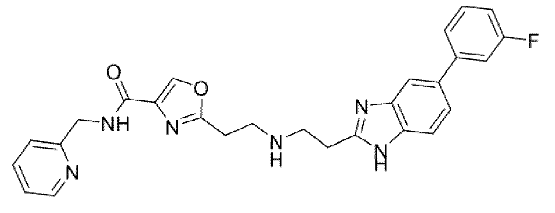 |
| **70** | 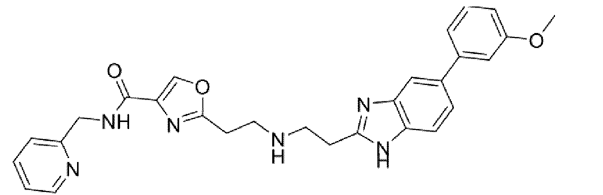 |
| **71** | 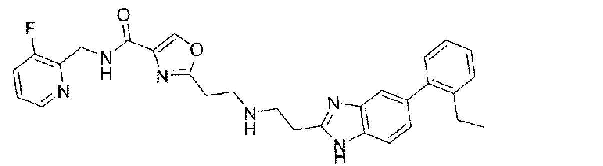 |
| **72** | 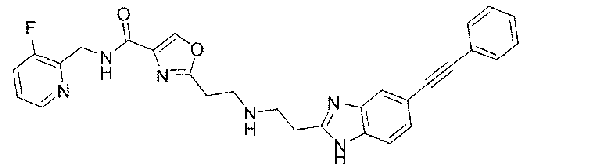 |
| **73** | 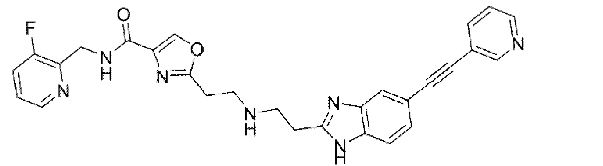 |
| **74** | 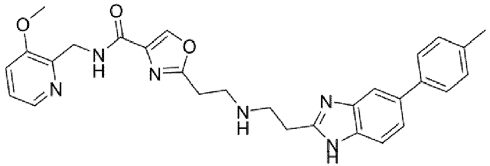 |
| **75** | 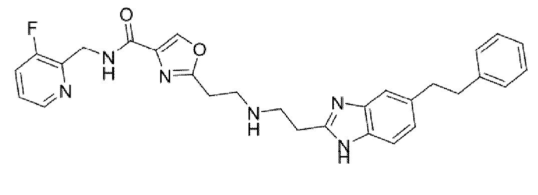 |
| **76** | 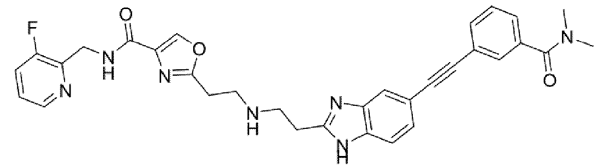 |
| **78** | 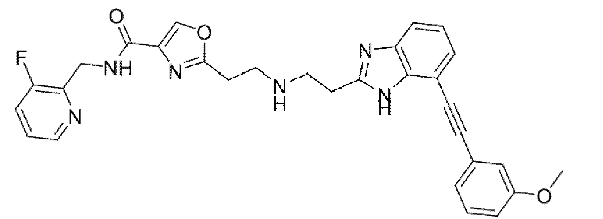 |
| **79** | 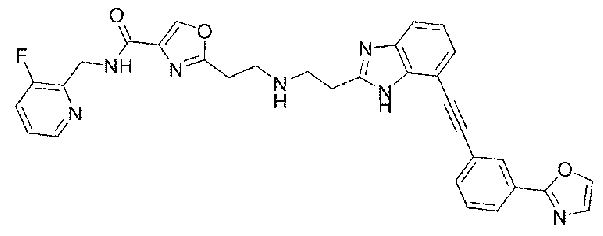 |
| **80** | 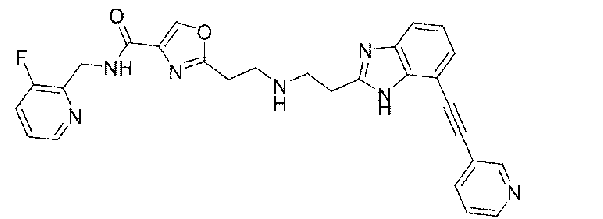 |
| **81** | 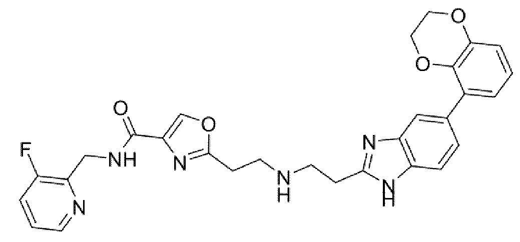 |
| **82** | 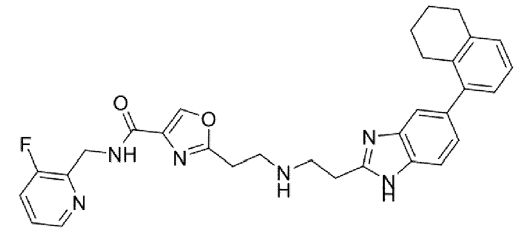 |
| **83** | 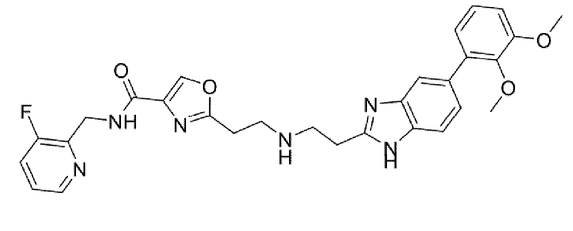 |
| **84** | 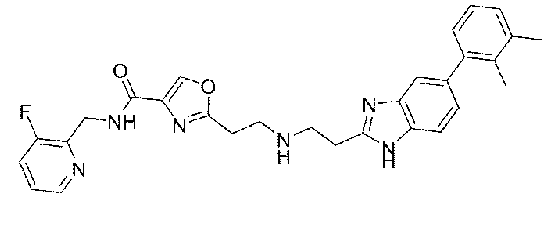 |
| **85** | 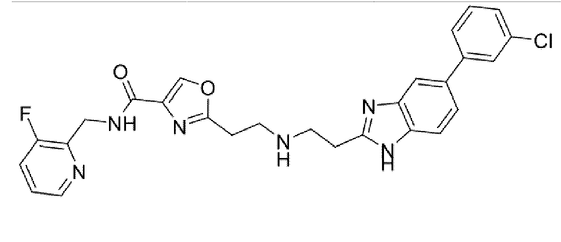 |
| **86** | 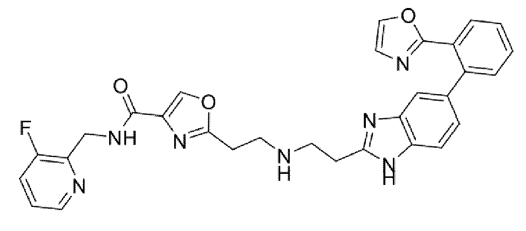 |
| **87** | 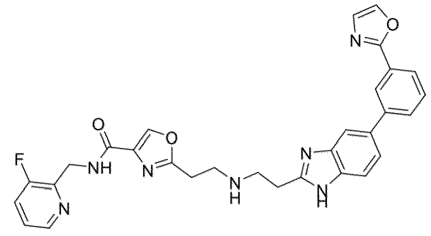 |
| **88** | 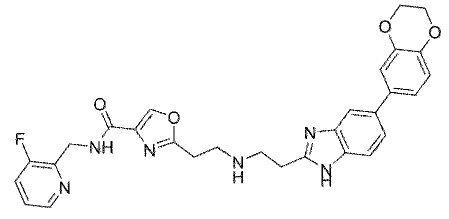 |
| **89** | 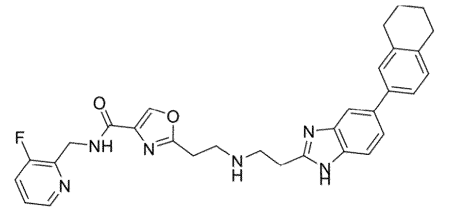 |
| **90** | 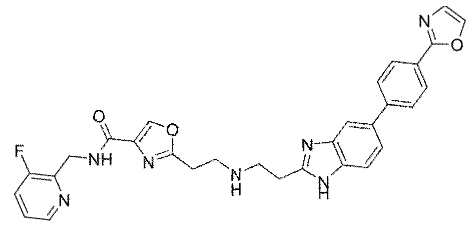 |
und pharmazeutisch akzeptable Salze davon

11. Verbindungen nach einem der vorangehenden Ansprüche zur Verwendung als Arzneimittel.

12. Verbindungen nach einem der Ansprüche 1 bis 10 zur Verwendung als Ferroportin-Inhibitor oder zur Verwendung in der Hemmung des durch Ferroportin vermittelten Eisentransports oder zur Verwendung zur Prophylaxe und/oder Behandlung von Eisenstoffwechselstörungen, die zu erhöhten Eisenspiegeln oder erhöhter Eisenabsorption und/oder Eisenüberladung führen.

13. Verbindungen, wie in einem der Ansprüche 1 bis 10 definiert, zur Verwendung zur Prophylaxe und/oder Behandlung von
- Krankheiten, die mit erhöhten Eisenspiegeln, erhöhter Eisenabsorption oder Eisenüberladung zusammenhängen oder dadurch verursacht werden, ausgewählt aus Thalassämie, einschließlich Alpha-Thalassämie, Beta-Thalassämie und Delta-Thalassämie, Hämoglobinopathie, Hämoglobin-E-Krankheit, Hämoglobin-H-Krankheit, Hämochromatose, hämolytischer Anämie, insbesondere Sichelzellenanämie oder kongenitaler dyserythropoetischer Anämie; und/oder- Krankheiten, die mit einer ineffektiven Erythropoese einhergehen, wie myelodysplastische Syndrome (MDS, Myelodysplasie), kongenitale dyserythropoetische Anämie und myeloproliferative Neoplasmen, wie Polycythemia vera; und/oder
- Krankheiten, die durch einen verminderten Hepcidinspiegel verursacht werden; und/oder
- Infektionen, die durch pathogene Mikroorganismen, wie das Bakterium Vibrio vulnificus, verursacht werden, in einer Zusatztherapie durch Begrenzung der für die pathogenen Mikroorganismen verfügbaren Eisenmenge; und/oder
- neurodegenerativen Erkrankungen wie die Alzheimer- und die Parkinson-Krankheit, indem die Ablagerung oder Zunahme von Eisen im Gewebe oder in den Zellen begrenzt wird; und/oder
- Bildung von Radikalen, reaktiven Sauerstoffspezies (ROS) und oxidativem Stress; und/oder
- Herz-, Leber- und Hormonschäden aufgrund von Eisenüberladung; und/oder
- Entzündungen, die durch einen Eisenüberschuss ausgelöst werden.

14. Arzneimittel, enthaltend eine oder mehrere der in einem der Ansprüche 1 bis 10 definierten Verbindungen, das außerdem enthalten kann
- einen oder mehrere pharmazeutische Träger und/oder Hilfsstoffe und/oder
- Lösungsmittel, und/oder
- mindestens einen zusätzlichen pharmazeutischen Wirkstoff, der vorzugsweise ausgewählt ist aus Wirkstoffen zur Prophylaxe und Behandlung von Eisenüberladung, Thalassämie oder Hämochromatose, Wirkstoffen zur Prophylaxe und Behandlung von neurodegenerativen Erkrankungen wie der Alzheimer- oder Parkinson-Krankheit und den damit verbundenen Symptomen sowie Eisenchelat-bildenden Verbindungen;
und wobei das Medikament vorzugsweise in Form einer Formulierung zur oralen oder parenteralen Verabreichung vorliegt.

15. Verbindungen, wie in einem der Ansprüche 1 bis 10 definiert, zur Verwendung in einer Kombinationstherapie, umfassend die gemeinsame Verabreichung der Verbindungen, wie in einem der vorhergehenden Ansprüche definiert, mit mindestens einer zusätzlichen pharmazeutisch aktiven Verbindung, wobei
die gemeinsame Verabreichung der Kombinationstherapie in einer Kombinationstherapie mit fester Dosierung durch gemeinsame Verabreichung der in einem der vorhergehenden Ansprüche definierten Verbindungen mit mindestens einer zusätzlichen pharmazeutisch wirksamen Verbindung in einer Formulierung mit fester Dosierung durchgeführt werden kann; oder
die gemeinsame Verabreichung der Kombinationstherapie in einer Kombinationstherapie mit freier Dosis durch gemeinsame Verabreichung der in einem der vorhergehenden Ansprüche definierten Verbindungen und der mindestens einen zusätzlichen pharmazeutisch wirksamen Verbindung in freien Dosen der jeweiligen Verbindungen entweder durch gleichzeitige Verabreichung der einzelnen Verbindungen oder durch aufeinanderfolgende Verwendung der einzelnen Verbindungen über einen Zeitraum verteilt durchgeführt werden kann;
wobei die eine oder mehreren anderen pharmazeutisch aktiven Verbindungen vorzugsweise aktive Verbindungen zur Verringerung der Eisenüberladung sind, die ausgewählt sind aus Tmprss6-ASO, Eisenchelatoren, Curcumin, SSP-004184, Deferitrin, Deferasirox, Deferoxamin und/oder Deferipron; und/oder pharmazeutisch wirksame Verbindungen, die ausgewählt sind aus Antioxidantien, wie n-Acetylcystein; Antidiabetika, wie GLP-1-Rezeptor-Agonisten; Antibiotika, wie Vancomycin (Van) oder Tobramycin; Arzneimittel zur Behandlung von Malaria; Antikrebsmittel; Antimykotika; Arzneimittel zur Behandlung neurodegenerativer Erkrankungen wie der Alzheimer- und der Parkinson-Krankheit, einschließlich Dopamin-Agonisten wie Levodopa; antivirale Arzneimittel wie Interferon-α oder Ribavirin; Immunsuppressiva wie Cyclosporin A oder Cyclosporin-A-Derivate, Eisenpräparate, Vitaminpräparate, Stimulatoren der Erythrozytenproduktion, entzündungshemmende Biologika, Anti-Thrombolytika, Statine, Vasopressoren und inotrope Verbindungen.

## Revendications

1. Composés selon la formule (I) 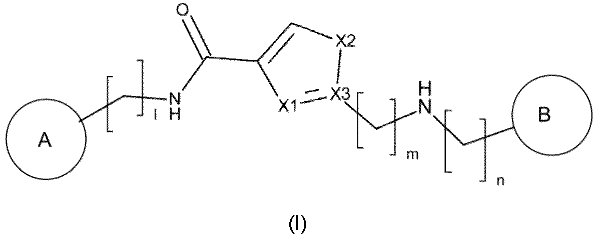 dans lequel
l est un nombre entier de 1 ou 2;
m et n sont indépendamment un nombre entier de 1, 2 ou 3;
X¹ est N, S ou O;
X² est N, S, O ou CR⁴;
X³est C ou N;
à condition que l'un des X¹ et X² soit N
et si X³ est N, alors X² est CR⁴;
et dans lequel
R⁴ représente
- H,
- halogène,
- C₁-C₃-alkyle linéaire ou ramifié,
- C₁-C₃-halogénoalkyle linéaire ou ramifié, ou
A représente l'un des groupes suivants (a-1), (a-2), (a-3), (a-4) et (a-5) 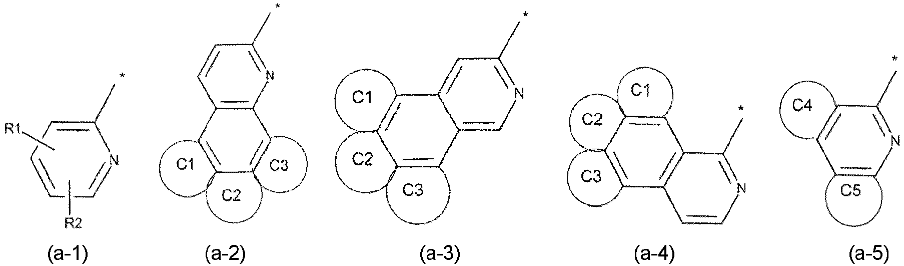
où * indique la position de liaison;
R¹ et R² représentent indépendamment l'un de l'autre
- H,
- halogène,
- C₁-C₃-alkyle linéaire ou ramifié,
- C₁-C₃-halogénoalkyle linéaire ou ramifié,
- C₁-C₃-alkoxy linéaire ou ramifié;
dans les formules (a-2), (a-3) et (a-4), l'un des éléments C1, C2 et C3 est présent et dans la formule (a-5), les éléments C4 et C5 sont présents, et
C1, C2, C3, C4 et C5 représentent de manière indépendante
- un cycle aryle fusionné à 6 membres,
- un cycle hétéroaryle fusionné à 5 ou 6 membres,
- un cycle cycloalkyle fusionné à 5 ou 6 membres,
- un cycle hétérocyclyle fusionné à 5 ou 6 membres; et
dans lequel les groupes (a-2), (a-3), (a-4) et (a-5) portent 0, 1, 2 ou 3 substituants, qui sont indépendamment choisis parmi les suivants
- halogène,
- C₁-C₃-alkyle linéaire ou ramifié,
- C₁-C₃-halogénoalkyle linéaire ou ramifié,
- C₁-C₃-alkoxy linéaire ou ramifié;
B représente l'un des groupes suivants (b-1), (b-2) et (b-3) 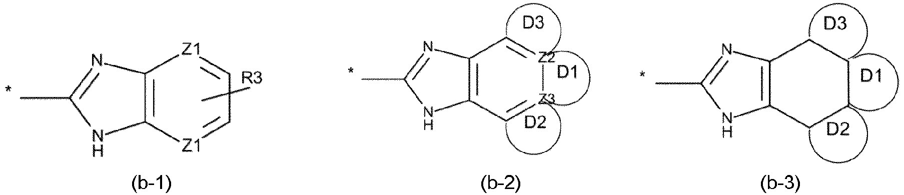
où * indique la position de liaison;
R³ représente
- H,
- aryle à 6 membres non substitué ou substitué,
- hétéroaryle à 5 ou 6 membres non substitué ou substitué,
- hétéroaryle bicyclique non substitué ou substitué,
- cycloalkyle à 5 ou 6 membres,
- hétérocycles à 5 ou 6 membres,
- hétérocyclylalkyle à 5 ou 6 membres,
- arylalkyle à 6 membres,
- arylalcinyle à 6 membres non substitué ou substitué,
- hétéroarylalkinyle à 5 ou 6 membres non substitué ou substitué, ou
- un groupe phényle, qui forme un cycle bicyclique fusionné avec un groupe cycloalkyle ou hétérocyclyle à 5 ou 6 membres;
Z¹ représente N ou C,
à condition qu'il n'y ait pas plus d'un Z¹ qui représente N;
dans les formules (b-2) et (b-3), l'un des éléments D1, D2 et D3 est présent et représente
- un cycle aryle fusionné à 6 membres,
- un cycle hétéroaryle fusionné à 5 ou 6 membres,
- un cycle cycloalkyle fusionné à 5 ou 6 membres,
- un cycle hétérocyclyle fusionné à 5 ou 6 membres; et
dans lequel les groupes (b-2) et (b-3) portent 0, 1, 2 ou 3 substituants, qui sont indépendamment choisis parmi
- halogène,
- C₁-C₃-alkyle linéaire ou ramifié,
- C₁-C₃-halogénoalkyle linéaire ou ramifié,
- C₁-C₃-alkoxy linéaire ou ramifié;
Z² et Z³ représentent N ou C,
à condition que Z² puisse représenter N lorsque D2 est présent et que Z³ puisse représenter N lorsque D3 est présent;
et dans lequel les composés (I) sont **caractérisés par le fait que** au moins un des groupes A et B contient au moins 3 cycles;
et leurs sels pharmaceutiquement acceptables.

2. Composés selon la revendication 1, dans lesquels
R³ représente
- H,
- aryle à 6 membres non substitué ou substitué,
- hétéroaryle à 5 ou 6 membres non substitué ou substitué,
- hétéroaryle bicyclique non substitué ou substitué,
- cycloalkyle à 5 ou 6 membres,
- hétérocycles à 5 ou 6 membres,
- hétérocyclylalkyle à 5 ou 6 membres,
- arylalcinyle à 6 membres.

3. Composés selon la revendication 1 ou 2,
dans lequel
l est un nombre entier de 1 ou 2;
m et n sont indépendamment un nombre entier de 1, 2 ou 3;
X¹ est N, S ou O;
X² est N, S, O ou CR⁴;
X³ est C ou N;
à condition que l'un des X¹ et X² soit N
et si X³ est N, alors X² est CR⁴;
et dans lequel
R⁴ représente H;
A représente l'un des groupes suivants (a-1), (a-2), (a-3), (a-4) et (a-5) 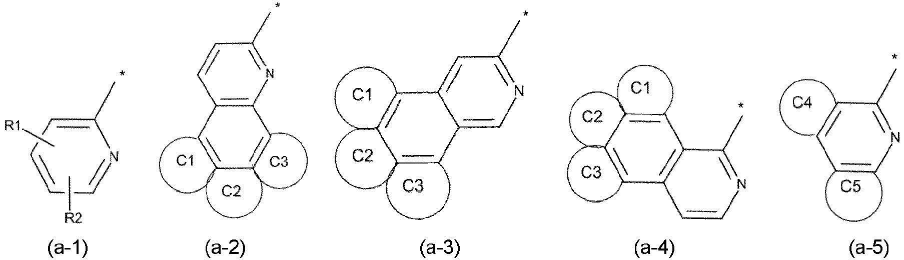
où * indique la position de liaison;
R¹ et R² représentent indépendamment l'un de l'autre
- hydrogène,
- halogène,
- C₁-C₃-alkyle linéaire ou ramifié,
- C₁-C₃-halogénoalkyle linéaire ou ramifié,
- C₁-C₃-alkoxy linéaire ou ramifié;
dans les formules (a-2), (a-3) et (a-4), l'un des éléments C1, C2 et C3 est présent et dans la formule (a-5), les éléments C4 et C5 sont présents, et
C1, C2, C3, C4 et C5 représentent un cycle phényle fusionné; et
dans lequel les groupes (a-2), (a-3), (a-4) et (a-5) portent 0 ou 1 substituant, choisi parmi
- halogène,
- C₁-C₃-alkyle linéaire ou ramifié,
- C₁-C₃-halogénoalkyle linéaire ou ramifié,
- C₁-C₃-alkoxy linéaire ou ramifié;
B représente l'un des groupes suivants (b-1), (b-2) et (b-3) 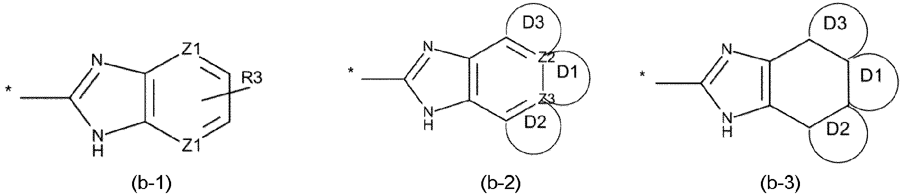
où * indique la position de liaison;
R³ représente
- H,
- phényle non substitué ou substitué,
- hétéroaryle à 5 ou 6 membres non substitué ou substitué,
- hétéroaryle bicyclique non substitué ou substitué,
- hétérocyclyle à 6 membres,
- hétérocyclylalkyle à 6 membres,
- phénylalkyle,
- phényléthinyle non substitué ou substitué ou hétéroaryléthinyle à 6 membres non substitué ou substitué, ou
- un groupe phényle, qui forme un cycle bicyclique fusionné avec un groupe cycloalkyle ou hétérocyclyle à 5 ou 6 membres;
Z¹ représente N ou C,
à condition qu'il n'y ait pas plus d'un Z¹ qui représente N;
dans les formules (b-2) et (b-3), l'un des éléments D1, D2 et D3 est présent et représente
- un cycle phényle fusionné,
- un cycle hétéroaryle fusionné à 6 membres,
- un cycle cycloalkyle fusionné à 6 membres,
- un cycle hétérocyclyle fusionné à 5 ou 6 membres; et
dans lequel les groupes (b-2) et (b-3) portent 0 ou 1 substituant, choisi parmi
- halogène,
- C₁-C₃-alkyle linéaire ou ramifié,
- C₁-C₃-halogénoalkyle linéaire ou ramifié,
- C₁-C₃-alkoxy linéaire ou ramifié;
Z² et Z³ représentent N ou C,
à condition que Z² puisse représenter N lorsque D2 est présent et que Z³ puisse représenter N lorsque D3 est présent;
et dans lesquels les composés (I) sont **caractérisés par le fait qu'**au moins un des groupes A et B contient au moins 3 cycles;
et leurs sels pharmaceutiquement acceptables.

4. Composés selon l'une des revendications 1 à 3, dans lesquels
R¹ et R² représentent indépendamment l'un de l'autre
- halogène,
- C₁-C₃-alkyle linéaire ou ramifié,
- C₁-C₃-halogénoalkyle linéaire ou ramifié,
- C₁-C₃-alkoxy linéaire ou ramifié; et
R³ représente
- H,
- phényle non substitué ou substitué,
- hétéroaryle à 5 ou 6 membres non substitué ou substitué,
- hétéroaryle bicyclique non substitué ou substitué,
- hétérocyclyle à 6 membres,
- hétérocyclylalkyle à 6 membres, et
- phényléthinyle.

5. Composés selon l'une des revendications 1 à 4, **caractérisés par le fait que** l'un des groupes A et B contient 3 cycles; et leurs sels pharmaceutiquement acceptables.

6. Composés selon l'une des revendications 1 à 5, dans lesquels
- le groupe A est un groupe (a-1); et/ou
- le groupe A est un groupe (a-2), (a-3) ou (a-5); et/ou
- le groupe A est un groupe (a-5); et/ou
- le groupe B est un groupe (b-1) ou (b-2);
et leurs sels pharmaceutiquement acceptables.

7. Composés selon l'une des revendications 1 à 6, dans lesquels les substituants possibles sont choisis parmi les groupes suivants
- les substituants halogènes représentent F, Cl et Br,
- les substituants C₁-C₃-alkyle linéaires ou ramifiés représentent le méthyle et l'éthyle,
- les substituants C₁-C₃-halogénoalkyles linéaires ou ramifiés représentent le trifluorométhyle (CF₃),
- les substituants C₁-C₃-alkoxy linéaires ou ramifiés représentent le méthoxy;
- les substituants hétéroaryles à 5 ou 6 membres représentent l'oxazolyle,
- un substituant alcoxyalkyléther représente un groupe méthoxyéthyléther,
- un substituant dialkylaminocarbonyle représente le diméthylaminocarbonyle,
de préférence, les substituants possibles sont choisis parmi les groupes suivants
- les substituants halogènes représentent F, Cl et Br,
- les substituants C₁-C₃-alkyle linéaires ou ramifiés représentent le méthyle,
- les substituants C₁-C₃-halogénoalkyles linéaires ou ramifiés représentent le trifluorométhyle (CF₃),
- les substituants C₁-C₃-alkoxy linéaires ou ramifiés représentent le méthoxy;
et leurs sels pharmaceutiquement acceptables.

8. Composés selon l'une des revendications 1 à 7, dans lesquels X¹, X² et X³ sont choisis pour former l'un des groupes suivants: 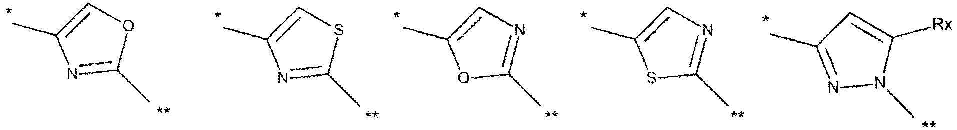
où * indique le site de liaison au groupe aminocarbonyle et ** indique le site de liaison au groupe -[(CH₂)]ₘ-amino-[(CH₂)]₍ₙ)- ;
et dans lequel
R⁴ représente
- H,
- halogène,
- C₁-C₃-alkyle linéaire ou ramifié,
- C₁-C₃-halogénoalkyle linéaire ou ramifié;
où, de préférence, X¹, X² et X³ sont choisis pour former l'un des groupes suivants: 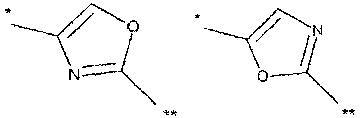 ou
où, de préférence, X¹, X² et X³ sont choisis pour former l'un des groupes suivants: 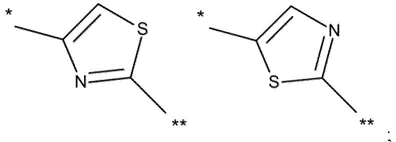
et leurs sels pharmaceutiquement acceptables.

9. Composés selon l'une quelconque des revendications précédentes, dans lesquels, pour le groupe A, les groupes suivants sont sélectionnés parmi les groupes (a-1), (a-2), (a-3) et (a-5): 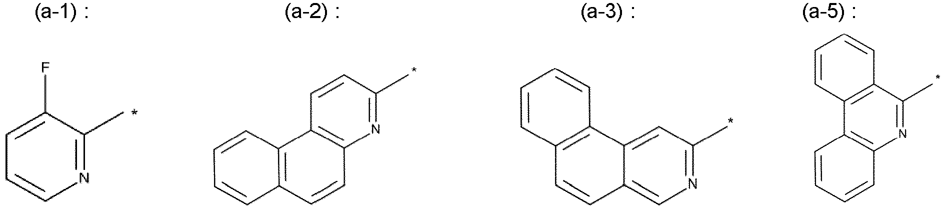 et leurs sels pharmaceutiquement acceptables.

10. Composés selon l'une quelconque des revendications précédentes, qui sont choisis parmi
| **No.** | **Structure chimique** |
|---|---|
| **1** | 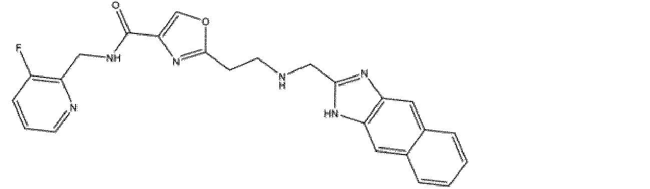 |
| **2** | 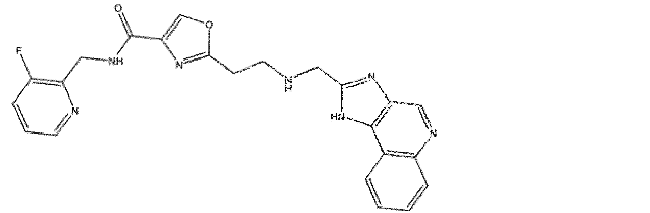 |
| **3** | 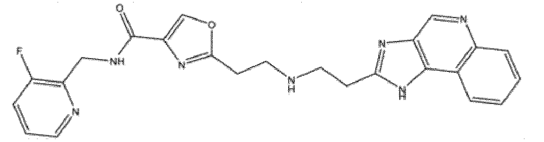 |
| **4** | 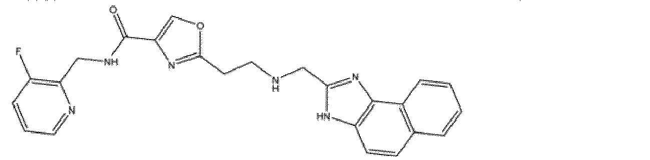 |
| **5** | 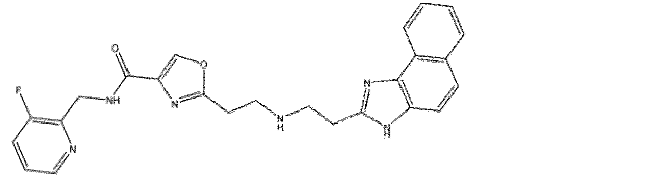 |
| **6** | 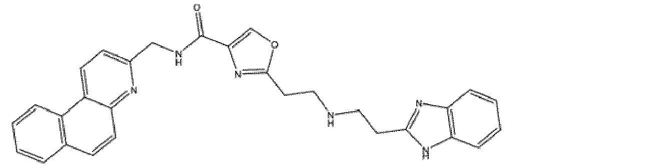 |
| **7** | 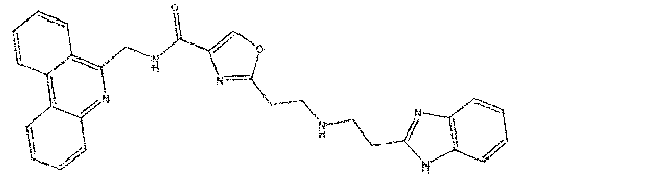 |
| **8** | 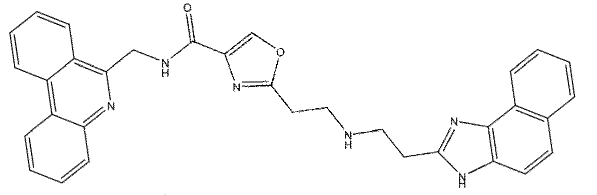 |
| **9** | 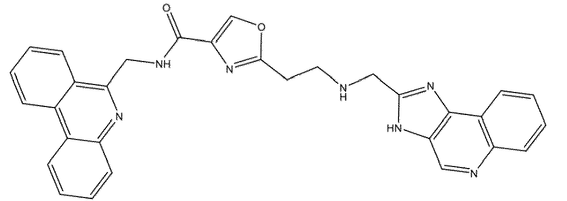 |
| **10** | 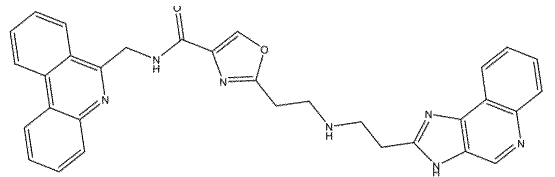 |
| **11** | 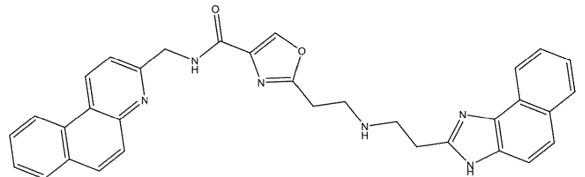 |
| **12** | 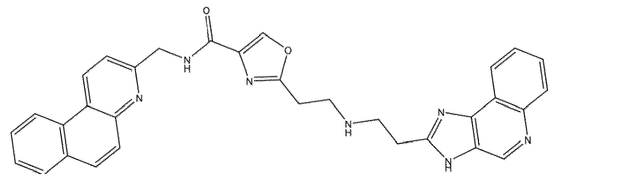 |
| **13** | 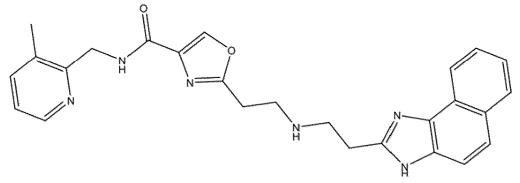 |
| **14** | 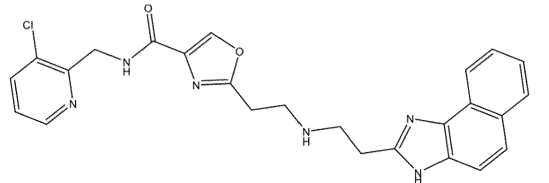 |
| **15** | 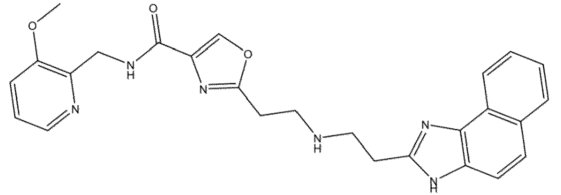 |
| **16** | 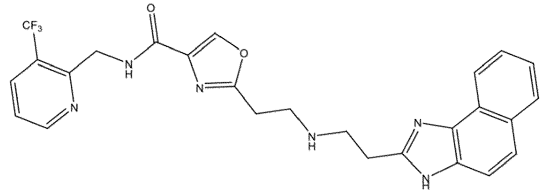 |
| **17** | 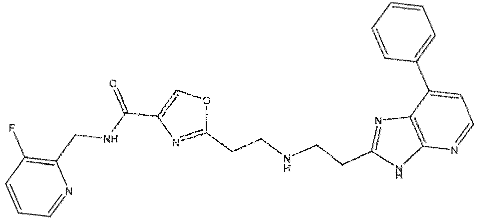 |
| **18** | 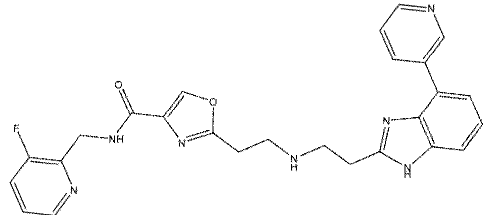 |
| **19** | 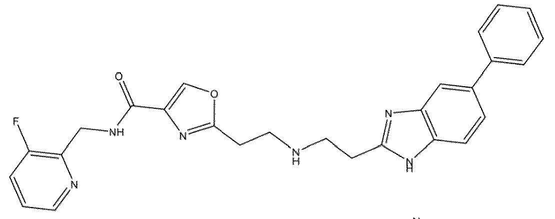 |
| **20** | 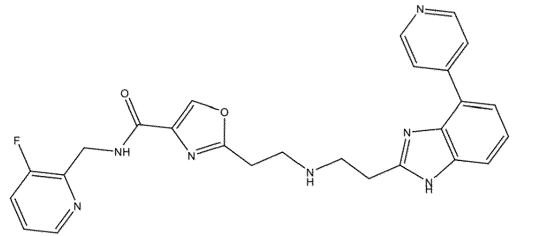 |
| **21** | 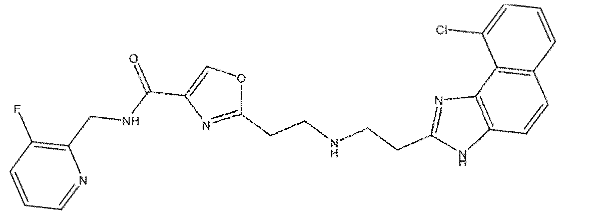 |
| **22** | 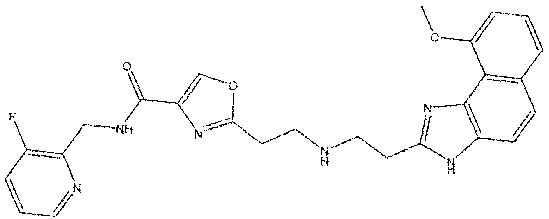 |
| **23** | 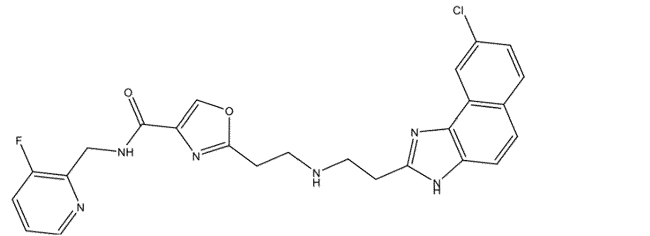 |
| **24** | 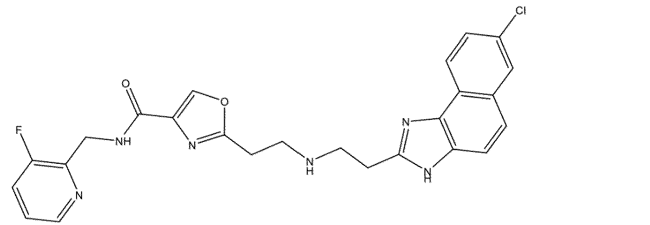 |
| **25** | 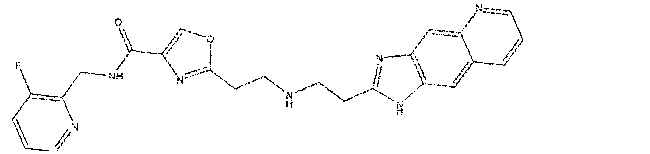 |
| **26** | 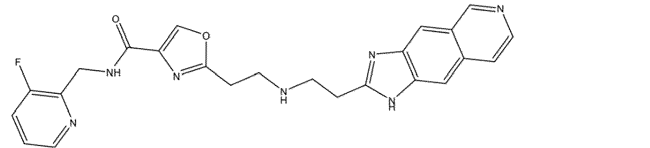 |
| **27** | 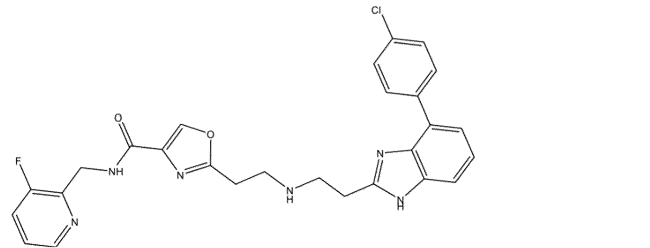 |
| **28** | 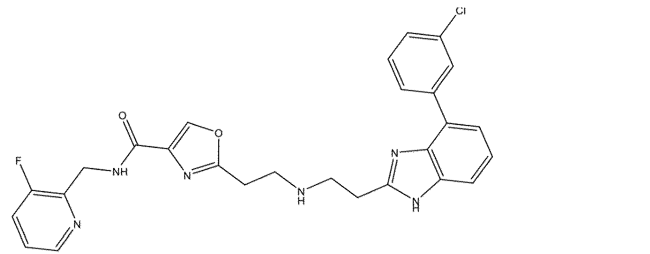 |
| **29** | 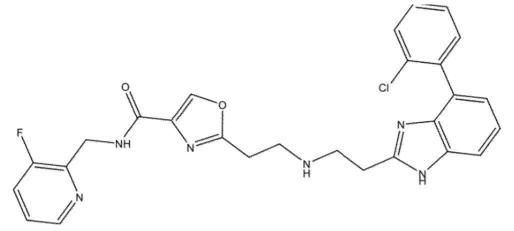 |
| **30** | 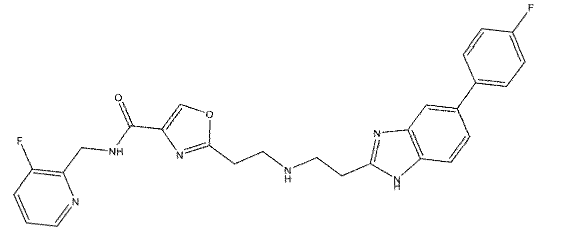 |
| **31** | 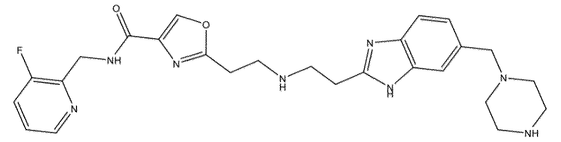 |
| **32** | 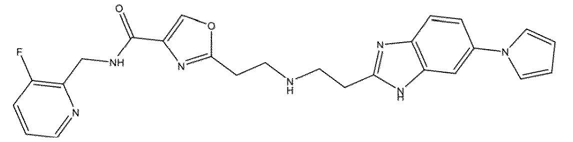 |
| **33** | 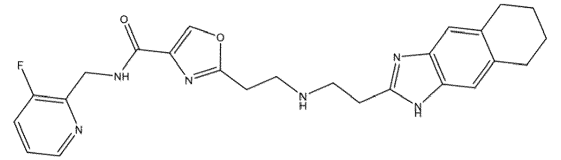 |
| **34** | 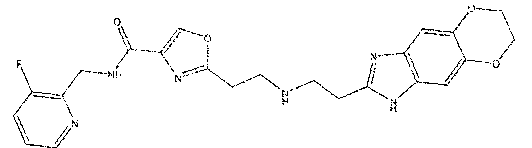 |
| **35** | 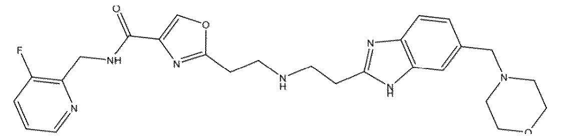 |
| **36** | 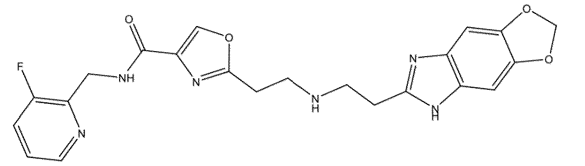 |
| **37** | 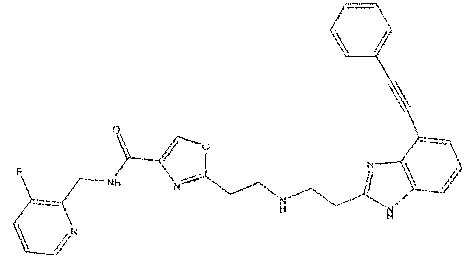 |
| **38** | 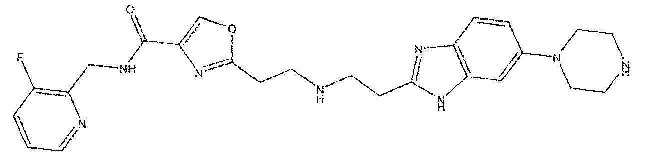 |
| **39** | 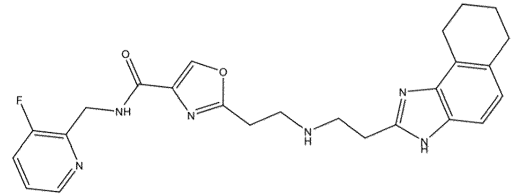 |
| **40** | 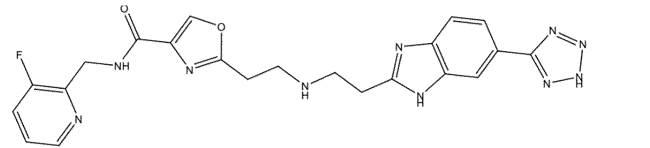 |
| **41** | 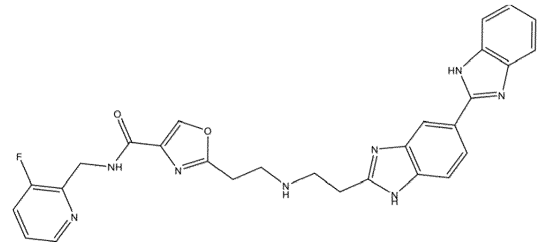 |
| **42** | 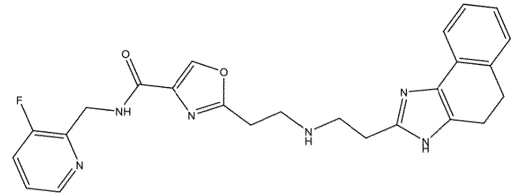 |
| **43** | 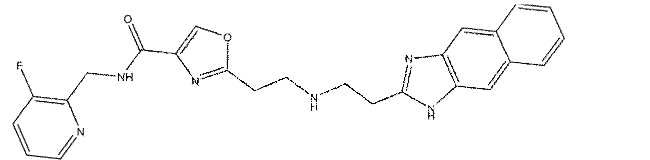 |
| **44** | 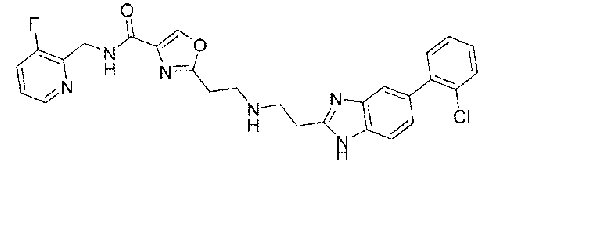 |
| **45** | 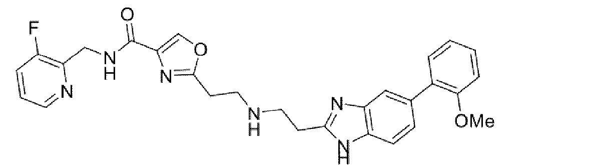 |
| **46** | 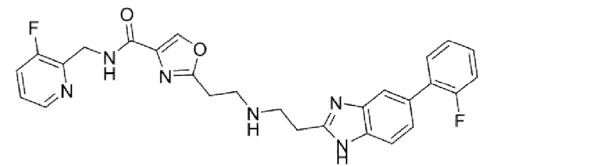 |
| **47** | 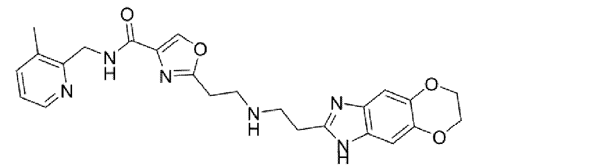 |
| **48** | 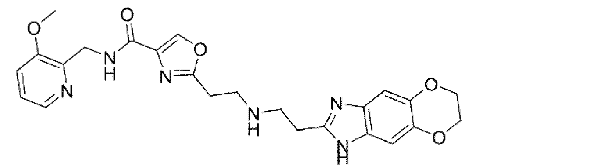 |
| **49** | 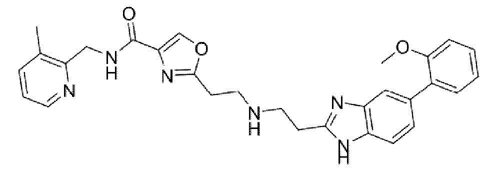 |
| **50** | 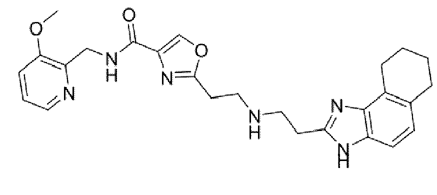 |
| **51** | 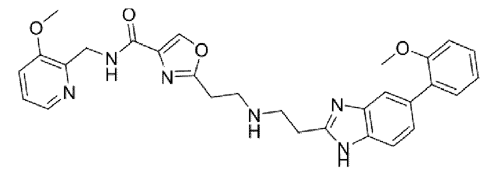 |
| **52** | 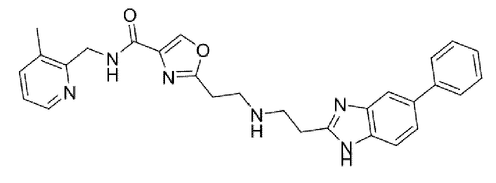 |
| **53** | 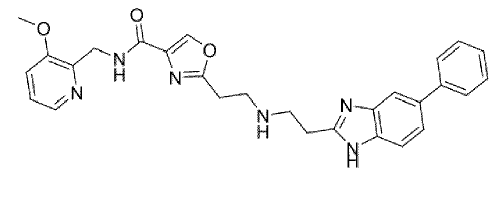 |
| **54** | 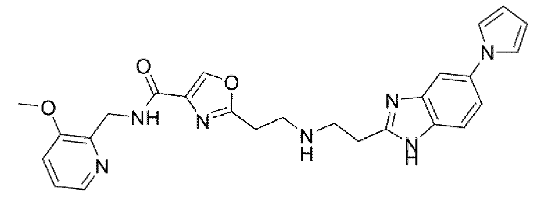 |
| **55** | 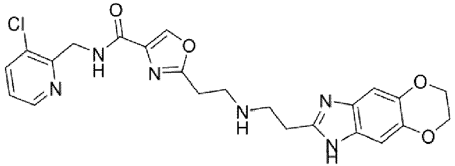 |
| **56** | 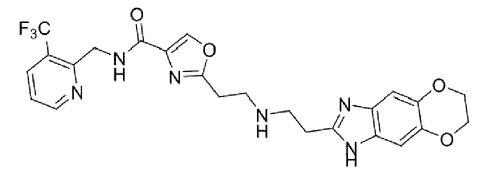 |
| **57** | 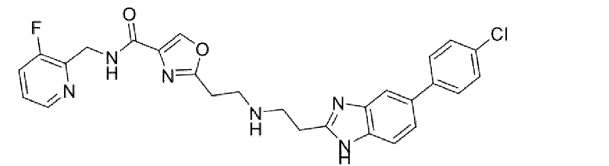 |
| **58** | 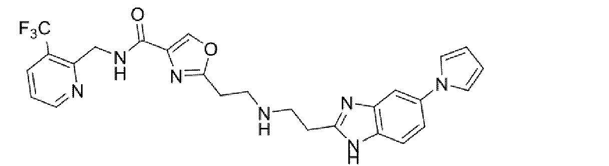 |
| **59** | 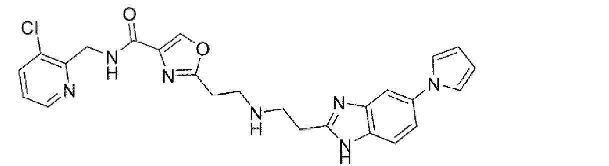 |
| **60** | 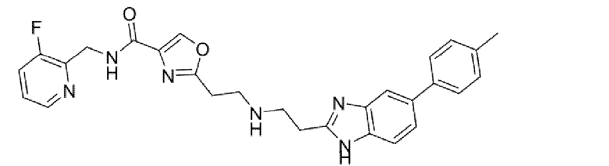 |
| **61** | 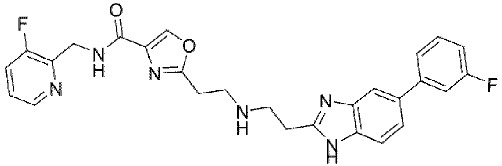 |
| **62** | 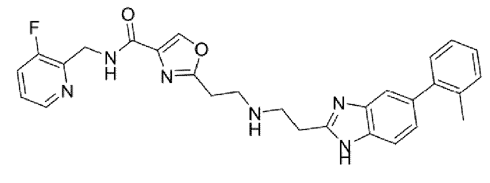 |
| **63** | 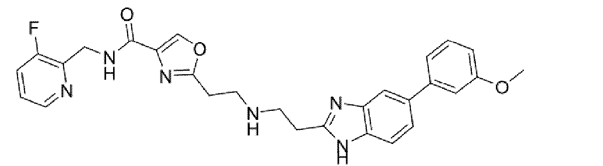 |
| **64** | 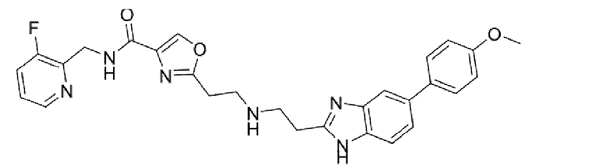 |
| **65** | 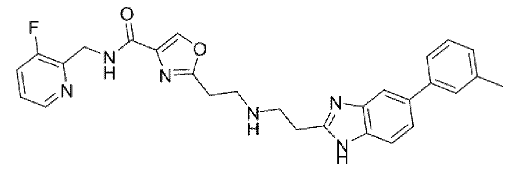 |
| **66** | 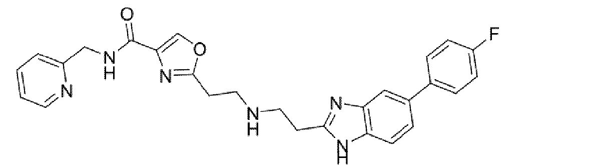 |
| **68** | 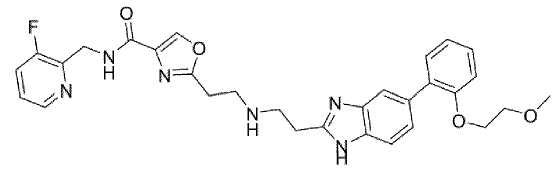 |
| **69** | 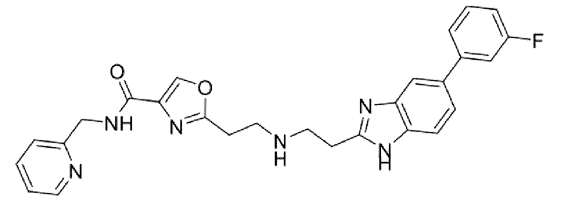 |
| **70** | 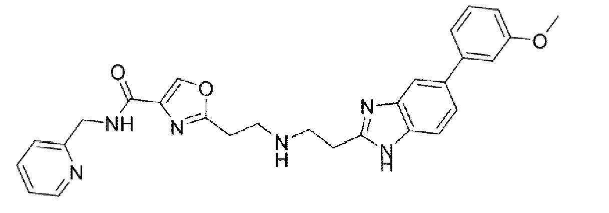 |
| **71** | 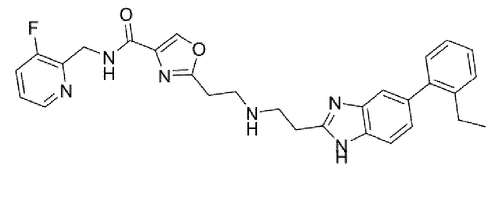 |
| **72** | 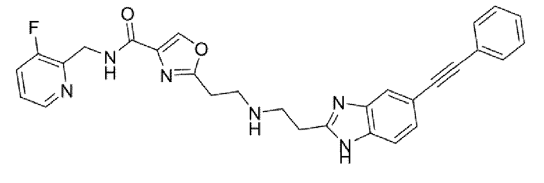 |
| **73** | 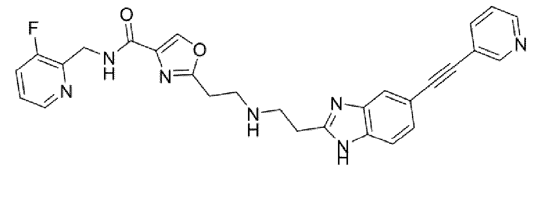 |
| **74** | 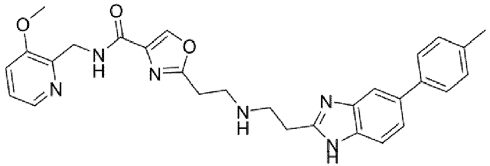 |
| **75** | 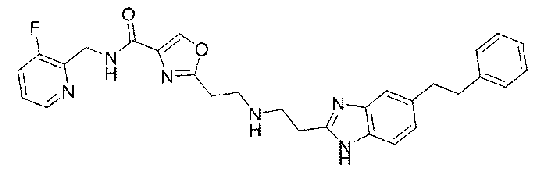 |
| **76** | 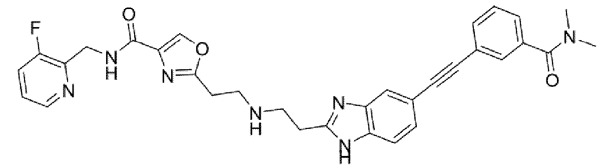 |
| **78** | 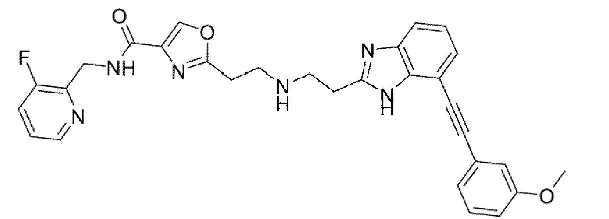 |
| **79** | 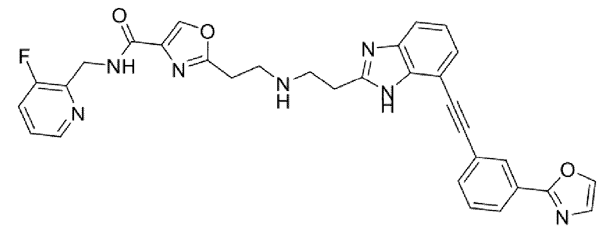 |
| **80** | 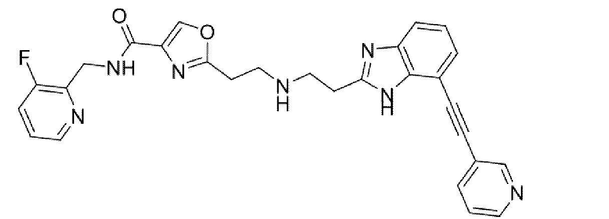 |
| **81** | 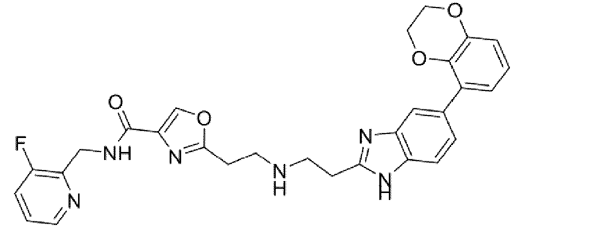 |
| **82** | 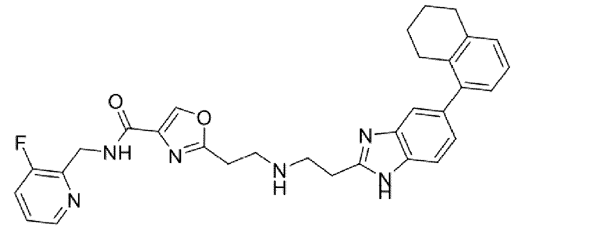 |
| **83** | 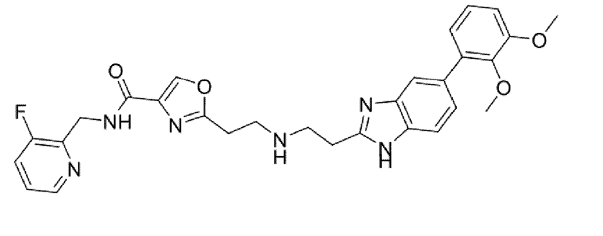 |
| **84** | 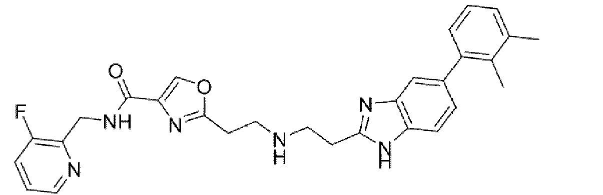 |
| **85** | 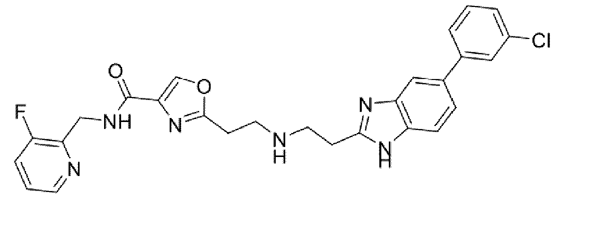 |
| **86** | 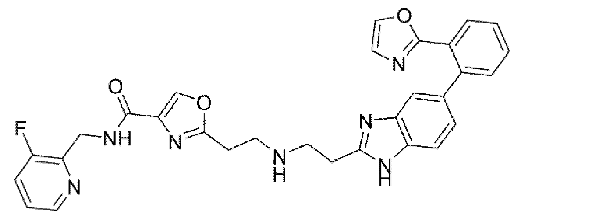 |
| **87** | 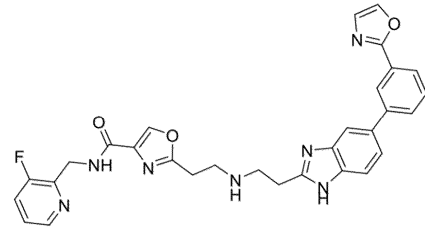 |
| **88** | 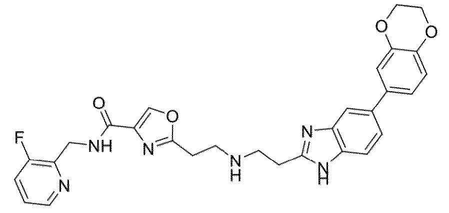 |
| **89** | 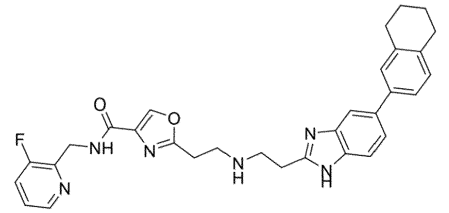 |
| **90** | 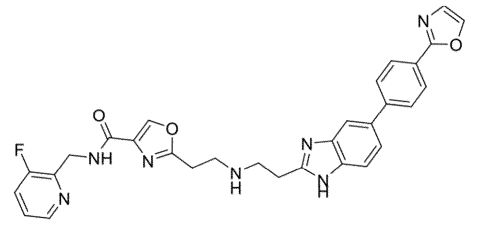 |
et leurs sels pharmaceutiquement acceptables

11. Composés selon l'une quelconque des revendications précédentes pour l'utilisation en tant que médicament.

12. Composés selon l'une des revendications 1 à 10 pour l'utilisation en tant qu'inhibiteur de la ferroportine ou pour l'utilisation dans l'inhibition du transport du fer médié par la ferroportine ou pour l'utilisation dans la prophylaxie et/ou le traitement des troubles du métabolisme du fer conduisant à des niveaux accrus de fer ou à une absorption accrue du fer, et/ou à une surcharge en fer.

13. Composés définis dans l'une des revendications 1 à 10 pour l'utilisation dans la prophylaxie et/ou le traitement de
- les maladies liées ou causées par une augmentation du taux de fer, une augmentation de l'absorption du fer ou une surcharge en fer, choisies parmi les thalassémies, y compris l'alpha-thalassémie, la bêta-thalassémie et la delta-thalassémie, l'hémoglobinopathie, la maladie de l'hémoglobine E, la maladie de l'hémoglobine H, l'hémochromatose, l'anémie hémolytique, y compris en particulier la drépanocytose ou l'anémie dysérythropoïétique congénitale; et/ou les maladies associées à une érythropoïèse inefficace, telles que les syndromes myélodysplasiques (SMD, myélodysplasie), l'anémie dysérythropoïétique congénitale et les néoplasmes myéloprolifératifs, tels que la polycythémie vera; et/ou
- des maladies causées par des niveaux réduits d'hepcidine; et/ou
- les infections causées par des micro-organismes pathogènes, tels que la bactérie Vibrio vulnificus, dans le cadre d'un traitement d'appoint, en limitant la quantité de fer disponible pour lesdits micro-organismes pathogènes; et/ou
- les maladies neurodégénératives telles que la maladie d'Alzheimer et la maladie de Parkinson en limitant le dépôt ou l'augmentation du fer dans les tissus ou les cellules; et/ou
- la formation de radicaux, d'espèces réactives de l'oxygène (ROS) et de stress oxydatif; et/ou
- des lésions cardiaques, hépatiques et endocriniennes causées par une surcharge en fer; et/ou
- l'inflammation déclenchée par l'excès de fer.

14. Médicament contenant un ou plusieurs des composés définis dans l'une quelconque des revendications 1 à 10, pouvant contenir en outre
- un ou plusieurs supports pharmaceutiques et/ou auxiliaires et/ou
- solvants, et/ou
- au moins un autre composé pharmaceutiquement actif, qui est de préférence choisi parmi les composés actifs pour la prophylaxie et le traitement de la surcharge en fer, de la thalassémie ou de l'hémochromatose, les composés actifs pour la prophylaxie et le traitement des maladies neurodégénératives, telles que la maladie d'Alzheimer ou la maladie de Parkinson, et les symptômes associés, et les composés chélateurs de fer;
et dans lequel ledit médicament se présente de préférence sous la forme d'une formulation destinée à l'administration orale ou parentérale.

15. Composés tels que définis dans l'une des revendications 1 à 10 pour l'utilisation dans une thérapie combinée, comprenant la co-administration des composés tels que définis dans l'une des revendications précédentes avec au moins un composé pharmaceutiquement actif supplémentaire, dans lequel
ladite co-administration de la thérapie combinée peut être effectuée dans une thérapie combinée à dose fixe par co-administration des composés tels que définis dans l'une quelconque des revendications précédentes avec au moins un composé pharmaceutiquement actif supplémentaire dans une formulation à dose fixe; ou
ladite co-administration de la thérapie combinée peut être effectuée dans une thérapie combinée à dose libre par co-administration des composés tels que définis dans l'une quelconque des revendications précédentes et d'au moins un composé pharmaceutiquement actif supplémentaire dans des doses libres des composés respectifs, soit par administration simultanée des composés individuels, soit par utilisation séquentielle des composés individuels répartis sur une période de temps;
dans lequel un ou plusieurs autres composés pharmaceutiquement actifs sont de préférence des composés actifs pour réduire la surcharge en fer, qui sont choisis parmi le Tmprss6-ASO, les chélateurs du fer, la curcumine, le SSP-004184, la déféritrine, le déférasirox, la déféroxamine et/ou le déferiprone ; et/ou des composés pharmaceutiquement actifs choisis parmi les antioxydants, tels que la n-acétyl-cystéine; les antidiabétiques, tels que les agonistes du récepteur GLP-1; les antibiotiques, tels que la vancomycine (Van) ou la tobramycine; les médicaments pour le traitement de la malaria; agents anticancéreux; médicaments antifongiques; médicaments pour le traitement des maladies neurodégénératives telles que la maladie d'Alzheimer et la maladie de Parkinson, comprenant des agonistes dopaminergiques tels que la lévodopa; médicaments antiviraux, tels que l'interféron-α ou la ribavirine; les immunosuppresseurs, tels que la cyclosporine A ou ses dérivés; les suppléments de fer; les suppléments de vitamines; les stimulateurs de la production de globules rouges; les produits biologiques anti-inflammatoires; les anti-thrombolytiques; les statines; les vasopresseurs; et les composés inotropes.
